# EUROPEAN PATENT APPLICATION

(11) **EP 4 059 936 A1**
(43) Date of publication of application: **21.09.2022**
(21) Application number: 22170836.5
(22) Date of filing: 25.03.2016
(51) Int. Cl.: C07F 5/00, A61K 31/395, A61K 31/555, A61K 51/00, A61P 35/00, A61P 43/00, C07B 59/00, C07D 255/02, C07D 257/02, C07F 1/08

(54) **METHOD FOR MANUFACTURING NOVEL NITROGEN-CONTAINING COMPOUND OR SALT THEREOF AND MANUFACTURING INTERMEDIATE OF NOVEL NITROGEN-CONTAINING COMPOUND OR SALT THEREOF**

(30) Priority: 25.03.2015 JP 2015062306
(62) Divisional of application: 21168086.3
(71) Applicant: FUJIFILM Corporation, Tokyo 106-8620 (JP)
(72) Inventor: FUKUNAGA, Hirofumi, Kanagawa 258-8577 (JP); SHINJO, Sachiko, Kanagawa 258-8577 (JP); NAKAGAWA, Daisuke, Kanagawa 258-8577 (JP); SEKINE, Shinichiro, Kanagawa 258-8577 (JP); YAMAKAWA, Takayuki, Kanagawa 258-8577 (JP)
(74) Representative: Hoffmann Eitle

(57) **Abstract**

Provided are a method for efficiently manufacturing a nitrogen-containing compound, which is used for manufacturing a treatment agent for integrin-related diseases, or a salt thereof and a manufacturing intermediate of the compound or a salt thereof.

A method for manufacturing a novel nitrogen-containing compound or a salt thereof includes (1) a step of obtaining a compound represented by Formula [10] or a salt thereof through an amidation reaction; and (2) a step of deprotecting the compound represented by Formula [10] or a salt thereof.

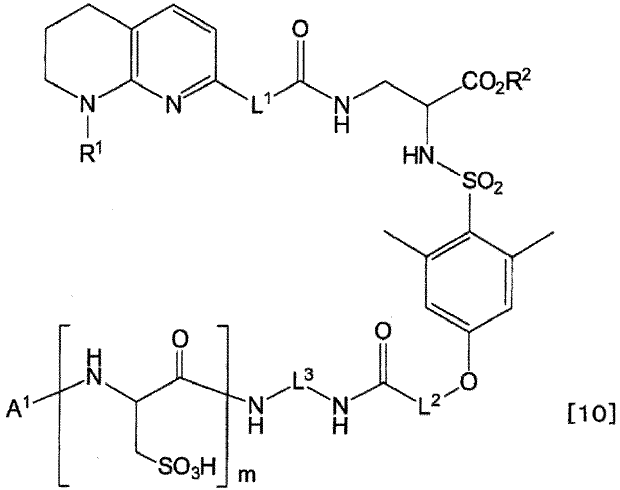

## Description

### Field of the Invention

The present invention relates to a method for manufacturing a novel nitrogen-containing compound or a salt thereof and a manufacturing intermediate of the compound or a salt thereof.

### Background Art

Integrins are a kind of cell adhesion receptors which constitute a family of heterodimeric glycoprotein complexes formed of α and β subunits and are mainly involved in the cell adhesion to extracellular matrix and the transmission of information from extracellular matrix.

Among the integrins, integrins α_{V}β₃ and α_{V}β₅ which are vitronectin receptors are known to be expressed at a low level on epithelial cells or matured endothelial cells while hyper-expressed in various tumor cells or new blood vessels. The hyper-expression of integrins α_{V}β₃ and α_{V}β₅ is considered to be involved in the exacerbation of cancer such as infiltration or metastasis accompanying tumor angiogenesis and be highly correlated to the malignancy (Non-Patent Document 1). It has been revealed that the hyper-expression of integrin is observed in cancer such as head and neck cancer, colorectal cancer, breast cancer, small cell lung cancer, non-small cell lung cancer, glioblastoma, malignant melanoma, pancreatic cancer, and prostatic cancer (Non-Patent Document 2).

Furthermore, it has been revealed that, in the integrin-related diseases such as ischemic diseases including an ischemic heart disease or a peripheral vascular disease, the integrin is hyper-expressed in endothelial cells of blood vessels at the time of angiogenesis following ischemia (Non-Patent Document 3).

The relationship between the aforementioned diseases and the expression of integrin is very interesting as a target of pharmaceutical products, and there are reports relating to the treatment using a low-molecular weight compound (Patent Documents 1 to 4) or a compound into which a radioactive isotope is introduced (Patent Documents 5 to 7) or relating to the imaging of diseases.

For example, an attempt at performing imaging by using a peptide ligand having an Arg-Gly-Asp (RGD) sequence is reported in Non-Patent Documents 4 and 5 and the like, and an attempt using a non-peptide low-molecular weight compound is reported in Cardiovascular Research, Vol. 78, pp. 395~403, 2008, and the like. In addition, the compound into which ¹⁸F of a positron nuclide is introduced (Non-Patent Documents 6 and 7) is used to portray a human tumor (Non-Patent Documents 8 and 9)

### Prior Art Documents

### Patent Documents

Patent Document 1: US6001961A
Patent Document 2: US6130231A
Patent Document 3: US2002/169200A
Patent Document 4: US2001/53853A
Patent Document 5: JP2002-532440A
Patent Document 6: WO2013/048996
Patent Document 7: WO2011/149250A

### Non-Patent Documents

Non-Patent Document 1: Nature Reviews cancer, Vol. 10, pp. 9-23, 2010
Non-Patent Document 2: Clin. Cancer Res. Vol. 12, pp. 3942-3949, 2006
Non-Patent Document 3: Circulation, Vol. 107, pp. 1046-1052, 2003
Non-Patent Document 4: Cancer Res. Vol. 61, pp. 1781-1785, 2001
Non-Patent Document 5: Cardiovascular Research, Vol. 78, pp. 395-403, 2008
Non-Patent Document 6: Clin. Cancer Res., Vol. 13, pp. 6610-6616, 2007
Non-Patent Document 7: J. Nucl. Med., Vol. 49, pp. 879-886, 2008
Non-Patent Document 8: Cancer Res., Vol. 62, pp. 6146-6151, 2002
Non-Patent Document 9: Int. J. Cancer., Vol. 123, pp.709-715, 2008

### SUMMARY OF THE INVENTION

According to the knowledge of the inventors of the present invention, a nitrogen-containing compound represented by the following Formula [11] is an excellent integrin-binding compound which exhibits high integrating properties and persistency with respect to angiogenesis and tumor relating to integrins and shows a high clearance rate in blood. The complex of the nitrogen-containing compound represented by Formula [11] or a salt thereof and a metal is useful as a treatment agent for diagnosis or treatment of integrin-related diseases.

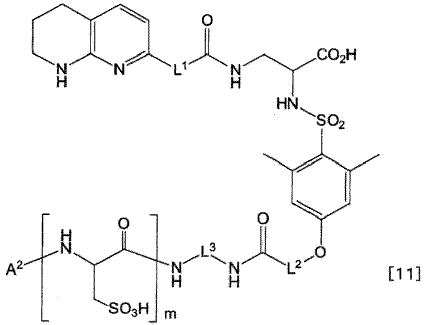

(In the formula, L¹ represents a group represented by Formula [2a] 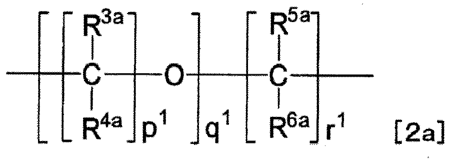 (in the formula, R^{3a}, R^{4a}, R^{5a}, and R^{6a} are the same as or different from each other and represent a hydrogen atom or a C₁₋₆ alkyl group; p¹ represents an integer of 1 to 3; q¹ represents an integer of 0 to 3; and r¹ represents an integer of 1 to 6); L² represents a group represented by Formula [2b] 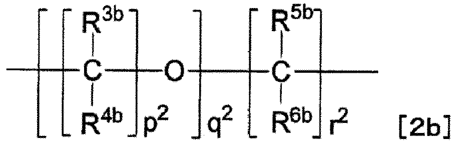 (in the formula, R^{3b}, R^{4b}, R^{5b}, and R^{6b} are the same as or different from each other and represent a hydrogen atom or a C₁₋₆ alkyl group; p² represents an integer of 1 to 3; q² represents an integer of 0 to 3; and r² represents an integer of 1 to 6); L³ represents a group represented by Formula [2c] 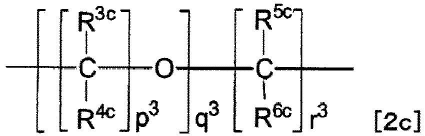 (in the formula, R^{3c}, R^{4c}, R^{5c}, and R^{6c} are the same as or different from each other and represent a hydrogen atom or a C₁₋₆ alkyl group; p³ represents an integer of 1 to 3; q³ represents an integer of 0 to 3; and r³ represents an integer of 1 to 6); A² represents any one of the groups represented by Formulae [12] to [17] 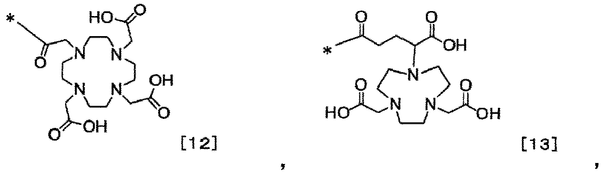 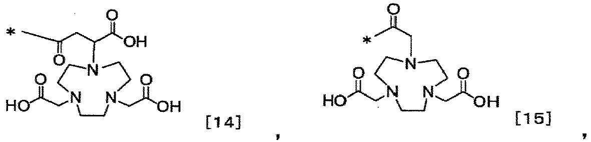 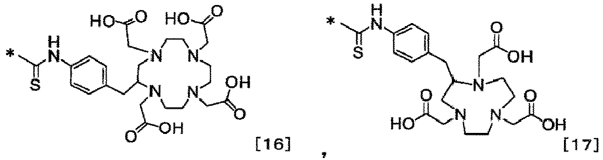 (in the formulae, * represents a binding position); and m represents an integer of 1 to 3).

Specific examples of the nitrogen-containing compound represented by Formula [11] include 2,2',2"-(10-(2-(((R)-1-((2-(4-(4-(N-((S)-1-carboxy-2-(5-(5,6,7,8-tetrahydro-1,8-naphthyridin-2-yl)pentanamide)ethyl)sulfamoyl)-3,5-diemthylphenoxy)butanamide)ethyl)amino)-1-oxo-3-s ulfopropan-2-yl)amino)-2-oxoethyl)-1,4,7,10-tetraazacyclododecane-1,4,7-triyl)triacetic acid (hereinafter, referred to as a compound A as well), 2,2'-(7-((R)-1-carboxy-4-(((R)-1-((2-(4-(4-(N-((S)-1-carboxy-2-(5-(5,6,7,8-tetrahydro-1,8-nap hthyridin-2-yl)pentanamide)ethyl)sulfamoyl)-3,5-dimethylphenoxy)pentanamide)ethyl)amino) -1-oxo-3-sulfopropan-2-yl)amino)-4-oxobutyl)-1,4,7-triazonane-1,4-diyl)diacetic acid (hereinafter, referred to as a compound B as well), and the like.

Objects of the present invention are to provide an efficient method for manufacturing a nitrogen-containing compound used for manufacturing a treatment agent for treating integrin-related diseases or a salt thereof and to provide a manufacturing intermediate of the compound or the salt.

In order to achieve the aforementioned objects, the inventors of the present invention repeated thorough research. As a result, they found that, by the following manufacturing method, a nitrogen-containing compound used for manufacturing a treatment agent for treating integrin-related diseases or a salt thereof can be efficiently manufactured. Furthermore, they found that the following manufacturing intermediate is an intermediate advantageous for efficiently manufacturing the nitrogen-containing compound used for manufacturing a treatment agent for treating integrin-relaetd diseases or a salt thereof. Based on what they found, the inventors accomplished the present invention.

That is, the present invention provides the following.
<1> A method for manufacturing a compound represented by Formula [11] or a salt thereof, comprising (1) a step of reacting a compound represented by Formula [1] or a salt thereof 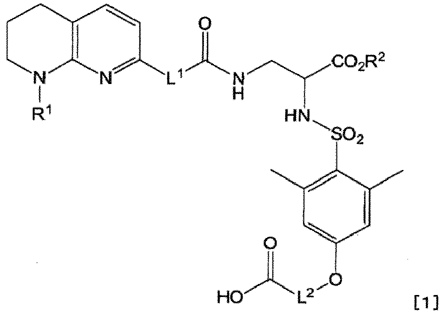
   (in the formula, R¹ represents a hydrogen atom or an amino-protecting group; R² represents a carboxyl-protecting group; L¹ represents a group represented by Formula [2a] 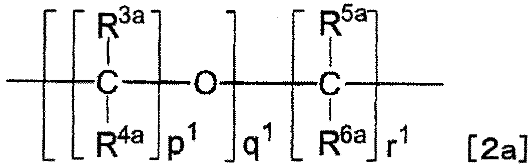
   (in the formula, R^{3a}, R^{4a}, R^{5a}, and R^{6a} are the same as or different from each other and represent a hydrogen atom or a C₁₋₆ alkyl group; p¹ represents an integer of 1 to 3; q¹ represents an integer of 0 to 3; and r¹ represents an integer of 1 to 6); and L² represents a group represented by Formula [2b] 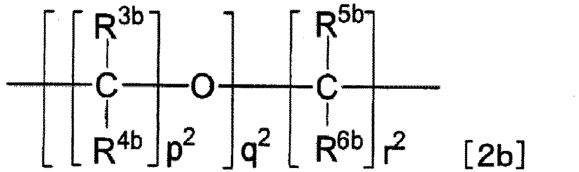 (in the formula, R^{3b}, R^{4b}, R^{5b}, and R^{6b} are the same as or different from each other and represent a hydrogen atom or a C₁₋₆ alkyl group; p² represents an integer of 1 to 3, q² represents an integer of 0 to 3; and r² represents an integer of 1 to 6)) with a compound represented by Formula [3] or a salt thereof 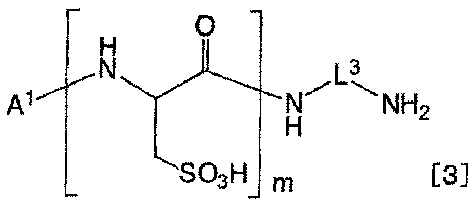 (in the formula, L³ represents a group represented by Formula [2c] 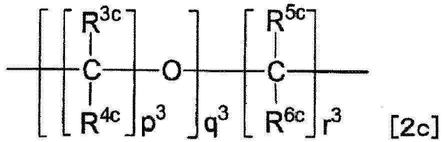 (in the formula, R^{3c}, R^{4c}, R^{5c}, and R^{6c} are the same as or different from each other and represent a hydrogen atom or a C₁₋₆ alkyl group; p³ represents an integer of 1 to 3; q³ represents an integer of 0 to 3; and r³ represents an integer of 1 to 6); A¹ represents any one of the groups represented by Formulae [4] to [9] 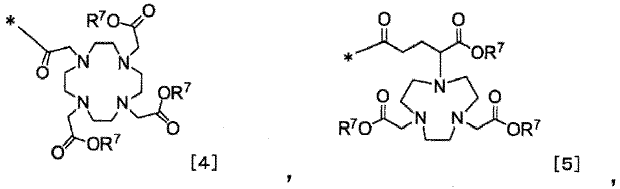 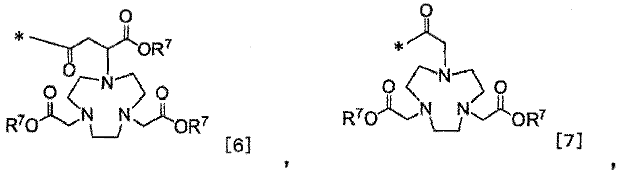 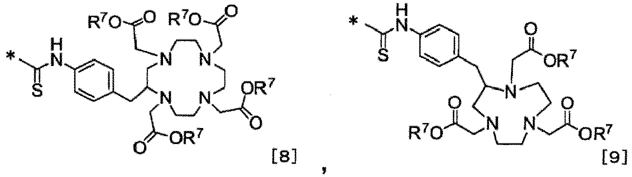 (in the formulae, * represents a binding position; and R^{7,}s are the same as or different from each other and represent a carboxyl-protecting group); and m represents an integer of 1 to 3)) so as to obtain a compound represented by Formula [10] or a salt thereof; 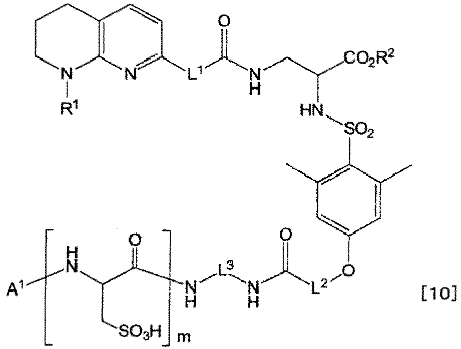 (in the formula, R¹, R2, L¹, L², L³, A¹, and m have the same definition as R¹, R², L¹, L², L³, A¹, and m described above); and (2) a step of deprotecting the compound represented by Formula [10] or a salt thereof, 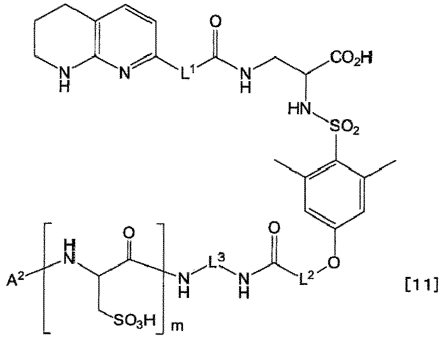 (in the formula, A² represents any one of the groups represented by Formulae [12] to [17] 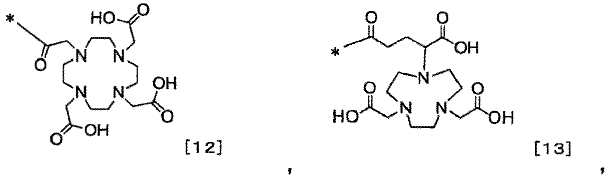 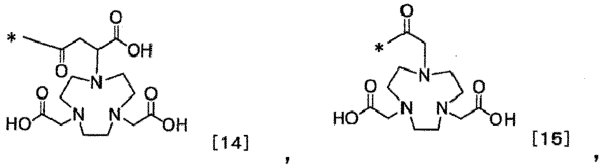 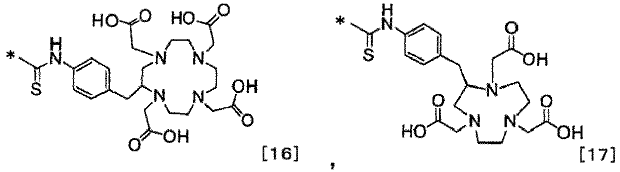 (in the formulae, * represents a binding position); and L¹, L², L³, and m have the same definition as L¹, L², L³, and m described above).
<2> The manufacturing method described in <1>, in which R² is a C₁₋₆ alkyl group which may be substituted or a benzyl group which may be substituted.
<3> The manufacturing method described in <1> or <2>, in which L³ is a group represented by Formula [18c] 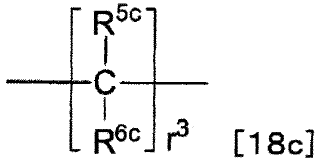 (in the formula, R^{5c} and R^{6c} may be the same as or different from each other and represent a hydrogen atom or a C₁₋₆ alkyl group; and r³ represents an integer of 1 to 6).
<4> The manufacturing method described in any one of <1> to <3>, in which L¹ is a group represented by Formula [18a] 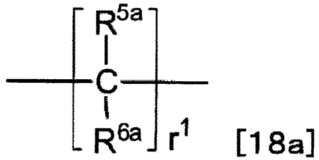 (in the formula, R^{5a} and R^{6a} are the same as or different from each other and represent a hydrogen atom or a C₁₋₆ alkyl group; and r¹ represents an integer of 1 to 6).
<5> The manufacturing method described in any one of <1> to <4>, in which L² is a group represented by Formula [18b] 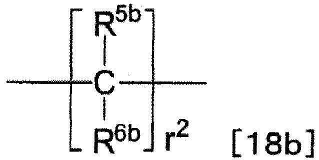 (in the formula, R^{5b} and R^{6b} are the same as or different from each other and represent a hydrogen atom or a C₁₋₆ alkyl group; and r² represents an integer of 1 to 6).
<6> The manufacturing method described in any one of <1> to <5>, in which R¹ is a hydrogen atom, a C₁₋₆ alkoxycarbonyl group which may be substituted, an arylsulfonyl group which may be substituted, or a heterocyclic sulfonyl group which may be substituted.
<7> The manufacturing method described in any one of <1> to <6>, in which R⁷ is a C₁₋₆ alkyl group which may be substituted or a benzyl group which may be substituted.
<8> The manufacturing method described in any one of <1> to <7>, in which the step of deprotecting is a step of deprotecting by using an acid.
<9> A method for manufacturing a metal complex, comprising a step of reacting the compound represented by Formula [11] or a salt thereof obtained by the manufacturing method described in any one of <1> to <7> with a metal ion.
<10> A compound represented by Formula [19] or a salt thereof 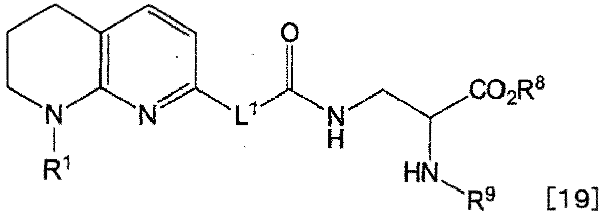
   (in the formula, R⁸ represents a C₂₋₆ alkyl group which may be substituted or a benzyl group which may be substituted; R⁹ represents a hydrogen atom, an amino-protecting group, or a group represented by Formula [20] 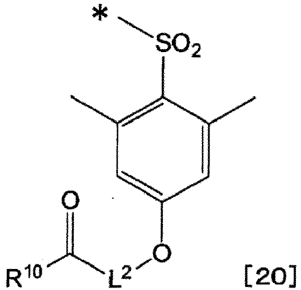
   (in the formula, * represents a binding position; R¹⁰ represents a hydroxyl group or a group represented by Formula [21] 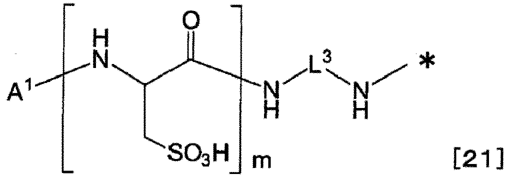
   ] (in the formula, *, L³, A¹, and m have the same definition as *, L³, A¹, and m described above); and L² has the same definition as L² described above); and R¹ and L¹ have the same definition as R¹ and L¹ described above).
<11> The compound described in <10> or a salt thereof, in which R⁸ is a C₂₋₆ alkyl group which may be substituted.
<12> The compound described in <10> or <11> or a salt thereof, in which L³ is a group represented by Formula [18c] 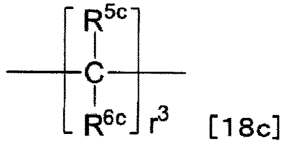 (in the formula, R^{5c}, R^{6c}, and r³ have the same definition as R^{5c}, R^{6c}, and r³ described above).
<13> The compound described in any one of <10> to <12> or a salt thereof, in which L¹ is a group represented by Formula [18a] 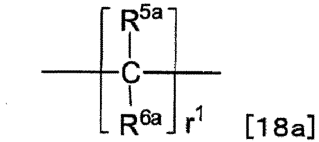 (in the formula, R^{5a}, R^{6a}, and r¹ have the same definition as R^{5a}, R^{6a}, and r¹ described above).
<14> The compound described in any one of <10> to <13> or a salt thereof, in which L² is a group represented by Formula [18b] 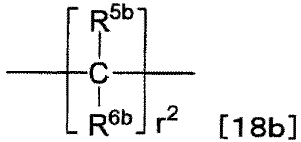 (in the formula, R^{5b}, R^{6b}, and r² have the same definition as R^{5b}, R^{6b}, and r² described above).
<15> The compound described in any one of <10> to <14> or a salt thereof, in which R¹ is a hydrogen atom, a C₁₋₆ alkoxycarbonyl group which may be substituted, an arylsulfonyl group which may be substituted, or a heterocyclic sulfonyl group which may be substituted.
<16> The compound described in any one of <10> to <15> or a salt thereof, in which R⁷ is a C₁₋₆ alkyl group which may be substituted or a benzyl group which may be substituted.
<17> A compound represented by Formula [3] or a salt thereof 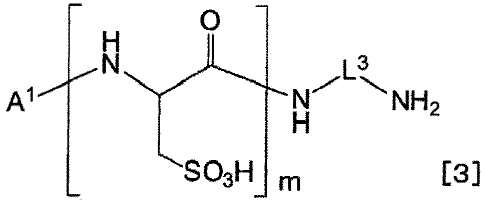 (in the formula, L³, A¹, and m have the same definition as L³, A¹, and m described above).
<18> The compound described in <17> or a salt thereof, in which R⁷ is a C₁₋₆ alkyl group which may be substituted or a benzyl group which may be substituted.

According to the manufacturing method of the present invention, it is possible to industrially obtain a novel nitrogen-containing compound having high optical purity or a salt thereof in a simple manner through short steps.

Furthermore, the manufacturing intermediate of the present invention is useful as an intermediate of a novel nitrogen-containing compound or a salt thereof.

### BRIEF DESCRIPTION OF THE DRAWINGS

Fig. 1 shows results obtained by imaging an integrin expression tumor by PET using [⁶⁴Cu]-(compound A).
Fig. 2 shows results obtained by imaging an integrin expression tumor by PET using [⁶⁴Cu]-(compound B).
Fig. 3 shows results obtained by imaging an integrin expression tumor by using a gamma camera.
Fig. 4 shows results obtained by imaging an integrin expression tumor in an intracranial tumor model.
Fig. 5 shows a trend of radioactivity concentration in blood of a monkey for which [^{m}In]-(compound A) is used.
Fig. 6 shows results obtained by temporally performing planar imaging on a monkey for which [^{m}In]-(compound A) is used.

### DESCRIPTION OF THE PREFERRED EMBODIMENTS

Hereinafter, the present invention will be specifically described.

In the present invention, unless otherwise specified, each term has the following meaning.

A halogen atom means a fluorine atom, a chlorine atom, a bromine atom, or an iodine atom.

AC₁₋₆ alkyl group means a linear or branched C₁₋₆ alkyl group such as an ethyl, methyl, propyl, isopropyl, butyl, sec-butyl, isobutyl, tert-butyl, pentyl, isopentyl, 2-methylbutyl, 2-pentyl, 3-pentyl, or hexyl group.

A C₂₋₆ alkyl group means a linear or branched C₂₋₆ alkyl group such as an ethyl, propyl, isopropyl, butyl, sec-butyl, isobutyl, tert-butyl, pentyl, isopentyl, 2-methylbutyl, 2-pentyl, 3-pentyl, or hexyl group.

A C₃₋₈ cycloalkyl group means a C₃₋₈ cycloalkyl group such as a cyclopropyl, cyclobutyl, cyclopentyl, or cyclohexyl group.

An aryl group means a C₆₋₁₃ aryl group such as a phenyl, naphthyl, or fluorenyl group.

An Ar C₁₋₆ alkyl group means a C₆₋₁₀ Ar C₁₋₆ alkyl group such as a benzyl, diphenylmethyl, trityl, phenethyl, 2-phenylpropyl, 3-phenylpropyl, or naphthylmethyl group.

A C₁₋₆ alkoxy group means a linear, cyclic, or branched C₁₋₆ alkyloxy group such as a methoxy, ethoxy, propoxy, isopropoxy, cyclopropoxy, butoxy, isobutoxy, sec-butoxy, tert-butoxy, cyclobutoxy, pentyloxy, or hexyloxy group.

A C₁₋₆ alkoxy C₁₋₆ alkyl group means a C₁₋₆ alkyloxy C₁₋₆ alkyl group such as a methoxymethyl or 1-ethoxyetyl group.

AC₁₋₆ alkylamino group means a linear, branched, or cyclic C₁₋₆ alkylamino group such as a methylamino, ethylamino, propylamino, isopropylamino, cyclopropylamino, butylamino, sec-butylamino, tert-butylamino, cyclobutylamino, pentylamino, cyclopentylamino, hexylamino, or cyclohexylamino group.

A di(C₁₋₆ alkyl)amino group means a linear, branched, or cyclic di(C₁₋₆ alkyl)amino group such as a dimethylamino, diethylamino, dipropylamino, diisopropylamino, dibutylamino, di(tert-butyl)amino, dipentylamino, dihexylamino, (ethyl)(methyl)amino, (methyl)(propyl)amino, (cyclopropyl)(methyl)amino, (cyclobutyl)(methyl)amino, or (cyclohexyl)(methyl)amino group.

A C₂₋₆ alkanoyl group means a linear or branched C₂₋₆ alkanoyl group such as an acetyl, propionyl, valeryl, isovaleryl, or pivaloyl group.

An aroyl group means a C₆₋₁₀ aryl group such as a benzoyl or naphthoyl group.

A heterocyclic carbonyl group means a monocyclic or bicyclic heterocyclic carbonyl group such as a furoyl, thenoyl, pyrrolidinylcarbonyl, piperidinylcarbonyl, piperazinylcarbonyl, morpholinylcarbonyl, or pyridinylcarbonyl group.

An acyl group means a formyl group, a C₂₋₆ alkanoyl group, an aroyl group, or a heterocyclic carbonyl group.

A C₁₋₆ alkoxycarbonyl group means a linear or branched C₁₋₆ alkyloxycarbonyl group such as a methoxycarbony, ethoxycarbonyl, isopropoxycarbonyl, tert-butoxycarbonyl, or 1,1 -dimethylpropoxycarbonyl group.

An Ar C₁₋₆ alkoxycarbonyl group means a C₆₋₁₃ Ar C₁₋₆ alkyloxycarbonyl group such as a benzyloxycarbonyl, phenethyloxycarbonyl, or fluorenylmethyloxycarbonyl group.

An aryloxycarbonyl group means a C₆₋₁₀ aryloxycarbonyl group such as a phenyloxycarbonyl or naphthyloxycarbonyl group.

A C₁₋₆ alkylsulfonyl group means a C₁₋₆ alkylsulfonyl group such as a methylsulfonyl, ethylsulfonyl, or propylsulfonyl group.

An arylsulfonyl group means a C₆₋₁₀ arylsulfonyl group such as a benzenesulfonyl, p-toluenesulfonyl, or naphthalenesulfonyl group.

A C₁₋₆ alkylsulfonyloxy group means a C₁₋₆ alkylsulfonyloxy group such as a methylsulfonyloxy or ethylsulfonyloxy group.

An arylsulfonyloxy group means a C₆₋₁₀ arylsulfonyloxy group such as a benzenesulfonyloxy or p-toluenesulfonyloxy group.

A heterocyclic sulfonyl group means a monocyclic or bicyclic heterocyclic sulfonyl group such as a piperidinesulfonyl, pyridinesulfonyl, quinolinesulfonyl, dihydrobenzofuransulfonyl, benzofuransulfonyl, chromanesulfonyl, and chromenesulfonyl.

A monocyclic nitrogen-containing heterocyclic group means a monocyclic heterocyclic group containing only nitrogen atoms as heteroatoms forming the ring, such as an aziridinyl, azetidinyl, pyrrolidinyl, pyrrolinyl, pyrrolyl, piperidyl, tetrahydropyridyl, dihydropyridyl, pyridyl, homopiperidinyl, octahydroazocinyl, imidazolidinyl, imidazolinyl, imidazolyl, pyrazolidinyl, pyrazolinyl, pyrazolyl, piperazinyl, pyrazinyl, pyridazinyl, pyrimidinyl, homopiperazinyl, triazolyl, or tetrazolyl group.

A monocyclic oxygen-containing heterocyclic group means a monocyclic heterocyclic group containing only oxygen atoms as heteroatoms forming the ring, such as an oxetanyl, tetrahydrofuranyl, furanyl, tetrahydropyranyl, pyranyl, 1,3-dioxanyl, or 1,4-dioxanyl group.

A monocyclic sulfur-containing heterocyclic group means a monocyclic heterocyclic group containing only sulfur atoms as heteroatoms forming the ring, such as a thienyl group.

A monocyclic nitrogen-oxygen-containing heterocyclic group means a monocyclic heterocyclic group containing only nitrogen atoms and oxygen atoms as heteroatoms forming the ring, such as an oxazolyl, isoxazolyl, oxadiazolyl, morpholinyl, or oxazepanyl group.

A monocyclic nitrogen·sulfur-containing heterocyclic group means a monocyclic heterocyclic group containing only nitrogen atoms and sulfur atoms as heteroatoms forming the ring, such as a thiazolyl, isothiazolyl, thiadiazolyl, monomorpholinyl, 1-oxidothiomorpholinyl, or 1,1-dioxidothiomorpholinyl group.

A monocyclic heterocyclic group means a monocyclic nitrogen-containing heterocyclic group, a monocyclic oxygen-containing heterocyclic group, a monocyclic sulfur-containing heterocyclic group, a monocyclic nitrogen-oxygen-containing heterocyclic group, or a monocyclic nitrogen·sulfur-containing heterocyclic group.

A bicyclic nitrogen-containing heterocyclic group means a bicyclic heterocyclic group containing only nitrogen atoms as heteroatoms forming the ring, such as an indolinyl, indolyl, isoindolinyl, isoindolyl, benzimidazolyl, indazolyl, benzotriazolyl, pyrazolopyridinyl, quinolyl, tetrahydroquinolinyl, tetrahydroisoquinolinyl, isoquinolinyl, quinolidinyl, cinnolinyl, phthalazinyl, quinazolinyl, dihydroquinoxalinyl, quinoxalinyl, naphthyridinyl, purinyl, phtheridinyl, or quinuclidinyl group.

A bicyclic oxygen-containing heterocyclic group means a bicyclic heterocyclic group containing only oxygen atoms as heteroatoms forming the ring, such as a dihydrobenzofuranyl, benzofuranyl, isobenzofuranyl, dihydrobenzofuranyl, chromanyl, chromenyl, isochromanyl, chromanyl, 1,3-benzodioxolyl, 1,3-benzodioxanyl, or 1,4-benzodioxanyl group.

A bicyclic sulfur-containing heterocyclic group means a bicyclic heterocyclic group containing only sulfur atoms as heteroatoms forming the ring, such as a 2,3-dihydrobenzothienyl or benzothienyl group.

A bicyclic nitrogen-oxygen-containing heterocyclic group means a bicyclic heterocyclic group containing only nitrogen atoms and oxygen atoms as heteroatoms forming the ring, such as a benzoxazolyl, benzisoxazolyl, benzoxadiazolyl, benzomorpholinyl, dihydropyranopyridyl, dioxopyrrolidyl, fluoropyridinyl, dihydrodioxinopyridyl, or dihydropyridooxazinyl group.

A bicyclic nitrogen·sulfur-containing heterocyclic group means a bicyclic heterocyclic group containing only nitrogen atoms and sulfur atoms as heteroatoms forming the ring, such as a benzothiazolyl, benzisothiazolyl, or benzothiadiazolyl group.

A bicyclic heterocyclic group means a bicyclic nitrogen-containing heterocyclic group, a bicyclic oxygen-containing heterocyclic group, a bicyclic sulfur-containing heterocyclic group, a bicyclic nitrogen-oxygen-containing heterocyclic group, or a bicyclic nitrogen·sulfur-containing heterocyclic group.

A heterocyclic group means a monocyclic heterocyclic group or a bicyclic heterocyclic group.

A silyl group means a trialkylsilyl group such as a trimethylsilyl, triethylsilyl, or tributylsilyl group.

An amino-protecting group includes all of the groups that can be used as a general amino group-protecting group, and examples thereof include those described in W. Greene et al., Protective Groups in Organic Synthesis, 4th edition, pp. 696~926, 2007, John Wiley & Sons, INC. Specifically, examples of the amino-protecting group include an Ar C₁₋₆ alkyl group, a C₁₋₆ alkoxy C₁₋₆ alkyl group, an acyl group, a C₁₋₆ alkoxycarbonyl group, an Ar C₁₋₆ alkoxycarbonyl group, an aryloxycarbonyl group, a C₁₋₆ alkylsulfonyl group, a heterocyclic sulfonyl group, an arylsulfonyl group, a silyl group, and the like. These groups may be substituted with one or more substituents selected from the substituent group A.

Substituent group A: a halogen atom, a nitro group, a cyano group, an amino group which may be protected, a hydroxyl group which may be protected, a C₁₋₆ alkyl group, a C₃₋₈ cycloalkyl group, an aryl group, a C₁₋₆ alkoxy group, a C₁₋₆ alkylamino group, a di(C₁₋₆ alkyl)amino group, a heterocyclic group, and an oxy group.

A carboxyl-protecting group include all of the groups that can be used as a general carboxyl group-protecting group, and examples thereof include those described in W. Greene et al., Protective Groups in Organic Synthesis, 4th edition, pp. 533~646, 2007, John Wiley & Sons, INC. Specific examples of the carboxyl-protecting group include a C₁₋₆ alkyl group, an aryl group, a benzyl group, an Ar C₁₋₆ alkyl group, a C₁₋₆ alkoxy C₁₋₆ alkyl group, a silyl group, and the like. These groups may be substituted with one or more substituents selected from the substituent group A.

A hydroxyl-protecting group include all of the groups that can be used as a general hydroxyl group-protecting group, and examples thereof include those described in W. Greene et al., Protective Groups in Organic Synthesis, 4th edition, pp. 16~299, 2007, John Wiley & Sons, INC. Specific examples of the hydroxyl-protecting group include a C₁₋₆ alkyl group, an Ar C₁₋₆ alkyl group, a C₁₋₆ alkoxy C₁₋₆ alkyl group, an acyl group, a C₁₋₆ alkoxycarbonyl group, an Ar C₁₋₆ alkoxycarbonyl group, a C₁₋₆ alkylsulfonyl group, an arylsulfonyl group, a silyl group, a tetrahydrofuranyl group, a tetrahydropyranyl group, and the like. These groups may be substituted with one or more substituents selected from the substituent group A.

Examples of a leaving group include a halogen atom, a C₁₋₆ alkylsulfonyloxy group, an arylsulfonyloxy group, and the like. The C₁₋₆ alkylsulfonyloxy group and the arylsulfonyloxy group may be substituted with one or more substituents selected from the substituent group A.

Examples of halogenated hydrocarbons include methylene chloride, chloroform, dichloroethane, and the like.

Examples of ethers include diethylether, diisopropylether, dioxane, tetrahydrofuran, anisole, ethylene glycol dimethyl ether, diethylene glycol dimethyl ether, diethylene glycol diethyl ether, and the like.

Examples of alcohols include methanol, ethanol, propanol, 2-propanol, butanol, 2-methyl-2-propanol, and the like.

Examples of esters include methyl acetate, ethyl acetate, propyl acetate, butyl acetate, and the like.

Examples of amides include N,N-dimethylformamide, N,N-dimethylacetamide, N-methylpyrrolidone, and the like.

Examples of nitriles include acetonitrile, propionitrile, and the like.

Examples of an inorganic base include sodium hydroxide, potassium hydroxide, lithium hydroxide, sodium tert-butoxide, potassium tert-butoxide, sodium hydrogen carbonate, sodium carbonate, potassium carbonate, lithium carbonate, cesium carbonate, and the like.

Examples of an organic base include triethylamine, N,N-diisopropylethylamine, 1,8-diazabicyclo(5.4.0)undec-7-ene (DBU), 4-dimethylaminopyridine, pyridine, imidazole, N-methylimidazole, N-methylmorpholine, and the like.

Examples of the salt of the compound represented by Formula [1], [3]. [10], [11], or [19] include a generally known salt in a basic group such as an amino group and in an acidic group such as a hydroxyl group and a carboxyl group.

Examples of the salt in a basic group include a salt with a mineral acid such as hydrochloric acid, hydrobromic acid, nitric acid, and sulfuric acid; a salt with organic carboxylic acid such as formic acid, acetic acid, citric acid, oxalic acid, fumaric acid, maleic acid, succinic acid, malic acid, tartaric acid, aspartic acid, trichloroacetic acid, and trifluoroacetic acid; and a salt with a sulfonic acid such as methanesulfonic acid, benzenesulfonic acid, p-toluenesulfonic acid, mesitylenesulfonic acid, and naphthalenesulfonic acid.

Examples of the salt in an acidic group include a salt with an alkali metal such as lithium, sodium, and potassium; a salt with an alkaline earth metal such as potassium and magnesium; an ammonium salt; a salt with a nitrogen-containing organic base such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine; and the like.

Among the above salts, pharmacologically acceptable salts are exemplified as preferred salts.

Examples of a metal of metal ion and a metal complex include a paramagnetic metal, an X-ray-absorbing metal, a radioactive metal, and the like.

In a case where a metal complex is used as a treatment agent for diagnosis or treatment, examples of the metal complex include the following metal complexes according to the use thereof.

Examples of the metal complex used in a treatment agent for nuclear magnetic resonance diagnosis or the like include complexes containing a paramagnetic metal ion (for example, an ion of a metal selected from the group consisting of Co, Mn, Cu, Cr, Ni, V, Au, Fe, Eu, Gd, Dy, Tb, Ho, and Er) as a metal component.

Examples of the metal complex used in a treatment agent for X-ray diagnosis or the like include complexes containing an X-ray-absorbing metal ion (for example, an ion of a metal selected from the group consisting of Re, Sm, Ho, Lu, Pm, Y, Bi, Pb, Os, Pd, Gd, La, Au, Yb, Dy, Cu, Rh, Ag, and Ir) as a metal component.

Examples of the metal complex used in a treatment agent for radiodiagnosis, treatment, or the like include complexes containing a non-cytotoxic radioactive metal ion (for example, an ion of a metal selected from the group consisting of a ¹⁸F aluminum complex, a ¹⁸F gallium complex, a ¹⁸F indium complex, a ¹⁸F lutetium complex, a ¹⁸F thallium complex, ⁴⁴Sc, ⁴⁷SC, ⁵¹Cr, ^{52m}Mn, ⁵⁵CO, ⁵⁷CO, ⁵⁸CO, ⁵²Fe, ⁵⁹Fe, ⁶⁰CO, ⁶²Cu, ⁶⁴Cu, ⁶⁷Cu, ⁶⁷Ga, ⁶⁸Ga, ⁷²As, ⁷²Se, ⁷³Se, ⁷⁵Se, ⁷⁶As, ⁸²Rb, ⁸²Sr, ⁸⁵Sr, ⁸⁹Sr, ⁸⁹Zr, ⁸⁶Y, ⁸⁷Y, ⁹⁰Y, ⁹⁵Tc, ^{99m}Tc, ¹⁰³Ru, ¹⁰³Pd, ¹⁰⁵Rh, ¹⁰⁹Pd, ¹¹¹In, ^{114m}In, ^{117m}Sn, 111Ag ^{113m}In, ¹⁴⁰_{La}, ¹⁴⁹_{Pm}, ¹⁴⁹_{Tb}, ¹⁵²Tb, ¹⁵⁵Tb, ¹⁶¹Tb, ¹⁵³Sm, ¹⁵⁹Gd, ¹⁶⁵Dy, ¹⁶⁶Dy, ¹⁶⁶Ho, ¹⁶⁵Er, ¹⁶⁹Yb, ¹⁷⁵Yb, 177Lu, ¹⁸⁶Re, ¹⁸⁸Re, ¹⁹²Ir, 197Hg, ¹⁹⁸Au, ¹⁹⁹Au, ²⁰¹T1, ²⁰³Hg, ²¹¹At, ²¹²Bi, ²¹²Pb, ²¹³Bi, ²¹⁷Bi, ²²³Ra, ²²⁵Ac, and ²²⁷Th) as a metal component.

In a case where a metal complex is used as a treatment agent for radiodiagnosis, as a metal, a non-cytotoxic radioactive metal can be used.

Examples of the non-cytotoxic radioactive metal include a gamma ray-emitting nuclide and a positron-emitting nuclide. Specific examples thereof include a ¹⁸F aluminum complex, ¹⁸F gallium complex, ¹⁸F indium complex, ¹⁸F lutetium complex, ¹⁸F thallium complex, ^{99m}Tc, ¹¹¹In, ^{113m}In, ^{114m}In, ⁶⁷Ga, ⁶⁸Ga, ⁸²Rb, ⁸⁶Y, ⁸⁷Y, ¹⁵²Tb, ¹⁵⁵Tb, ²⁰¹T1, ⁵¹Cr, ⁵²Fe, ⁵⁷Co, ⁵⁸Co, ⁶⁰Co, ⁸²Sr, ⁸⁵Sr, ¹⁹⁷Hg, ⁴⁴Sc, ⁶²Cu, ⁶⁴Cu, ⁸⁹Zr, and the like.

In a case where a metal complex is used as a treatment agent for radiotherapy, as a metal, a cytotoxic radioactive metal can be used.

Examples of the cytotoxic radioactive metal include α-ray-emitting nuclide and a β-ray-emitting nuclide. Specific examples thereof include ⁹⁰Y, ^{114m}In, ^{117m}Sn, ¹⁸⁶Re, ¹⁸⁸Re, ⁶⁴Cu, ⁶⁷Cu, ⁵⁹Fe, ⁸⁹Sr, ¹⁹⁸Au, ²⁰³Hg, ²¹²Pb, ^{I65}Dy, ¹⁰³Ru, ¹⁴⁹Tb, ¹⁶¹Tb_{,} ²¹²Bi, ¹⁶⁶Ho, ¹⁶⁵Er, ¹⁵³Sm, 177Lu, ²¹³Bi, ²²³Ra, ²²⁵Ac, ²²⁷Th, and the like.

The treatment means diagnosis or treatment for various diseases.

The diagnosis means a process of determining whether a certain disease is a disease of interest or determining the state of a disease of interest.

The treatment means the improvement of the state of a disease of interest, the inhibition of the progress of a disease of interest, or the like.

The treatment agent means a substance administered for the procedure.

R¹ is preferably a hydrogen atom, a C₁₋₆ alkoxycarbonyl group which may be substituted, a heterocyclic sulfonyl group which may be substituted, or an arylsulfonyl group which may be substituted, more preferably a hydrogen atom, a C₁₋₆ alkoxycarbonyl group which may be substituted with one or more substituents selected from the substituent group A, a heterocyclic sulfonyl group which may be substituted with one or more substituents selected from the substituent group A, or an arylsulfonyl group which may be substituted with one or more substituents selected from the substituent group A, and even more preferably a hydrogen atom, a C₁₋₆ alkoxycarbonyl group, a 2,2,4,6,7-pentamethyldihydrobenzofuran-5-sulfonyl group, or a 2,2,5,7,8-pentamethylchromane-6-sulfonyl group.

R² is preferably a C₁₋₆ alkyl group which may be substituted or a benzyl group which may be substituted, more preferably a C₁₋₆ alkyl group which may be substituted with one or more substituents selected from the substituent group A or a benzyl group which may be substituted with one or more substituents selected from the substituent group A, even more preferably a C₁₋₆ alkyl group which may be substituted with a halogen atom or a benzyl group which may be substituted with one or more groups selected from a halogen atom, a nitro group, and a C₁₋₆ alkoxy group, and particularly preferably a C₁₋₆ alkyl group which may be substituted with a halogen atom.

R^{3a} is preferably a hydrogen atom.

R^{3b} is preferably a hydrogen atom.

R^{3c} is preferably a hydrogen atom.

R^{4a} is preferably a hydrogen atom.

R^{4b} is preferably a hydrogen atom.

R^{4c} is preferably a hydrogen atom.

R^{5a} is preferably a hydrogen atom.

R^{5b} is preferably a hydrogen atom.

R^{5c} is preferably a hydrogen atom.

R^{6a} is preferably a hydrogen atom.

R^{6b} is preferably a hydrogen atom.

R^{6c} is preferably a hydrogen atom.

R⁷ is preferably a C₁₋₆ alkyl group which may be substituted or a benzyl group which may be substituted, more preferably a C₁₋₆ alkyl group which may be substituted with one or more substituents selected from the substituent group A or a benzyl group which may be substituted with one or more substituents selected from the substituent group A, even more preferably a C₁₋₆ alkyl group which may be substituted with a halogen atom or a benzyl group which may be substituted with one or more groups selected from a halogen atom, a nitro group, and a C₁₋₆ alkoxy group, and particularly preferably a C₁₋₆ alkyl group which may be substituted with a halogen atom.

R8 is preferably a C₂₋₆ alkyl group which may be substituted, a C₂₋₆ alkyl group which may be substituted with one or more substituents selected from the substituent group A, or a benzyl group which may be substituted with one or more substituents selected from the substituent group A, even more preferably a C₂₋₆ alkyl group which may be substituted with a halogen atom or a benzyl group which may be substituted with one or more groups selected from a halogen atom, a nitro group, and a C₁₋₆ alkoxy group, and still more preferably a C₂₋₆ alkyl group.

R⁹ is preferably a hydrogen atom or a group represented by Formula [20].

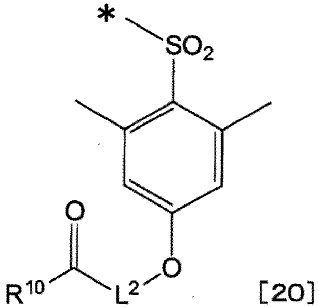

(In the formula, *, R¹⁰, and L² have the same definition as *, R¹⁰, and L² described above.)

In a case where R⁹ is an amino-protecting group, the amino-protecting group is preferably a C₁₋₆ alkyl group which may be substituted with one or more substituents selected from the substituent group A, a C₁₋₆ alkoxycarbonyl group which may be substituted with one or more substituents selected from the substituent group A, or an Ar C₁₋₆ alkoxycarbonyl group which may be substituted with one or more substituents selected from the substituent group A, and more preferably a benzyl group, a tert-butoxycarbonyl group, a benzyloxycarbonyl group, or a 9-fluorenylmethyloxycarbonyl group.

L¹ is preferably a group represented by Formula [18a].

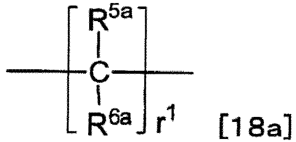

(In the formula, R^{5a}, R^{6a}, and r¹ have the same definition as R^{5a}, R^{6a}, and r¹ described above.)

L² is preferably a group represented by Formula [18b].

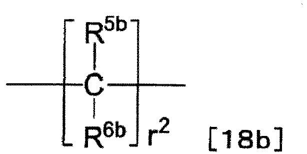

(In the formula, R^{5b}, R^{6b}, and r² have the same definition as R^{5b}, R^{6b}, and r² described above.)

L³ is preferably a group represented by Formula [18c].

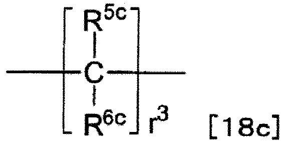

(In the formula, R^{5c}, R^{6c}, and r³ have the same definition as R^{5c}, R^{6c}, and r³ described above.)

A¹ is preferably a group represented by Formula [4] or [5].

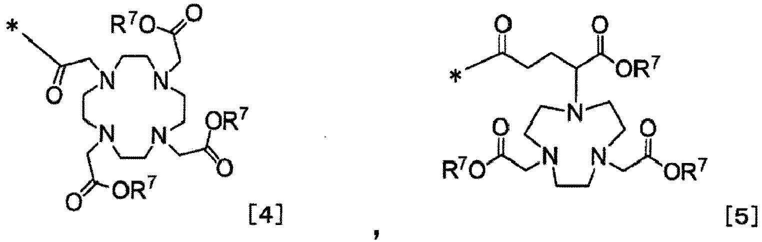

(In the formula, * and R⁷ have the same definition as * and R⁷ described above.)

A² is preferably a group represented by Formula [12] or [13].

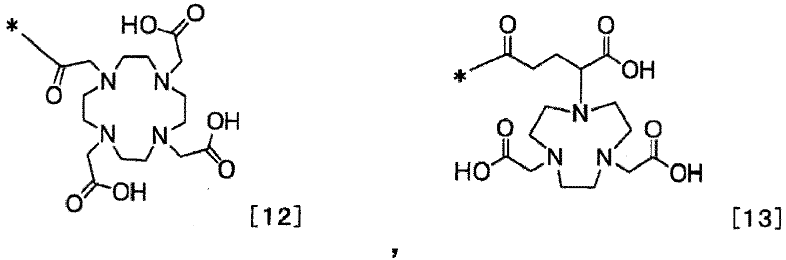

(In the formula, * has the same definition as ^{∗}described above.)
m is preferably 1 or 2.
p¹ is preferably 1 or 2.
p² is preferably 1 or 2.
p³ is preferably 1 or 2.
q¹ is preferably 0 or 1 and more preferably 0.
q² is preferably 0 or 1 and more preferably 0.
q³ is preferably 0 or 1 and more preferably 0.
r¹ is preferably an integer of 3 to 5, more preferably 3 or 4, and even more preferably 4.
r² is preferably an integer of 2 to 4, more preferably 3 or 4, and even more preferably 3.
r³ is preferably an integer of 2 to 4, more preferably 2 or 3, and even more preferably 2.

As the non-cytotoxic radioactive metal, from the viewpoint of the half-life, the radiation energy, the ease of a labeling reaction, and the like, a ¹⁸F aluminum complex, ¹¹¹In, ⁶⁷Ga, ⁶⁸Ga, ⁶⁴Cu, and ⁸⁹Zr are preferable.

As the cytotoxic radioactive metal, from the viewpoint of the half-life, the radiation energy, the ease of a labeling reaction, and the stability of the complex, ⁶⁴Cu, ⁶⁷Cu, ⁹⁰Y, ¹⁵³Sm, ¹⁶⁶Ho, ¹⁷⁷Lu, and ²²⁵Ac are preferable.

Next, the manufacturing method of the present invention will be described.

### Manufacturing method 1

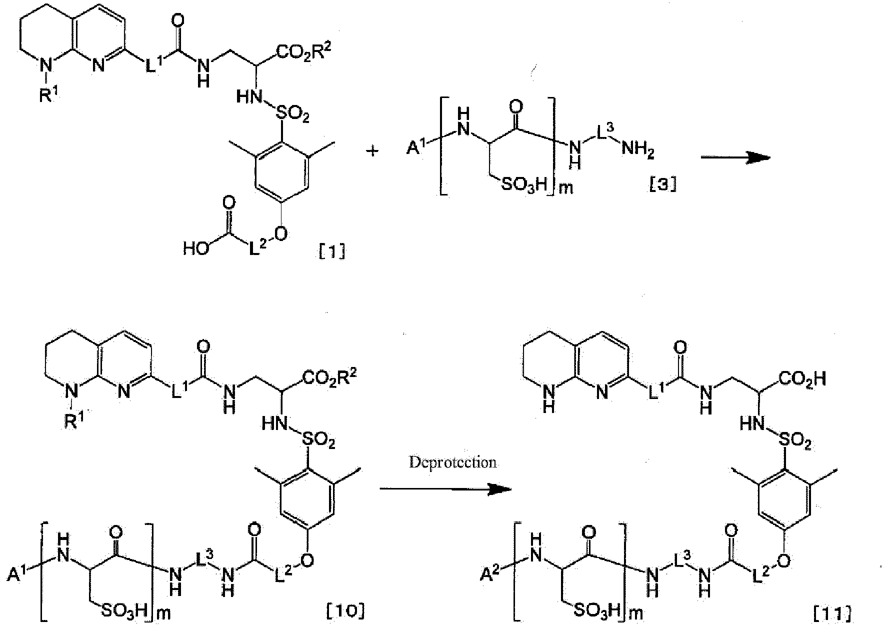

(In the formulae, R¹, R², L¹, L², L³, A¹, A², and m have the same definition as R¹, R², L¹, L², L³, A¹, A², and m described above).

(1)
By performing a reaction between the compound represented by Formula [1] and the compound represented by Formula [3] in the presence of a condensing agent and in the presence or absence of a base, the compound represented by Formula [10] can be manufactured.

This reaction can be performed by the methods described, for example, in Bioconjugate Chem. Vol. 3, p. 2, 1992, Chemical Reviews, Vol. 97, p. 2243, 1997, and the like.

The solvent used in this reaction is not particularly limited as long as the solvent does not affect the reaction. Examples of the solvent include ethers, esters, halogenated hydrocarbons, nitriles, amides, alcohols, and water, and these solvents may be used by being mixed together. As the solvent, amides are preferable, and N,N-dimethylformamide, N,N-dimethylacetamide, and N-methylpyrrolidone are more preferable.

The amount of the solvent used is not particularly limited, and may be greater than the amount of the compound represented by Formula [1] by a factor of 1 to 1,000 (v/w).

Examples of the base that is used as desired in this reaction include an inorganic base and an organic base. As the base, an organic base is preferable, and triethylamine or N,N-diisopropylethylamine is more preferable.

The amount of the base used may be greater than the amount of the compound represented by Formula [1] by a factor of 1 to 50 in terms of mole, and preferably greater than the amount of the compound by a factor of 1 to 10 in terms of mole.

Examples of the condensing agent used in this reaction include carbodiimides such as N,N'-dicyclohexylcarbodiimide and 1-ethyl-3-(3-dimethylaminopropyl)carbodiimide; carbonyls such as carbonyldiimidazole; acid azides such as diphenylphosphoryl azide; acid cyanides such as diethylphosphoryl cyanide; active carbamates such as 2-ethoxy-1-ethoxycarbonyl-1,2-dihydroquinoline; ureas such as O-benzotriazol-1 -yl-1,1,3,3 -tetramethyluronium=hexafluorophosphate and O-(7-azabenzotriazol-1-yl)-1,1,3,3-tetramethyluronium=hexafluorophosphate; a phosphonium salt such as benzotriazol-1-yloxy-trisdimethylaminophosphonium hexafluorophosphate and (benzotriazol-1-yloxy)tripyrrolidinophosphonium hexafluorophosphate; and the like. As the condensing agent, carbodiimides or ureas are preferable, and 1-ethyl-3-(3-dimethylaminopropyl)carbodiimide, O-benzotriazol-1-yl-1,1,3,3-tetramethyluronium=hexafluorophosphate, and O-(7-azabenzotriazol-1-yl)-1,1,3,3-tetramethyluronium=hexafluorophosphate are more preferable.

As a condensing method, after the compound represented by Formula [1] and the compound represented by Formula [3] are mixed together, the condensing agent may be added. As another method, after being activated in advance by the condensing agent, the compound represented by Formula [1] may be reacted with the compound represented by Formula [3]. Furthermore, it is possible to use an active ester such as N-hydroxysuccinimide or pentafluorophenol.

The amount of the compound represented by Formula [3] used is not particularly limited, and may be greater than the amount of the compound represented by Formula [1] by a factor of 0.5 to 10 in terms of mole.

The reaction temperature may be -30°C to 100°C, and is preferably 0°C to 50°C.

The reaction time may be 1 minute to 72 hours.

(2)
By deprotecting the compound represented by Formula [10], the compound represented by Formula [11] can be manufactured.

This reaction can be performed, for example, by the method described in T. W. Greene et al., Protective Groups in Organic Synthesis, 4th edition, pp. 696~926, 2007, John Wiley & Sons, INC.

As the method for deprotecting the compound represented by Formula [10], by deprotecting the compound by using an acid, the decrease of optical purity of the compound represented by Formula [11] can be inhibited.

Examples of the acid include hydrochloric acid, hydrobromic acid, sulfuric acid, methanesulfonic acid, acetic acid, formic acid, p-toluenesulfonic acid, trifluoromethanesulfonic acid, trifluoroacetic acid, and the like. Among these, hydrochloric acid, formic acid, and trifluoroacetic acid are preferable.

The amount of the acid used may be equal to or greater than the amount of the compound represented by Formula [10] by a factor of 1 (w/w), and is preferably greater than the amount of the compound by a factor of 1 to 100 (w/w). The acid may be used singly as a solvent, or may be used by being diluted with a solvent that does not affect the reaction.

### Manufacturing method 2

A complex of the compound represented by Formula [11] or a salt thereof and a metal can be manufactured as below, for example.

By mixing the compound represented by Formula [11] or a salt thereof with a metal ion in the presence of a buffer solution, the complex can be manufactured.

The buffer solution used in this reaction is not particularly limited as long as the buffer solution does not affect the reaction. Examples of the buffer solution include a sodium acetate buffer solution, an ammoniuim acetate buffer solution, a sodium citrate buffer solution, and an ammonium citrate buffer solution.

The pH of the buffer solution is preferably within a range of 3 to 6.

The reaction temperature and the reaction time vary with the combination of the compound represented by Formula [11] or a salt thereof and a radioactive metal, but may be 0°C to 150°C and 5 to 60 minutes respectively.

The complex obtained by the aforementioned manufacturing method can be isolated and purified by a general method such as extraction, crystallization, distillation, or column chromatography.

In a case where a radioactive metal is used as a metal, the complex can also be manufactured based on the aforementioned manufacturing method. Considering the fact that the radioactive metal emits radiation and the fact that the radioactive metal is a trace metal, attention needs to be paid to the following points.

It is not preferable to unnecessary prolong the reaction time, because the compound is likely to be decomposed due to radiation. Generally, a labeled compound can be obtained at a radiochemical yield of greater than 80%. However, in a case where higher purity is required, the compound can be purified by a method such as preparative liquid chromatography, preparative TLC, dialysis, solid phase extraction, and/or ultrafiltration.

Furthermore, by regarding a metal fluoride complex, which is a combination of a fluoride and a metal, as a metal, a complex can be manufactured by performing a reaction between the metal fluoride complex and the compound represented by Formula [11] or a salt thereof. This reaction can be performed, for example, by the method described in JP5388355A.

In order to inhibit the decomposition caused by radiation, it is preferable to add an additive such as gentisic acid, ascorbic acid, benzyl alcohol, tocopherol, gallic acid, a gallic acid ester, or α-thioglycerol.

Next, the method for manufacturing raw materials for manufacturing will be described.

### Manufacturing method A

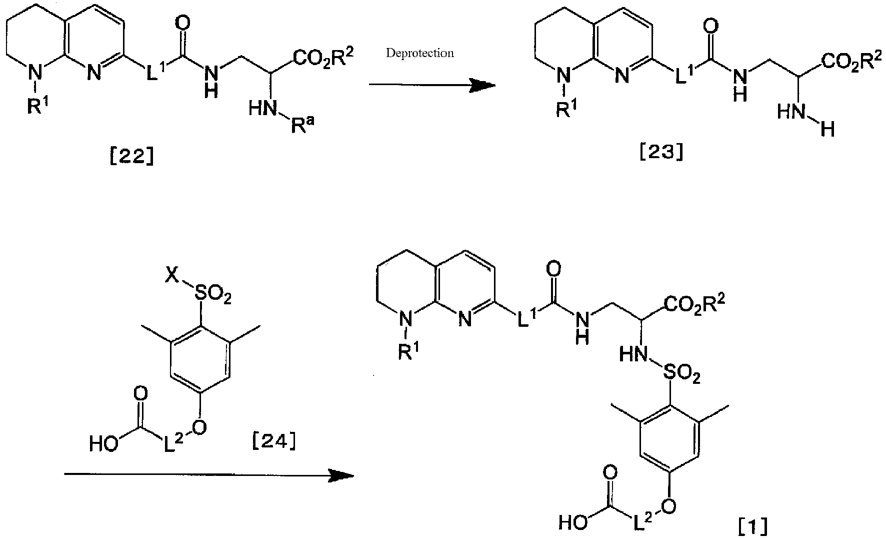

(In the formulae, X represents a halogen atom; R^{a} represents an amino-protecting group; and R¹, R², L¹, and L² have the same definition as R¹, R², L¹, and L² described above.)

As the compound represented by Formula [24], for example, 4-(4-(chlorosulfonyl)-3,5-dimethylphenoxy)butanoic acid is known.
(1)
   By deprotecting the protecting group R^{a} of the compound represented by Formula [22], the compound represented by Formula [23] can be manufactured.

This reaction may be performed based on (2) of Manufacturing method 1, under the condition in which the amino-protecting group R¹ and the carboxyl-protecting group R² are not simultaneously deprotected. For example, in a case where the amino-protecting group R¹ and the carboxyl-protecting group R² are protecting groups that can be deprotected under the acidic conditions, as R^{a}, a protecting group such as benzyloxycarbonyl group that can be deprotected through hydrogenation reduction under the neutral conditions or a protecting group such as 9-fluorenyloxycarbonyl group that can be deprotected under the basic conditions is selected, and treated under the neutral conditions or the basic conditions.

(2) By performing a reaction between the compound represented by Formula [23] and the compound represented by Formula [24] in the presence of a base, the compound represented by Formula [1] can be manufactured.

The solvent used in this reaction is not particularly limited as long as the solvent does not affect the reaction. Examples of the solvent include ethers, esters, halogenated hydrocarbons, nitriles, and amides. These solvents may be used by being mixed together. As the solvent, halogenated hydrocarbons and ethers are preferable, and methylene chloride and tetrahydrofuran are more preferable.

The amount of the solvent used is not particularly limited, and may be greater than the amount of the compound represented by Formula [23] by a factor of 1 to 1,000 (v/w).

Examples of the base used in this reaction include an inorganic base and an organic base. As the base, sodium hydrogen carbonate, sodium carbonate, potassium carbonate, and N-methylimidazole are preferable, and sodium hydrogen carbonate and sodium carbonate are more preferable.

The amount of the base used may be greater than the amount of the compound represented by Formula [23], by a factor of 1 to 50 in terms of mole, and is preferably greater than the amount of the compound by a factor of 1 to 10 in terms of mole.

The amount of the compound represented by Formula [24] used is not particularly limited. The amount may be greater than the amount of the compound represented by Formula [23] by a factor of 1 to 50 in terms of mole, and is preferably greater than the amount of the compound by a factor of 1 to 10 in terms of mole.

The reaction temperature may be -30°C to 100°C, and is preferably 0°C to 50°C.

The reaction time is preferably 1 minute to 72 hours.

### Manufacturing method Aa

In a case where R¹ is a hydrogen atom, the compound represented by formula [22] is a compound represented by Formula [27].

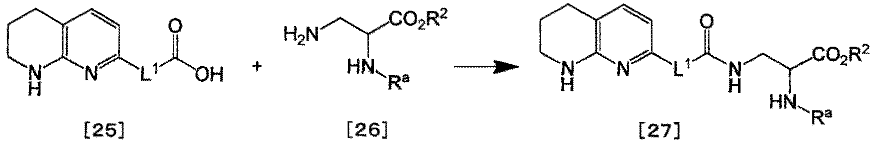

(In the formulae, R^{a}, R², and L¹ have the same definition as R^{a}, R², and L¹ described above.)

As the compound represented by Formula [25], for example, 5-(5,6,7,8-tetrahydro-1,8-naphthyridin-2-yl)pentanoic acid is known.

As the compound represented by Formula [26], for example, (S)-tert-butyl 3-amino-2-(((benzyloxy)carbonyl)amino)propanoate is known.

By performing a reaction between the compound represented by Formula [25] and the compound represented by Formula [26] in the presence of a condensing agent and in the presence or absence of a base, the compound represented by Formula [27] can be manufactured.

This reaction may be performed based on (1) of Manufacturing method 1.

### Manufacturing method Ab

In a case where R¹ is an amino-protecting group, the compound represented by Formula [22] is a compound represented by Formula [31].

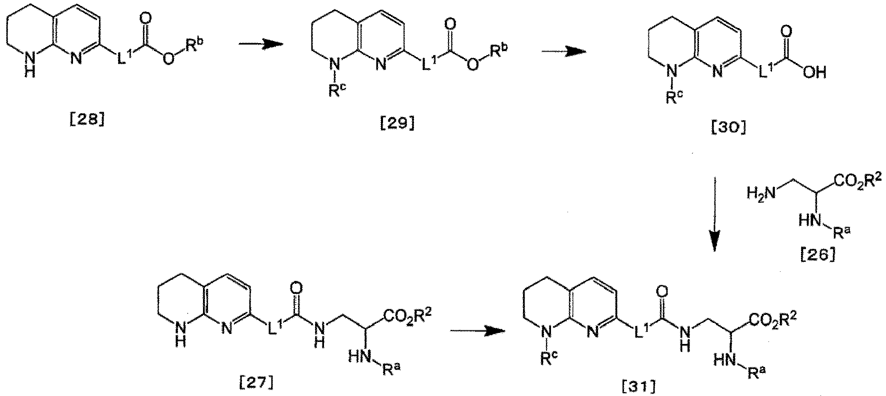

(In the formulae, R^{b} represents a carboxyl-protecting group, R^{c} represents an amino-protecting group, and R^{a}, R², and L¹ have the same definition as R^{a}, R², and L¹ described above.)

As the compound represented by Formula [28], for example, ethyl 5-(5,6,7,8-tetrahydro-1,8-naphthyridin-2-yl)pentanoate and methyl 5-(5,6,7,8-tetrahydro-1,8-naphthyridin-2-yl)pentanoate are known.

The compound represented by Formula [31] can be manufactured from the compound represented by Formula [28].
(1) By protecting an amino group of a 1,2,3,4-tetrahydro-1,8-naphthyridinyl group of the compound represented by Formula [28], the compound represented by Formula [29] can be manufactured.

R^{c} is preferably a C₁₋₆ alkoxycarbonyl group which may be substituted, a heterocyclic sulfonyl group which may be substituted, or an arylsulfonyl group which may be substituted, more preferably a C₁₋₆ alkoxycarbonyl group which may be substituted with one or more substituents selected from the substituent group A, a heterocyclic sulfonyl group which may be substituted with one or more substituents selected from the substituent group A, or an arylsulfonyl group which may be substituted with one or more substituents selected from the substituent group A, and even more preferably a C₁₋₆ alkoxycarbonyl group, a 2,2,4,6,7-pentamethyldihydrobenzofuran-5-sulfonyl group, or a 2,2,5,7,8-pentamethylchromane-6-sulfonyl group.

In a case where R^{c} is a 2,2,4,6,7-pentamethyldihydrobenzofuran-5-sulfonyl group or a 2,2,5,7,8-pentamethylchromane-6-sulfonyl group, R^{c} can be selectively deprotected.

(2) By deprotecting the protecting group R^{b} of the compound represented by Formula [29], a compound represented by Formula [30] can be manufactured.

This reaction may be performed based on (2) of Manufacturing method 1, under the conditions in which the protecting group R^{c} is not simultaneously deprotected. For example, in a case where the protecting group R^{c} is a C₁₋₆ alkoxycarbonyl group, a C₁₋₆ alkylsulfonyl group, an arylsulfonyl group, or a heterocyclic sulfonyl group, the compound represented by Formula [30] can be manufactured by alkaline hydrolysis.

(3) By performing a reaction between the compound represented by Formula [30] and the compound represented by Formula [26] in the presence of a condensing agent and in the presence or absence of a base, the compound represented by Formula [31] can be manufactured.

This reaction may be performed based on (1) of Manufacturing method 1.

As another method, by protecting an amino group of the compound represented by Formula [27], the compound represented by Formula [31] can be manufactured.

This reaction may be performed based on (1) of Manufacturing method Ab.

### Manufacturing method B

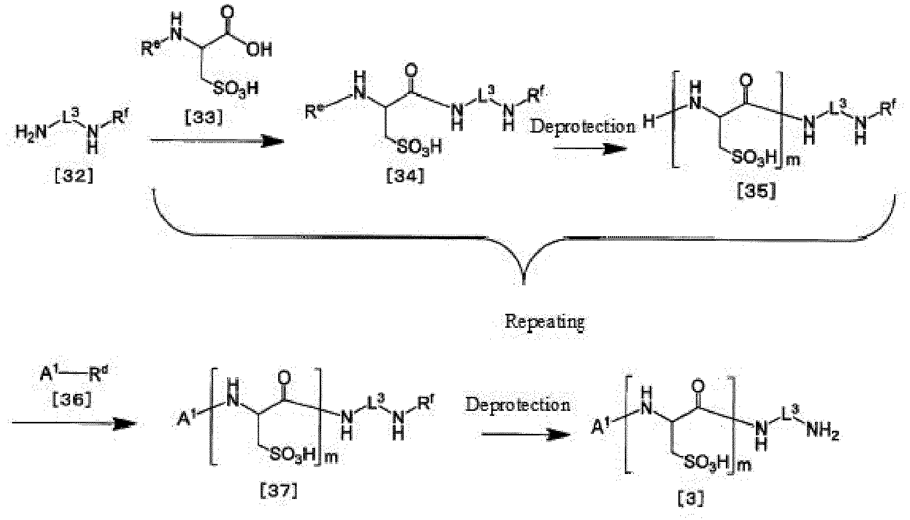

(In the formulae, R^{d} represents a hydroxyl group or a leaving group; R^{e} represents an amino-protecting group; R^{f} represents an amino-protecting group; and L³, A¹, and m have the same definition as L³, A¹, and m described above.)

As the compound represented by Formula [32], for example, benzyl (2-aminoethyl)carbamate is known.

As the compound represented by Formula [33], for example, (R)-2-((((9H-fluoren-9-yl)methoxy)carbonyl)amino)-3-sulfopropanoic acid is known.
(1)
   By performing a reaction between the compound represented by Formula [32] and the compound represented by Formula [33] in the presence of a base, the compound represented by Formula [34] can be manufactured.

This reaction may be performed based on (1) of Manufacturing method 1.

(2)
The compound represented by Formula [35] can be manufactured by deprotecting the protecting group R^{e} of the compound represented by Formula [34].

This reaction may be performed based on (2) of Manufacturing method 1.

In a case where m is 2 or 3, by repeating an operation of reacting the compound represented by Formula [34] with the compound represented by Formula [33] and then deprotecting the protecting group R^{e}, the compound represented by Formula [35] can be manufactured.

(3)
The compound represented by Formula [36] is a compound known as a bifunctional chelate.

As the compound represented by Formula [36], for example, 1,4,7,10-tetraazacyclododecane-1,4,7,10-tetraacetic tri-tert-butyl ester (DOTA) having a protected carboxyl group and ((R)-4-(4,7-bis(2-(tert-butoxy)-2-oxoethyl)-1,4,7-triazonan-1-yl)-5-(tert-butoxy)-5-oxopentano ic acid (NODAGA) having a protected carboxyl group are known.

In a case where R^{d} in Formula [36] is a hydroxyl group, by performing a reaction between the compound represented by Formula [35] and the compound represented by Formula [36] in the presence of a condensing agent and in the presence or absence of a base, the compound represented by Formula [37] can be manufactured.

In a case where R^{d} in Formula [36] is an active ester of a succinimide oxide group or the like, by performing a reaction between the compound represented by Formula [35] and the compound represented by Formula [36] in the presence or absence of a base, the compound represented by Formula [37] can be manufactured.

This reaction may be performed based on (1) of Manufacturing method 1.

(4) By deprotecting the protecting group R^{f} of the compound represented by Formula [37], the compound represented by Formula [3] can be manufactured.

This reaction may be performed based on (2) of Manufacturing method 1.

By being subjected to a known reaction such as condensation, addition, oxidation, reduction, transposition, substitution, halogenation, dehydration, or hydrolysis or by being subjected to a reaction performed by appropriately combining the above reactions, the compounds obtained by the aforementioned manufacturing methods can be induced into other compounds.

The compounds obtained by the aforementioned manufacturing methods can be isolated and purified by a general method such as extraction, crystallization, distillation, or column chromatography. Furthermore, the compounds obtained by the aforementioned manufacturing methods may be used as they are for the next reaction without being isolated.

In a case where an amino, hydroxyl, or carboxyl group is present in the compounds obtained by the aforementioned manufacturing methods and intermediates thereof, the reaction can be performed by appropriately recombining the protecting groups of these. In a case where there are two or more protecting groups, the protecting groups can be selectively deprotected by being subjected to a known reaction.

Among the compounds used in the aforementioned manufacturing methods, the compound that can take a salt form can be used as a salt.

In a case where the compounds used in the aforementioned manufacturing methods have isomers (for example, an optical isomer, a geometric isomer, and a tautomer), these isomers can also be used. Furthermore, in a case where there are a solvate, a hydrate, and crystals of various shapes, these solvate, hydrate, and crystals of various shapes can also be used.

### Examples

Next, the present invention will be more specifically described based on reference examples and examples, but the present invention is not limited thereto.

Unless otherwise specified, as a carrier for silica gel column chromatography, 63 to 210 µm of silica gel 60N (spherical/neutral) (manufactured by KANTO KAGAKU) was used.

The mixing ratio in an eluent is a volume ratio.

For example, "hexane/ethyl acetate = 90/10 to 50/50" means that an eluent of "hexane:ethyl acetate = 90:10" was changed to an eluent of "hexane:ethyl acetate = 50:50".

¹H-NMR spectra were measured using Bruker A V300 (manufactured by Bruker) or JEOL JNM-AL400 model (JEOL) by using tetramethylsilane as internal standard, and δ values were described using ppm.

Unless otherwise specified, HPLC analysis was performed using Nexera HPLC System (Shimadzu Corporation) (column: TSKgel ODS-100Z (Tosoh Corporation), 4.6 × 150 mm, column: GL Intertsustain C18 (GL Sciences Inc.), 4.6 × 150 mm, or column: Waters BEH C18 (WATERS), 2.1 × 100 mm), solvent: (formic acid-based) A solution = formic acid:water (1:1000), B solution = formic acid: methanol: acetonitrile (1:800:200), (ammonium acetate-based) A solution = 5 mM aqueous ammoniuim acetate solution, B solution = 5 mM aqueous ammonium acetate solution:methanol:acetonitrile (5:36:9) or (TFA-based) A solution = TFA: water: acetonitrile (1:900:100), B solution = TFA: water: acetonitrile (1:100:900), gradient cycle: 0 min (A solution/B solution = 90/10), 30 min (A solution/B solution = 0/100), flow rate: 1.0 mL/min). The retention time (min) was described using rt (min). In a case where the analysis conditions are different, the conditions are described in reference examples or examples.

Unless otherwise specified, the preparative HPLC was performed using Waters 600E system (Waters) (column:SunFire PrepC18OBD 30 × 150 mm (Waters) or SunFire PrepC18OBD 19 × 150 mm (Waters), solvent: A solution = formic acid:water (1:1,000), B solution = formic acid:methanol:acetonitrile (1:800:200) or a solvent:A solution = 10 mM aqueous ammonium acetate solution, B solution = 10 mM aqueous ammonium acetate solution:methanol:acetonitrile (10:800:200)).

Unless otherwise specified, for TLC analysis, silica gel 6OF₂₅₄ (Merck) or RP-18F₂₅₄ (Merck) was used.

The MS and LC/MS analyses were performed using an ACQUITY SQD LC/MS System (Waters) (column: BEHC18 2.1 × 30 mm (Waters), A solution = 0.1% formic acid/water, B solution = 0.1% formic acid/acetonitrile, gradient cycle: 0 min (A solutiln/B solution = 95/5), 2 min (A solution/B solution = 5/95), 3 min (A solution/B solution = 5/95), flow rate: 0.5 mL/min). The retention time (min) was described using rt (min), and ESI positive and negative ion peaks were detetcted.

Each abbreviation means the following.
Boc: tert-butoxycarbonyl
(BOC)₂O: di-tert-butyl dicarbonate
^{t}BU: tert-butyl
DIEA: N,N-diisopropylethylamine
DMAc: N,N-diemthylacetamide
DMF: N,N-dimethylformamide
Et: ethyl
Fmoc: 9-fluorenylmethyloxycarbonyl
HBTU: 0-benaotriazol-1-yl 1,1,3,3-tetramethyluronium hexafluorophosphate
IPA: 2-propanol
Me: methyl
NMP: N-methylpyrrolidone
TBME: tert-butylmethylether
TFA: trifluoroacetic acid
THF: tetrahydrofuran
Z: benzyloxycarbonyl

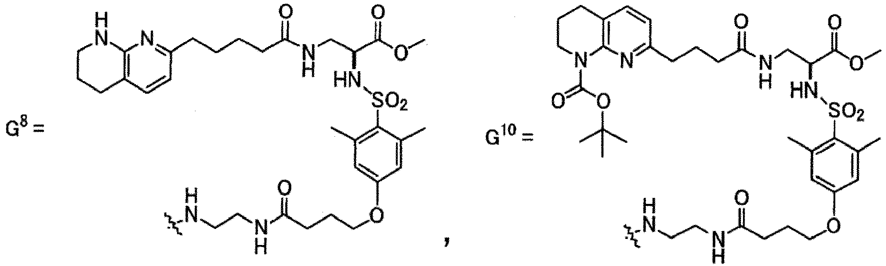

### Reference Example 1

(1)

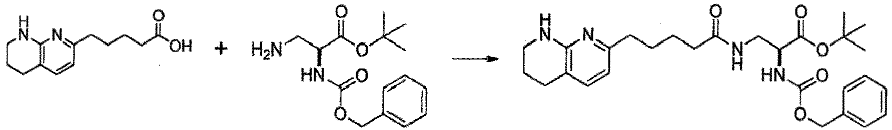

HBTU (67.9 g) was added in 5 divided portions at an interval of 10 minutes to a DMAc solution (500 mL) of 5-(5,6,7,8-tetrahydro-1,8-naphthyridin-2-yl)pentanoic acid (41.9 g), (S)-tert-butyl 3-amino-2-(((benzyloxy)carbonyl)amino)propanoate (50.0 g), and DIEA (57.8 mL), followed by stirring for 2 hours at room temperature. An aqueous saturated sodium hydrogen carbonate solution (50 mL) was added thereto, followed by stirring for 10 minutes. Then, an aqueous saturated sodium hydrogen carbonate solution (200 mL) was further added thereto, followed by stirring for 30 minutes. Ethyl acetate (300 M1) was added thereto, followed by stirring for 10 minutes. Thereafter, insoluble matter was removed by filtration, and the resultant was washed twice with ethyl acetate (100 mL). The organic layer was washed twice with an aqueous saturated sodium hydrogen carbonate solution (250 mL) and then twice with an aqueous saturated sodium chloride solution (100 mL). The organic layer was dried over anhydrous sodium sulfate, and the solvent was distilled away under reduced pressure. Ethyl acetate (300 mL) and hexane (170 mL) were added to the obtained residue, and the solution was stirred overnight so as to precipitate a solid. Hexane (430 mL) was then added thereto, followed by stirring for 2 hours at room temperature. The solid was collected by filtration, thereby obtaining (S)-tert-butyl 2-(((benzyloxy)carbonyl)amino)-3-(5-(5,6,7,8-tetrahydro-1,8-naphthyridin-2-yl)pentanamide) propanoate (69.0 g) as a light yellow solid.

MS (ESI, m/z): 511 [M + H]⁺

¹H-NMR (CDCl₃) δ: 7.38-7.27 (5H, m),7.05 (1H, d, J = 7.3 Hz),6.33 (1H, d, J = 7.3 Hz), 6.15-6.03 (2H, m), 6.01 (2H, brd, J = 5.9 Hz), 5.10 (2H, s), 4.95-4.82 (1H, m),4.31 (1H, dt, J = 5.9 Hz, 5.9 Hz), 3.64 (2H, t, J = 5.9 Hz), 3.42-3.31 (2H, m), 2.67 (2H, t, J = 6.3 Hz), 2.58-2.46 (2H, m), 2.23-2.11 (2H, m), 1.99-1.81 (2H, m), 1.73-1.59 (4H, m), 1.45 (9H, s)

HPCL (TSKgel ODS-100Z, formic acid-based) rt (min): 15.69

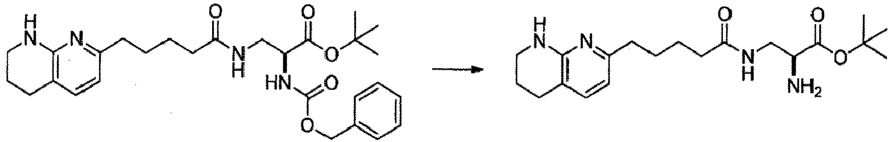

Methanol (25 mL) and 10% palladium on carbon (0.250 g) were added to (S)-tert-butyl 2-(((benzyloxy)carbonyl)amino)-3-(5-(5,6,7,8-tetrahydro-1,8-naphthyridin-2-yl)pentanamide) propanoate (5.00 g), followed by stirring for 1 hour in a hydrogen atmosphere at 0.4 MPa. The insoluble matter was removed by filtration, and methanol was distilled away under reduced pressure. An operation of adding acetonitrile (10 ml) to the residue and distilling away the solvent under reduced pressure was repeated twice, thereby obtaining (S)-tert-butyl 2-amino-3-(5-(5,6,7,8-terrahydro-1,8-naphthyridin-2-yl)pentanamide)propanoate (3.83 g) as a light yellow oily substance.

MS (ESI, m/z): 377 [M + H]⁺

¹H-NMR (CDCl₃) δ: 7.04 (d, J = 7.3 Hz, 1H), 6.33 (d, J = 7.3 Hz, 1H), 6.26-6.13 (m, 1H), 5.01-4.88 (m, 1H), 3.70-3.57 (m, 1H), 3.51-3.44 (m, 1H), 3.43-3.35 (m, 2H), 3.32-3.20 (m, 1H), 2.73-2.63 (m, 2H), 2.60-2.50 (m, 2H), 2.26-2.15 (m, 2H), 1.96-1.84 (m, 2H), 1.80-1.61 (m, 6H), 1.46 (s, 9H)

HPCL (TSKgel ODS-100Z, formic acid-based) rt (min): 21.41

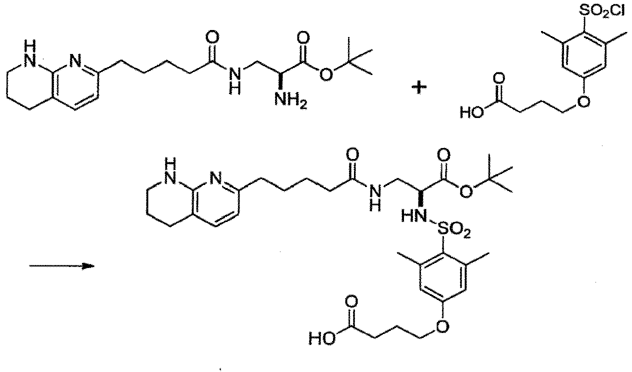

4-(4-(chlorosulfonyl)-3,5-dimethylphenoxy)butanoic acid (0.670 g) was added to a methylene chloride (20 mL) suspension of (S)-tert-butyl 2-amino-3-(5-(5,6,7,8-terrahydro-1,8-naphthyridin-2-yl)pentanamide)propanoate (1.99 g) and sodium hydrogen carbonate (1.23 g) with ice cooling, followed by stirring for 30 minutes. Then, 4-(4-(chlorosulfonyl)-3,5-dimethylphenoxy)butanoic acid (0.340 g) was added thereto, followed by stirring for 30 minutes. Thereafter, 4-(4-(chlorosulfonyl)-3,5-dimethylphenoxy)butanoic acid (0.340 g) was further added thereto, followed by stirring for 1 hour. The solution was stirred for 14 hours at room temperature, and the solvent was distilled away under reduced pressure. Ethyl acetate (30 mL) and water (30 mL) were added to the obtained residue, the solution was stirred, and sodium carbonate (3.0 g) was added thereto so as to adjust the pH to be 9.6. Liquid separation was performed by adding water (50 mL) and ethyl acetate (50 mL), and the aqueous layer was washed with ethyl acetate (40 mL). Acetonitrile (80 mL) and ammonium chloride (30 g) were added to the aqueous layer, followed by stirring. Then, the organic layer was separated, the aqueous layer was extracted using acetonitrile (40 mL), and the entirety of the organic layer was dried over anhydrous sodium sulfate. The solvent was distilled away under reduced pressure, thereby obtaining (S)-4-(4-(N-(1-(tert-butoxy)-1-oxo-3-(5-(5,6,7,8-tetrahydro-1,8-naphthyridin-2-yl)pentanamid e)propan-2-yl)sulfamoyl)-3,5-dimethylphenoxy)butanoic acid (2.58 g) as a yellow amorphous solid.

MS (ESI, m/z): 647 [M + H]⁺

¹H-NMR (CDCl₃) δ: 7.24 (1H, d, J = 7.3 Hz), 6.67 (2H, s), 6.27 (1H, d, J = 7.3 Hz), 6.08-5.86 (1H, m), 5.85-5.62 (1H, m), 4.17-3.99 (2H, m), 3.93-3.83 (1H, m), 3.58-3.25 (4H, m), 2.80-2.56 (11H, m), 2.54-2.42 (2H, m), 2.19-2.05 (2H, m), 2.01-1.76 (4H, m), 1.73-1.45 (4H, m), 1.40 (9H, s)

HPCL (TSKgel ODS-100Z, formic acid-based) rt (min): 15.90

### Reference Example 2

(1)

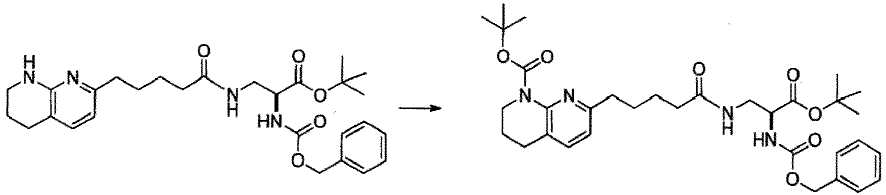

(BOC)₂O (3.1 mL) was added to a mixture of (S)-tert-butyl 2-(((benzyloxy)carbonyl)amino)-3-(5-(5,6,7,8-tetrahydro-1,8-naphthyridin-2-yl)pentanamide) propanoate (2.3 g), THF (25 mL), and DIEA (2.4 mL), and the mixture was heated for 8 hours under reflux. The solvent was distilled away under reduced pressure, and the resultant was purified by silica gel column chromatography (hexane/ethyl acetate = 1/1), thereby obtaining (S)-tert-butyl 7-(5-((2-(((benzyloxy)carbonyl)amino)-3-(tert-butoxy)-3-oxopropyl)amino)-5-oxopentyl)-3,4-dihydro-1,8-naphthyridin-1(2H)-carboxylate (1.86 g) as a light yellow oily substance.

LC/MS rt (min): 1.37

MS (ESI, m/z): 611.4 [M + H]⁺

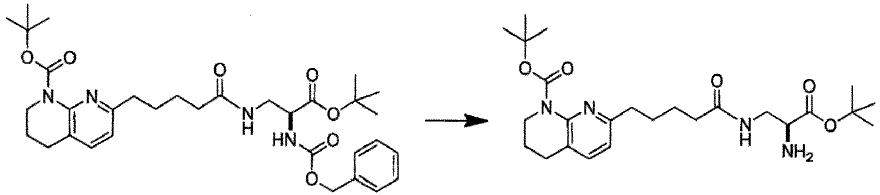

(S)-tert-butyl 7-(5-((2-(((benzyloxy)carbonyl)amino)-3-(tert-butoxy)-3-oxopropyl)amino)-5-oxopentyl)-3,4-dihydro-1,8-naphthyridin-1(2H)-carboxylate (750 mg), 10% palladium on carbon (0.13 g), and methanol (30 mL) were put into a stainless steel tube and stirred for 4 hours in a nitrogen atmosphere at 0.5 MPa. The insoluble matter was removed by filtration, and the solvent was distilled away under reduced pressure, thereby obtaining (S)-tert-butyl 7-(5-((2-amino-3-(tert-butoxy)-3-oxopropyl)amino)-5-oxopentyl)-3,4-dihydro-1,8-naphthyridi n-1(2H)-carbonxylaate (617 mg) as a light yellow oily substance.

LC/MS rt (min): 0.79

MS (ESI, m/z): 477.3 [M + H]⁺

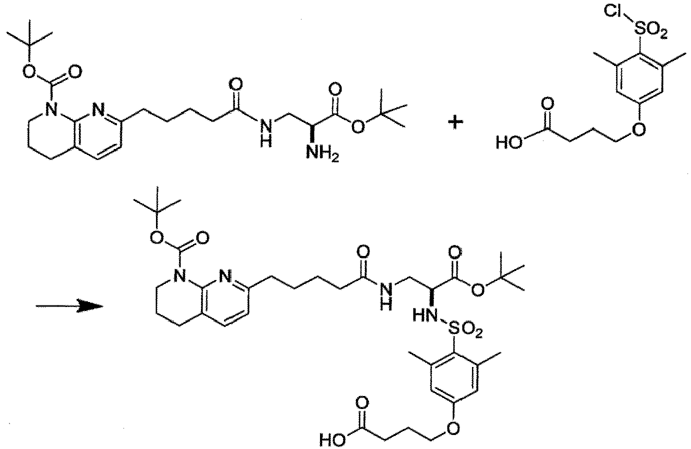

N-methylimidazole (0.5 mL) and 4-(4-(chlorosulfonyl)-3,5-dimethylphenoxy)butanoic acid (1.8 g) were added at 0°C to a THF (10 mL) solution of (S)-tert-butyl 7-(5-((2-amino-3-(tert-butoxy)-3-oxopropyl)amino)-5-oxopentyl)-3,4-dihydro-1,8-naphthyridi n-1(2H)-carbonxylaate (2.8 g), the mixture was stirred for 3 hours at 0°C, and the solvent was distilled away under reduced pressure. Ethyl acetate (30 mL) and water (30 mL) were added to the obtained residue, and the organic layer was fractionated, washed with an aqueous saturated sodium chloride solution (30 mL), and then dried over anhydrous sodium sulfate. The solvent was distilled away under reduced pressure, and the resultant was purified by silica gel column chromatography (diol silica (CHROMATOREX-DIOL, FUJI SILYSIA CHEMICAL LTD), hexane/ethyl acetate = 55/45 to 20/80), thereby obtaining (S)-4-(4-(N-(1-(tert-butoxy)-3-(5-(8-(tert-butoxycarbonyl)-5,6,7,8-tetrahydro-1,8-naphthyridin -2-yl)pentanamide)-1-oxopropan-2-yl)sulfamoyl)-3,5-dimethylphenoxy)butanoic acid (1.58 g) as a yellow amorphous solid.

LC/MS rt (min): 1.23

MS (ESI, m/z): 747.4 [M + H]⁺

¹H-NMR (CDCl₃) δ: 7.33 (1H, d, J = 7.9 Hz), 6.84 (1H, d, J = 7.9 Hz), 6.63 (2H, s), 5.75 (1H, t, J = 5.6 Hz), 5.64 (1H, d, J = 7.3 Hz), 4.17-3.97 (3H, m), 3.86-3.65 (3H, m), 3.54 -3.28 (2H, m), 2.77-2.68 (4H, m), 2.62 (6H, s), 2.51 (2H, t, J= 6.6 Hz), 2.15-1.86 (6H, m), 1.74-1.47 (6H, m), 1. 5 0 (9H, s), 1. 3 7 (9H, s)

### Reference Example 3

(1-A-1)

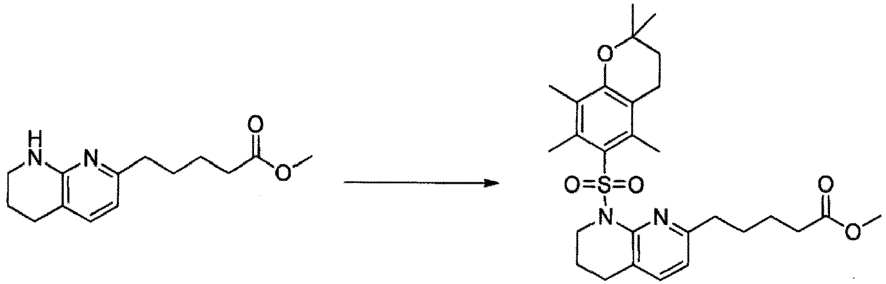

2,2,5,7,8-pentamethylchroman-6-yl)sulfonyl chloride (3.02 g) was added to a mixture of methyl 5-(5,6,7,8-tetrahydro-1,8-naphthyridine-2-yl)pentanoate (2.00 g), potassium carbonate (1.66 g), and acetonitrile (12 mL), followed by stirring for 1.5 hours at 70°C. By adding ethyl acetate (20 mL) and water (30 mL) thereto, the organic layer was fractionated. Thereafter, the organic layer was washed once with an aqueous saturated sodium hydrogen carbonate solution (20 mL) and then twice with an aqueous saturated sodium chloride solution (20 mL) and dried over anhydrous sodium sulfate, and the solvent was distilled away under reduced pressure. The obtained residue was purified by silica gel column chromatography (hexane/ethyl acetate = 90/10 to 75/25), thereby obtaining methyl 5-(8-((2,2,5,7,8-pentamethylchroman-6-yl)sulfonyl)-5,6,7,8-tetrahydro-1,8-naphthyridin-2-yl) pentanoate (2.87 g) as a light yellow foamy substance.

LC/MS rt (min): 2.06

MS (ESI, m/z): 515.5 [M + H]⁺

(1-A-2)

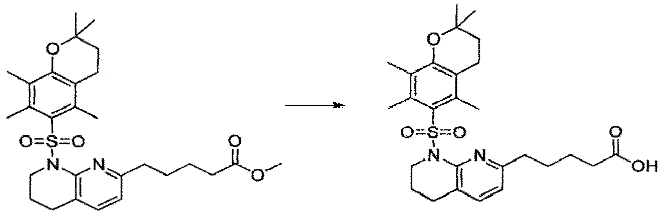

A 2.5 M aqueous sodium hydroxide solution (3 mL) and methanol (5 mL) were added to a mixture of methyl 5-(8-((2,2,5,7,8-pentamethylchroman-6-yl)sulfonyl)-5,6,7,8-tetrahydro-1,8-naphthyridin-2-yl) pentanoate (1.94 g), THF (10 mL), and water (1 mL), followed by stirring for 3 hours at room temperature. The solvent was distilled away under reduced pressure, and water (15 mL) and sodium hydrogen sulfate were added thereto so as to adjust the pH to be 4. By adding ethyl acetate (15 mL), the organic layer was fractionated. The organic layer was then washed with water (20 mL) and an aqueous saturated sodium chloride solution (20 mL) and dried over anhydrous sodium sulfate. The solvent was distilled away under reduced pressure, thereby obtaining 5-(8-((2,2,5,7,8-pentamethylchroman-6-yl)sulfonyl)-5,6,7,8-tetrahydro-1,8-naphthyridin-2-yl) pentanoic acid (2.00 g) as a colorless foamy substance.

LC/MS rt (min): 1.78

MS (ESI, m/z): 501.4 [M + H]⁺

¹H-NMR (CDCl₃) δ: 7.18 (1H, d, J = 7.2 Hz), 6.55 (1H, d, J = 7.1 Hz), 4.06-4.09 (2H, m), 2.75 (2H, t, J = 6.6 Hz), 2.64 (2H, t, J = 6.6 Hz), 2.59 (s, 3H), 2.53 (s, 3H), 2.35 (2H, t, J = 7.2Hz), 2.16 (2H, t, J = 7.2 Hz), 2.12 (3H, s), 2.02-2.08 (2H, m), 1.81 (2H, t, J =7.2 Hz), 1.33-1.47 (2H, m), 1.14-1.26 (2H, m)

(1-A-3)

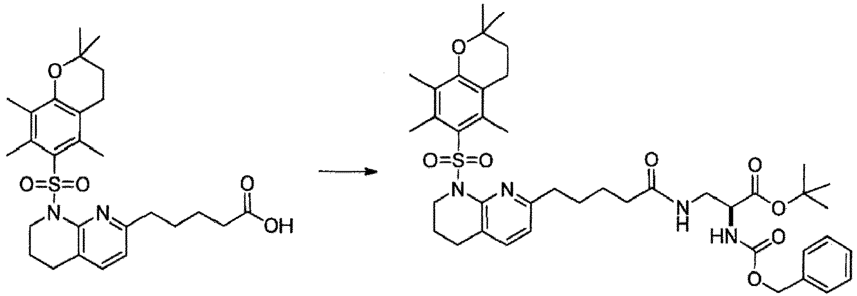

HBTU (1.22 g) was added to a DMF (8 mL) solution of 5-(8-((2,2,5,7,8-pentamethylchroman-6-yl)sulfonyl)-5,6,7,8-tetrahydro-1,8-naphthyridin-2-yl) pentanoic acid (1.47 g), (S)-tert-butyl 3-amino-2-(((benzyloxy)carbonyl)amino)propanoate (951 mg) and DIEA (1.13 mL), followed by stirring for 30 minutes at room temperature. Water (30 mL) and ethyl acetate (30 mL) were added thereto, followed by stirring. The organic layer was fractionated, sequentially washed with a 5% aqueous citric acid solution (15 mL), water (15 mL), an aqueous saturated sodium chloride solution (15 mL), an aqueous saturated sodium hydrogen carbonate solution (15 mL), and an aqueous saturated sodium chloride solution (15 mL), and dried over anhydrous sodium sulfate, and the solvent was distilled away under reduced pressure. The residue was purified by silica gel column chromatography (hexane/ethyl acetate = 60/40 to 30/70), thereby obtaining (S)-tert-butyl 2-(((benzyloxy)carbonyl)amino)-3-(5-(8-((2,2,5,7,8-pentamethylchroman-6-yl)sulfonyl)-5,6,7, 8-tetrahydro-1,8-naphthyridin-2-yl)pentanamide)propanoate (2.16 g) as a colorless foamy substance.

LC/MS rt (min): 2.08

MS (ESI, m/z): 777.7 [M + H]⁺

(1-B)

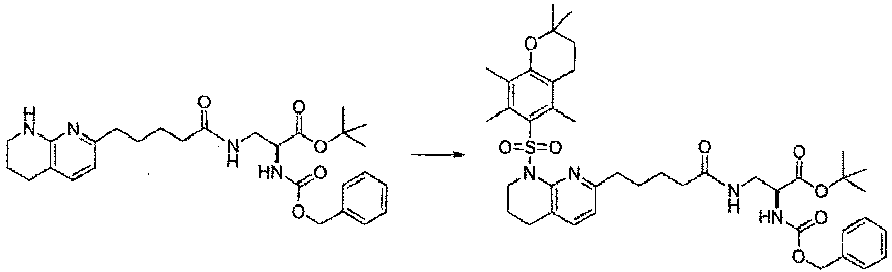

2,2,5,7,8-pentamethylchroman-6-yl)sulfonyl chloride (0.712 g) was added to a mixed solution of (S)-tert-butyl 2-(((benzyloxy)carbonyl)amino)-3-(5-(5,6,7,8-tetrahydro-l,8-naphthyridin-2-yl)pentanamide) propanoate (1.0 g), potassium carbonate (0.677 g), and acetonitrile (5.6 mL) at room temperature, followed by stirring for 1 hour at room temperature, and the solution was heated for 3 hours under reflux. The temperature of the reaction solution was returned to room temperature, water (10 mL) was added thereto, and extraction was performed using ethyl acetate. The organic layer was washed with an aqueous saturated sodium chloride solution, and dried over anhydrous sodium sulfate, and the solvent was distilled away under reduced pressure. The obtained residue was purified by silica gel column chromatography (hexane/ethyl acetate), thereby obtaining (S)-tert-butyl 2-(((benzyloxy)carbonyl)amino)-3-(5-(8-((2,2,5,7,8-pentamethylchroman-6-yl)sulfonyl)-5,6,7, 8-tetrahydro-1,8-naphthyridin-2-yl)pentanamide)propanoate (0.500 g) as a white solid.

LC/MS rt (min): 2.08

MS (ESI, m/z): 777.7 [M + H]⁺

¹H-NMR (CDCl₃) δ: 7.37-7.29 (m, 4H), 7.15 (d, 1H, J = 7.9 Hz), 6.53 (d, 1H, J = 7.9 Hz), 5.99-5.90 (m, 1H), 5.79-5.71 (m, 1H), 5.09 (s, 2H), 4.34-4.22 (m, 1H), 4.11-4.02 (m,2H), 3.72-3.50 (m, 2H), 2.73 (t, 2H, J = 6.3 Hz), 2.63 (t, 2H, J = 6.6 Hz), 2.56 (s, 3H), 2.53 (s, 3H), 2.31 (t, 2H, J = 7.3 Hz), 2.13-1.90 (m, 5H), 1.79 (t, 2H, J = 6.9 Hz), 1.58-1.50 (m, 8H), 1.48-1.35 (m, 8H), 1.33-1.15 (m, 6H)

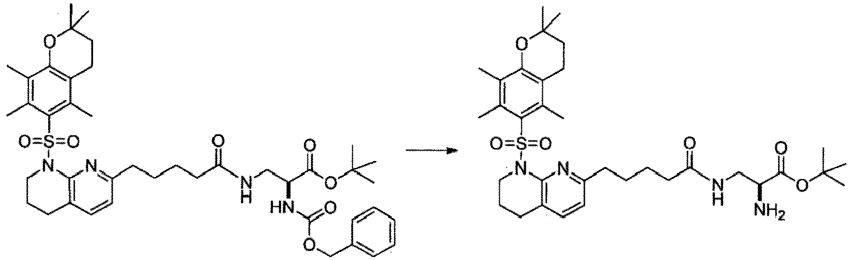

A methanol (14 mL) solution of (S)-tert-butyl 2-(((benzyloxy)carbonyl)amino)-3-(5-(8-((2,2,5,7,8-pentamethylchroman-6-yl)sulfonyl)-5,6,7, 8-tetrahydro-1,8-naphthyridin-2-yl)pentanamide)propanoate (0.500 g) was allowed to flow through a flow-type hydrogenation reactor (H-Cube, ThalesNano Inc.) equipped with a 10% palladium-on-carbon cartridge, and the solvent was distilled away under reduced pressure, thereby obtaining (S)-tert-butyl 2-amino-3-(5-(8-((2,2,5,7,8-pentamethylchroman-6-yl)sulfonyl)-5,6,7,8-tetrahydro-l,8-naphth yridin-2-yl)pentanamide)propanoate (0.309 g) as a light yellow solid.

LC/MS rt (min): 1.45

MS (ESI, m/z): 643.6 [M + H]⁺

¹H-NMR (CDCl₃) δ: 7.16 (d, 1H, J = 7.9 Hz), 6.54 (d, 1H, J = 7.9 Hz), 6.09-5.98 (m, 1H), 4.12-4.02 (m, 2H), 3.67-3.56 (m, 1H), 3.49-3.42 (m, 1H), 3.30-3.18 (m, 1H), 2.74 (t, 2H, J = 6.3 Hz), 2.64 (t, 2H, J = 6.9 Hz), 2.57 (s, 3H), 2.54 (s, 3H), 2.33 (t, 2H, J = 7.3 Hz), 2.14-1.97 (m, 6H), 1.90-1.68 (m, 4H), 1.51-1.37 (m, 11H), 1.34-1.16 (m, 9H)

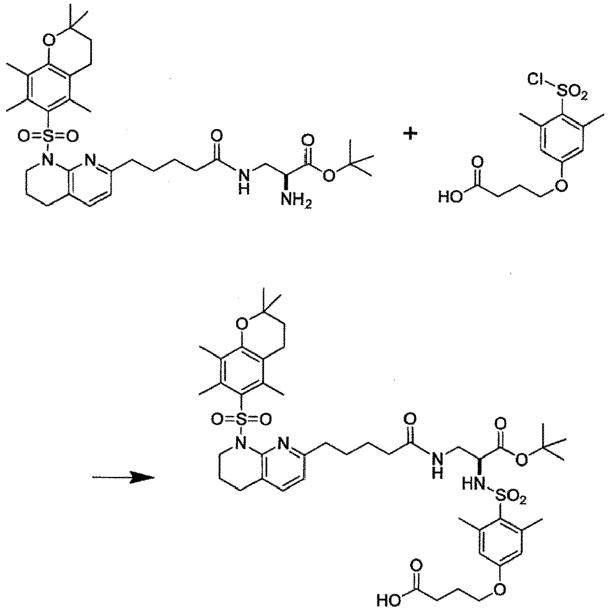

4-(4-(chlorosulfonyl)-3,5-dimethylphenoxy)butanoic acid (57.4 mg) was added to a mixed solution of (S)-tert-butyl 2-amino-3-(5-(8-((2,2,5,7,8-pentamethylchroman-6-yl)sulfonyl)-5,6,7,8-tetrahydro-1,8-naphth yridin-2-yl)pentanamide)propanoate (100 mg), sodium hydrogen carbonate (39.3 mg), and N,N-dimethylacetamide (1.6 mL), followed by stirring for 27 hours at room temperature, thereby obtaining a reaction mixture containing (S)-4-(4-(N-(1-(tert-butoxy)-1-oxo-3-(5-(8-((2,2,5,7,8-pentamethylchroman-6-yl)sulfonyl)-5,6 ,7,8-tetrahydro-1,8-naphthyridin-2-yl)pentanamide)propan-2-yl)sulfamoyl)-3,5-dimethylpheno xy)butanoic acid.

### Reference Example 4

(1-A-1)

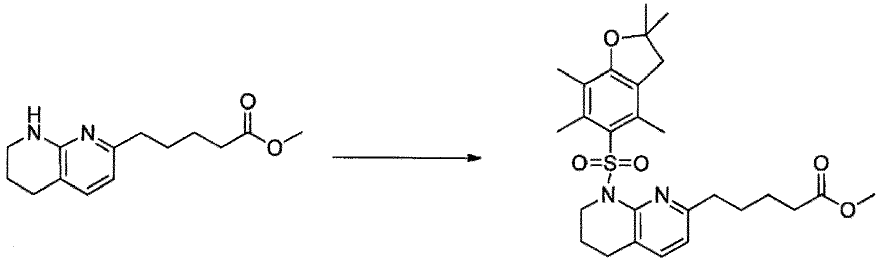

Acetonitrile (8 mL) was added to a mixture of methyl 5-(5,6,7,8-tetrahydro-1,8-naphthyridin-2-yl)pentanoate (1.04 g), 2,2,4,6,7-pentamethyl-2,3-dihydrobenzofuran-5-yl)sulfonyl chloride (1.33 g), and potassium carbonate (870 mg), followed by stirring for 8 hours at 70°C. By adding ethyl acetate (20 mL) and water (30 mL) thereto, the organic layer was fractionated. The organic layer was then washed with an aqueous saturated sodium hydrogen carbonate solution (30 mL) and dried over anhydrous sodium sulfate, and the solvent was distilled away under reduced pressure. The obtained reside was purified by silica gel column chromatography (hexane/ethyl acetate = 85/15 to 65/35) and then recrystallized from IPA/hexane, thereby obtaining methyl 5-(8-((2,2,4,6,7-pentamethyl-2,3-dihydrobenzofuran-5-yl)sulfonyl)-5,6,7,8-tetrahydro-1,8-nap hthyridin-2-yl)pentanoate (1.18 g) as a white solid.

LC/MS rt (min): 1.96

MS (ESI, m/z): 501.4 [M + H]⁺

¹H-NMR (CDCl₃) δ: 7.19 (1H, d, J = 8.1 Hz), 6.56 (1H, d, J = 8.1 Hz), 4.06-4.13 (2H, m), 3.64 (3H, s), 2.98 (2H, s), 2.75 (2H, t, J = 7.2 Hz), 2.56 (3H, s), 2.50 (3H, s), 2.37 (2H, t, J = 7.2 Hz), 2.16 (2H, t, J= 7.5 Hz), 2.08 (3H, s), 2.01-2.05 (2H, m), 1.22-1.32 (2H, m)

(1-A-2)

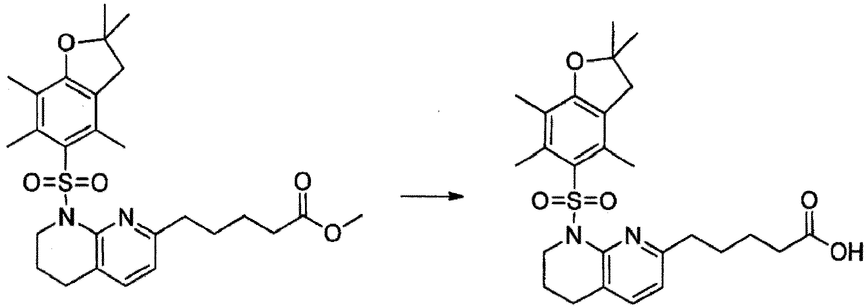

A 2.5 M aqueous sodium hydroxide solution (1.2 mL) was added to a mixture of methyl 5-(8-((2,2,4,6,7-pentamethyl-2,3-dihydrobenzofuran-5-yl)sulfonyl)-5,6,7,8-tetrahydro-1,8-nap hthyridin-2-yl)pentanoate (499 mg), THF (5 mL), water (0.3 mL), and MeOH (2.5 mL), followed by stirring for 7 hours at room temperature. Water (25 mL) and sodium hydrogen sulfate were added to the reaction mixture until the pH became 4, ethyl acetate was further added thereto (30 mL), and the organic layer was fractionated. The obtained organic layer was washed twice with water (20 mL) and then with once with an aqueous saturated sodium chloride solution (20 mL) and dried over anhydrous sodium sulfate. Thereafter, the solvent was distilled away under reduced pressure, thereby obtaining 5-(8-((2,2,4,6,7-pentamethyl-2,3-dihydrobenzofuran-5-yl)sulfonyl)-5,6,7,8-tetrahydro-1,8-nap hthyridin-2-yl)pentanoic acid (501 mg) as a colorless foamy substance.

LC/MS rt (min): 1.49

MS (ESI, m/z): 487.4 [M + H]⁺

(1-A-3)

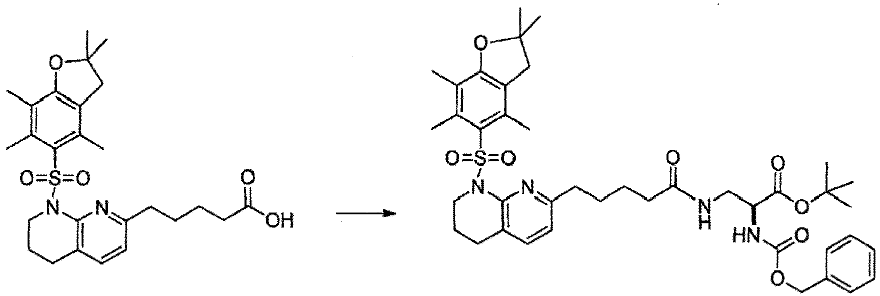

HBTU (436 mg) was added to a DMF (4 mL) solution of 5-(8-((2,2,4,6,7-pentamethyl-2,3-dihydrobenzofuran-5-yl)sulfonyl)-5,6,7,8-tetrahydro-1,8-nap hthyridin-2-yl)pentanoic acid (501 mg), (S)-tert-butyl 3-amino-2-(((benzyloxy)carbonyl)amino)propanoate (294 mg), and DIEA (0.42 mL), followed by stirring for 30 minutes at room temperature. An aqueous saturated ammonium chloride solution (20 mL) and ethyl acetate (30 mL) were added thereto, followed by stirring. Thereafter, the organic layer was fractionated, sequentially washed with water (20 mL) and an aqueous saturated sodium chloride solution (20 mL), and dried over anhydrous magnesium sulfate, and then the solvent was distilled away under reduced pressure. The residue was purified by silica gel column chromatography (hexane/ethyl acetate = 60/40 to 30/70), thereby obtaining (S)-tert-butyl 2-(((benzyloxy)carbonyl)amino)-3-(5-(8-((2,2,4,6,7-pentamethyl-2,3-dihydrobenzofuran-5-yl) sulfonyl)-5,6,7,8-tetrahydro-1,8-naphthyridin-2-yl)pentanamide)propanoate (768 mg) as a colorless foamy substance.

LC/MS rt (min): 2.01

MS (ESI, m/z): 763.6 [M + H]⁺

(1-B)

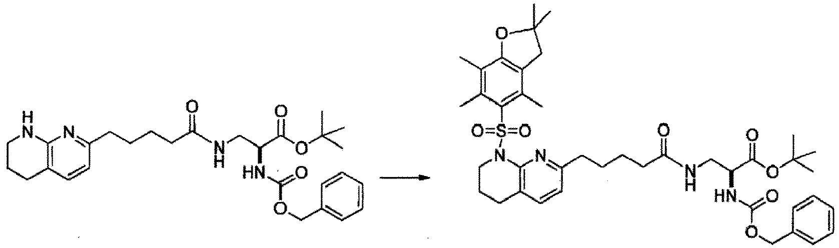

By using (S)-tert-butyl 2-(((benzyloxy)carbonyl)amino)-3-(5-(-5,6,7,8-tetrahydro-1,8-naphthyridin-2-yl)pentanamide) propanoate (1.0 g), (S)-tert-butyl 2-(((benzyloxy)carbonyl)amino)-3-(5-(8-((2,2,4,6,7-pentamethyl-2,3-dihydrobenzofuran-5-yl) sulfonyl)-5,6,7,8-tetrahydro-1,8-naphthyridin-2-yl)pentanamide)propanoate (136 mg) as a light yellow solid by the same method as described in (1-B) of Reference Example 3.

LC/MS rt (min): 2.01

MS (ESI, m/z): 763.7 [M + H]^{+ 1}H-NMR (CDCl₃) δ: 7.37-7.29 (m, 4H), 7.16 (d, 1H, J = 7.3 Hz), 6.54 (d, 1H, J = 7.3 Hz), 5.98-5.89 (m, 1H), 5.78-5.69 (m, 1H), 5.10 (s, 2H), 4.34-4.24 (m, 1H), 4.10-4.04 (m, 2H), 3.71-3.52 (m, 2H), 2.97 (s, 2H), 2.73 (t, 2H, J = 6.3 Hz), 2.55 (s, 3H), 2.49 (s, 3H), 2.35 (t, 2H, J= 7.6 Hz), 2.10-1.92 (m, 5H), 1.58-1.50 (m, 5H), 1.49-1.35 (m, 15H), 1.33-1.21 (m, 2H)

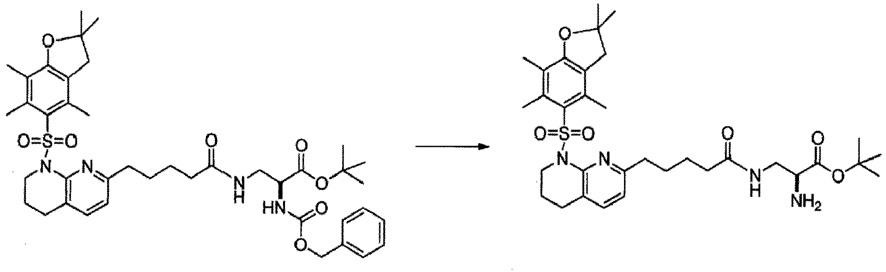

(S)-tert-butyl 2-(((benzyloxy)carbonyl)amino)-3-(5-(8-((2,2,4,6,7-pentamethyl-2,3-dihydrobenzofuran-5-yl) sulfonyl)-5,6,7,8-tetrahydro-1,8-naphthyridin-2-yl)pentanamide)propanoate (150 mg), methanol (3 mL), and 10% palladium on carbon (30 mg) were put into an autoclave, and stirred for 2.5 hours at room temperature in a nitrogen atmosphere at 0.9 MPa. Hydrogen was added thereto, followed by stirring for 3 hours at room temperature in a nitrogen atmosphere at 0.9 MPa. The insoluble matter was removed by filtration, and the solvent was distilled away under reduced pressure, thereby obtaining (S)-tert-butyl 2-amino-3-(5-(8-((2,2,4,6,7-pentamethyl-2,3-dihydrobenzofuran-5-yl)sulfonyl)-5,6,7,8-tetrahy dro-1,8-naphthyridin-2-yl)pentanamide)propanoate (130 mg) as a black oily substance.

LC/MS rt (min): 1.51

MS (ESI, m/z): 629 [M + H]⁺

¹H-NMR (CDCl₃, 300 MHz) δ: 7.17 (1H, d, J = 7.3 Hz), 6.55 (1H, d, J = 7.3 Hz), 6.01-5.94 (1H, m), 4.11-4.04 (2H, m), 3.66-3.56 (1H, m), 3.51-3.39 (1H, m), 3.28-3.18 (1H, m), 2.98 (2H, s), 2.74 (2H, t, J = 6.3 Hz), 2.56 (3H, s), 2.50 (3H, s), 2.36 (2H, t, J = 7.3 Hz), 2.10-1.97 (7H, m), 1.60-1.52 (4H, m), 1.46 (15H, s), 1.34-1.22 (2H, m)

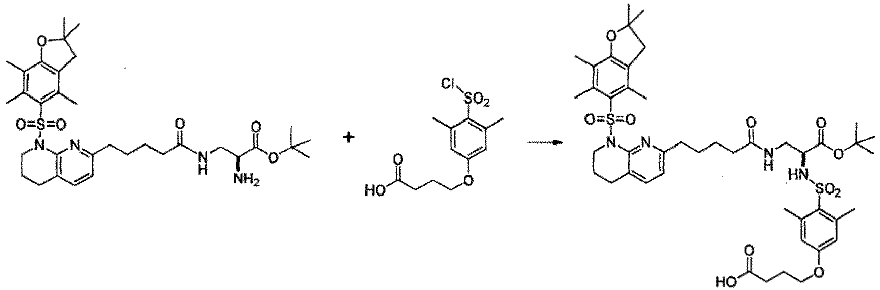

4-(4-(chlorosulfonyl)-3,5-dimethylphenoxy)butanoic acid (58 mg) was added to a mixture of (S)-tert-butyl 2-amino-3-(5-(8-((2,2,4,6,7-pentamethyl-2,3-dihydrobenzofuran-5-yl)sulfonyl)-5,6,7,8-tetrahy dro-1,8-naphthyridin-2-yl)pentanamide)propanoate (99 mg), DMAc (1 mL), and sodium hydrogen carbonate (40 mg), followed by stirring for 15 hours at room temperature, thereby obtaining a reaction mixture containing (S)-4-(4-(N-(1-(tert-butoxy)-1-oxo-3-(5-(8-((2,2,4,6,7-pentamethyl-2,3-dihydrobenzofuran-5-y 1)sulfonyl)-5,6,7,8-tetrahydro-1,8-naphthyridin-2-yl)pentanamide)propan-2-yl)sulfamoyl)-3,5-dimethylphenoxy)butanoic acid.

LC/MS rt (min): 1.86

MS (ESI, m/z): 899.4 [M + H]⁺

### Reference Example 5

(1)

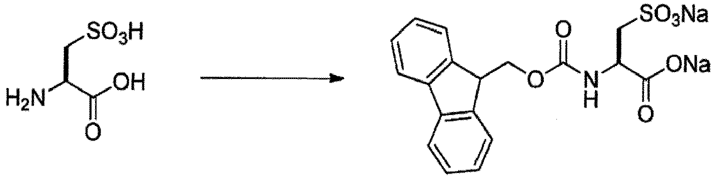

Sodium hydrogen carbonate (50 g) was added to an aqueous solution (400 mL) of L-cysteic acid (50 g), followed by stirring. An acetone solution (800 mL) of 9-fluorenylmethyl N-succinimidyl carbonate (109.7 g) was added dropwise to the above solution for 25 minutes, followed by stirring for 7 hours at room temperature. Acetone (400 mL) was added thereto, and the solid was collected by filtration and washed three times with acetone (100 mL). The solid was dissolved in water (600 mL) and heated to 50°C. Then, methanol (1,200 mL) was added thereto with stirring, and water (200 mL) and methanol (400 mL) were further added thereto, followed by stirring for 2 hours at room temperature. The solid was collected by filtration and washed three times with hydrous methanol (methanol:water = 2:1, 100 mL), thereby obtaining disodium (R)-2-((((9H-fluoren-9-yl)methoxy)carbonyl)amino)-3-sulfopropanoate (93.5 g) as a white solid.

LC/MS rt (min): 1.01

MS (ESI, m/z): 390.1 [free from M - H]⁻

¹H-NMR (300 MHz, D₂O) δ: 7.82 (d, 2H, J = 7.2 Hz), 7.66 (d, 2H, J = 7.8 Hz), 7.41 (t, 2H, J = 7.8 Hz), 7.33 (t, 2H, J = 7.2 Hz)

HPLC (TSKgel ODS-100Z, ammonium acetate-based) rt (min): 13.5

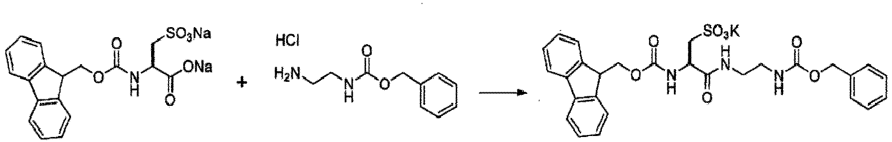

Methanesulfonic acid (46 g) was added dropwise to a DMAc (500 mL) suspension of disodium (R)-2-((((9H-fluoren-9-yl)methoxy)carbonyl)amino)-3-sulfopropanoate (87.1 g) for 5 minutes, followed by stirring for 40 minutes at room temperature. N-(benzyloxy)carbonyl-1,2-diaminoethane hydrochloride (51.2 g) was added thereto, followed by stirring for 30 minutes at room temperature. Then, DIEA (165 mL) and HBTU (83.5 g) were added thereto, followed by stirring for 1.5 hours at room temperature. Water (1 L) was added to the reaction solution, an aqueous potassium acetate solution (potassium acetate (196 g)/water (800 mL)) was added dropwise thereto with stirring. The reaction solution was stirred for 30 minutes at a temperature of 50°C to 55°C, then cooled to room temperature, and stirred for 2 hours. The solid was collected by filtration and washed three times with ice water (300 mL). Acetone (2 L) was added to the obtained solid, followed by stirring for 1 hour at room temperature. Then, the solid was collected by filtration and washed three times with acetone (200 mL), thereby obtaining potassium (R)-2-((((9H-fluoren-9-yl)methoxy)carbonyl)amino)-3-((2-(((benzyloxy)carbonyl)amino)ethyl )amino)-3-oxopropane-1-sulfonate (106.7 g) as a white solid.

LC/MS rt (min): 1.22

MS (ESI, m/z): 566.2 [free from M - H]⁻

HPLC (TSKgel ODS-100Z, ammonium acetate-based) rt (min): 24.2

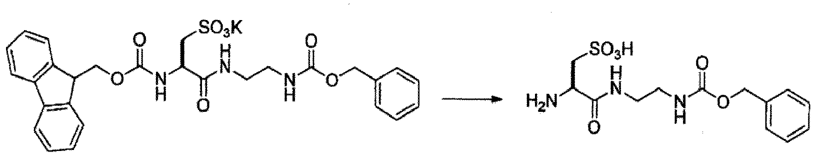

Diethylamine (85.5 mL) was added to a mixture of potassium (R)-2-((((9H-fluoren-9-yl)methoxy)carbonyl)amino)-3-((2-(((benzyloxy)carbonyl)amino)ethyl )amino)-3-oxopropane-1-sulfonate (50.0 g), water (100 mL), and acetonitrile (300 mL), followed by stirring for 7 hours at room temperature. The solvent was distilled away under reduced pressure, and an operation of adding acetonitrile (100 mL) and toluene (200 mL) to the residue and performing concentration under reduced pressure was carried out twice, thereby obtaining a white solid. Acetonitrile (750 mL) was added to the solid such that the solid was dissolved, and then the solid was collected by filtration, thereby obtaining (R)-2-amino-3-((2(((benzyloxy)carbonyl)amino)ethyl)amino)-3-oxopropane-1-sulfonate (38 g). The solid was dissolved in water (500 mL), the insoluble matter was removed by filtration, and then concentrated hydrochloric acid (15 mL) was added dropwise thereto at room temperature with stirring, followed by stirring for 3 hours at room temperature. The reactio mixture was cooled to 10°C. Thereafter, the solid was collected by filtration, washed three times with ice water (100 mL), and then dried, thereby obtaining (R)-2-amino-3-((2-(((benzyloxy)carbonyl)amino)ethyl)amino)-3-oxopropane-1-sulfonic acid (20.1 g) as a white solid.

LC/MS rt (min): 0.70

MS (ESI, m/z): 346.1 [M + H]⁺

HPLC (TSKgel ODS-100Z, formic acid-based) rt (min): 10.0

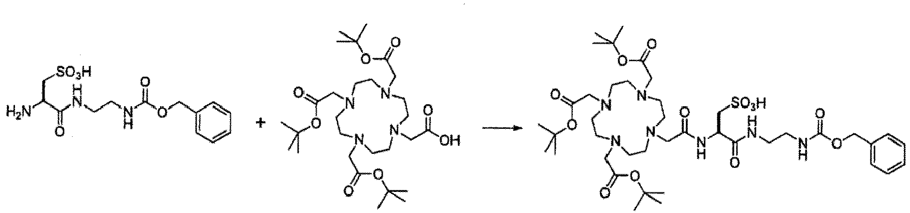

A mixture of (R)-2-amino-3-((2-(((benzyloxy)carbonyl)amino)ethyl)amino)-3-oxopropane-1-sulfonic acid (16.5 g), 2-(4,7,10-tris(2-(tert-butoxy)-2-oxoethyl)-1,4,7,10-tetraazacyclododecan-1-yl) acetic acid (24.8 g), DMAc (125 mL), and DIEA (18.9 mL) was stirred for 20 minutes at room temperature, and then HBTU (17.7 g) was added thereto in five divided portions at an interval of 10 minutes, followed by stirring for 1 hour at room temperature. An aqueous saturated sodium chloride solution (125 mL) and ethyl acetate (250 mL) were added to the reaction mixture, and then the organic acid was fractionated and washed twice with an aqueous saturated sodium chloride solution (125 mL) and then tiwce with an aqueous saturated sodium hydrogen carbonate solution (125 mL). The organic layer was dried over anhydrous sodium sulfate, and the solvent was distilled away under reduced pressure, thereby obtaining (R)-3-((2-(((benzyloxy)carbonyl)amino)ethyl)amino)-3-oxo-2-(2-(4,7,10-tris(2-(tert-butoxy)-2 -oxoethyl)-1,4,7,10-tetraazacyclododecan-1-yl)acetamide)propane-1-sulfonic acid (47.6 g) as a colorless foamy substance.

LC/MS rt (min): 1.21

MS (ESI, m/z): 900.6 [M + H]⁺

HPLC (TSKgel ODS-100Z, formic acid-based) rt (min): 18.5

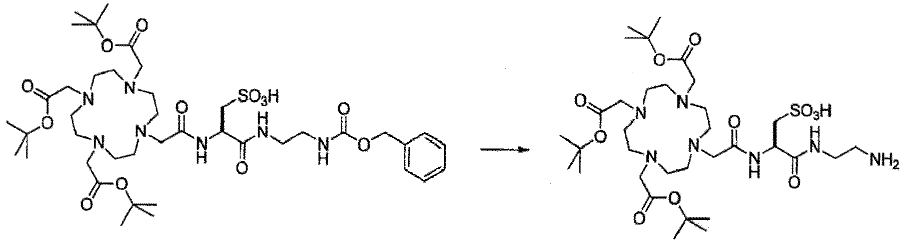

10% palladium on carbon (0.489 g) and a methanol (20 mL) solution of (R)-3-((2-(((benzyloxy)carbonyl)amino)ethyl)amino)-3-oxo-2-(2-(4,7,10-tris(2-(tert-butoxy)-2 -oxoethyl)-1,4,7,10-tetraazacyclododecan-1-yl)acetamide)propane-1-sulfonic acid (2.05 g) were put into a stainless steel tube, followed by stirring for 5 hours at 30°C in a nitrogen atmosphere at 0.5 MPa. The insoluble matter was removed by filtration, the solvent was distilled away under reduced pressure. Then, toluene (20 mL) was added thereto, and then the solvent was distilled away again under reduced pressure, thereby obtaining (R)-3-((2-aminoethyl)amino)-3-oxo-2-(2-(4,7,10-tris(2-tert-(butoxy)-2-oxoethyl)-1,4,7,10-tetr aazacyclododecan-1-yl)acetamide)propane-1-sulfonic acid (1.47 g) as a rose pink solid. ¹H-NMR (CDCl₃) δ: 8.58-8.35 (1H, m), 7.90-7.68 (1H, m), 4.73-4.59 (1H, m), 3.69-1.87 (33H, m), 1.49-1.34 (27H, m)

HPLC (TSKgel ODS-100Z) rt (min): 10.1

### Reference Example 6

(1-A)

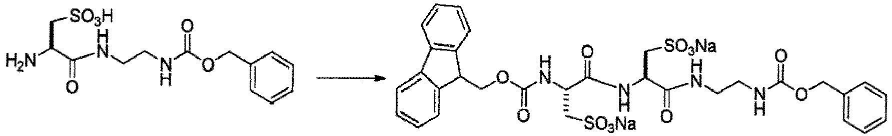

Methanesulfonic acid (43 µL) was added to a DMF (1 mL) suspension of disodium (R)-2-((((9H-fluoren-9-yl)methoxy)carbonyl)amino)-3-sulfopropanoate (130.6 mg), followed by stirring for 20 minutes at room temperature. Then, (R)-2-amino-3-((2-(((benzyloxy)carbonyl)amino)ethyl)amino)-3-oxopropane-2-sulfonic acid (103.6 mg), DIEA (230 ¡.tL), and HBTU (125 mg) were added thereto, followed by stirring for 20 minutes at room temperature. Water (0.4 mL) was added to the reaction solution, and a mixed solution of sodium acetate (sodium acetate (250 mg)/water (125 µL)methanol (3 mL)) was added thereto with stirring, and IPA (9 mL) and methanol (2 mL) were further added thereto. The obtained solid was collected by filtration, washed three times with methanol (1 mL), and dried under reduced pressure, thereby obtaining disodium (9R, 12R)-12-((((9H-fluoren-9-yl)methoxy)carbonyl)amino)-3,8,11-trioxo-1-phenyl-9-(sulfonatom ethyl)-2-oxa-4,7,10-triazatridecane-13-sulfonate (213 mg) as a white solid.

LC/MS rt (min): 1.15

MS (ESI, m/z): 717.1 [free from M - H]⁻

(1-B)

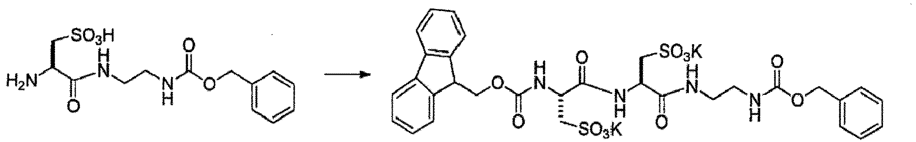

Methanesulfonic acid (2.54 g) was added dropwise to a DMF (35.0 mL) suspension of disodium (R)-2-((((9H-fluoren-9-yl)methoxy)carbonyl)amino)-3-sulfopropanoate (5.22 g), followed by stirring at room temperature until a homogenous solution was obtained. (R)-2-amino-3-((2-(((benzyloxy)carbonyl)amino)ethyl)amino)-3-oxopropane-1-sulfonic acid (4.14 g), DIEA (9.2 mL), and HBTU (5.0 g) were added to the solution, followed by stirring for 1.5 hours at room temperature. Water (50.0 mL) was added to the reaction solution, followed by stirring for 30 minutes at room temperature, and then the insoluble matter was removed by filtration. An aqueous potassium acetate solution (potassium acetate (11.8 g)/water (40.0 mL)) was added to the filtrate at room temperature with stirring, followed by stirring for 1 hour, and then the solid was collected by filtration and washed with ice water (50.0 mL). Acetonitrile (200 mL) was added to the solid, followed by stirring for 1 hour at 60°C. Then, the solid was cooled to 35°C and collected by filtration, thereby obtaining dipotassuim (9R, 12R)-12-((((9H-fluoren-9-yl)methoxy)carbonyl)amino)-3,8,11-trioxo-1-phenyl-9-(sulfonatom ethyl)-2-oxa-4,7,10-triazatridecane-13-sulfonate (8.71 g) as a white solid.

LC/MS rt (min): 1.15

MS (ESI, m/z): 717.2 [free from M - H]⁻

¹H-NMR (300 MHz, DMSO) δ: 8.42 (d, 1H, J = 6.6 Hz), 7.95 (1H, br), 7.89 (2H, d, J = 7.2 Hz), 7.70 (2H, t, J = 6.6 Hz), 7.52 (1H, d, J = 5.7 Hz),7.41 (2H, d, J = 7.2 Hz), 7.25-7.35 (7H, m), 7.10 (1H, br), 4.90-5.01 (2H, m), 4.25 (3H, s), 4.16-4.30 (1H, m), 2.79-3.13 (8H, m)

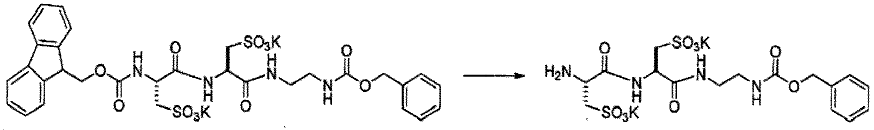

Diethylamine (1 mL) was added to an suspension water (1.4 mL) and acetonitrile (4.2 mL) of dipotassuim (9R, 12R)-12-((((9H-fluoren-9-yl)methoxy)carbonyl)amino)-3,8,11-trioxo-1-phenyl-9-(sulfonatom ethyl)-2-oxa-4,7,10-triazatridecane-13-sulfonate (556.5 mg), followed by stirring for 2 hours at room temperature. The solvent was distilled away under reduced pressure, acetonitrile (10 mL) and toluene (10 mL) were added to the residue, and the solvent was distilled away under reduced pressure. Acetonitrile (20 mL) was added thereto, and the resultant was stirred overnight. Then, the solid was collected by filtration, thereby obtaining dipotassium (9R, 12R)-12-amino-3,8, 11-trioxo-1-phenyl-9-(sulfonatomethyl)-2-oxa-4,7,10-triazatridecane-13-s ulfonate (452 mg) as a white solid.

LC/MS rt (min): 0.65

MS (ESI, m/z): 495.1 [free from M - H]⁻

¹H-NMR (DMSO-d₆/TFA-d) δ: 8.75 (1H, d, J = 7.2 Hz), 8.10 (2H, brs), 7.28-7.26 (2H, m), 7.17 (1H, brs), 5.01 (2H, brs), 4.43 (1H, q, J = 7.2Hz), 3.98-4.01 (1H, m), 2.78-3.18 (8H, m)

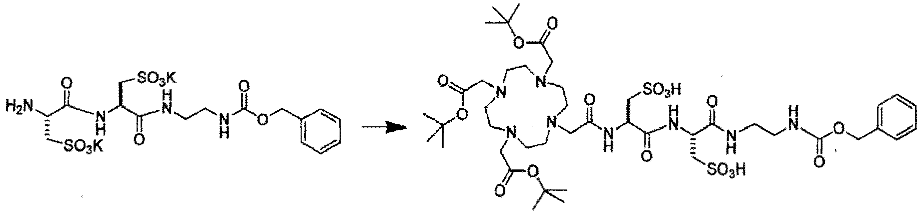

A DMF (4.5 mL) solution of dipotassium (9R, 12R)-12-amino-3,8,11-trioxo-1-phenyl-9-(sulfonatomethyl)-2-oxa-4,7,10-triazatridecane-13-s ulfonate (224 mg) and 2-(4,7,10-tris(2-(tert-butoxy)-2-oxoethyl)-1,4,7,10-tetraazacyclododecan-1-yl) acetic acid (236 mg) was stirred for 20 minutes at room temperature, and then DIEA (0.14 mL) and HBTU (157 mg) were added thereto, followed by stirring. Water (0.1 mL) and ethyl acetate (13.5 mL) were added thereto, followed by stirring. Thereafter, the precipitated solid was collected by filtration, and the solvent was distilled away under reduced pressure. Then, water (3 mL) and ethyl acetate (3 mL) were added thereto, and an aqueous layer was fractionated and washed with ethyl acetate (3 mL). Subsequently, an aqueous saturated sodium hydrogen carbonate solution (2 mL) was added thereto, and a reversed-phase silica gel column (inner diameter of glass column: 10.5 cm, Daisogel-SR120-40/60-ODS-RPS: 400 g) was charged with the solution, and elution was performed under a normal pressure by using an aqueous saturated sodium carbonate solution (6 mL), water (6 mL), 0.1% formic acid-containing water (6 mL), 0.1% formic acid/20% acetonitrile-containing water (6 mL), 0.1% formic acid/40% acetonitrile-containing water (12 mL), and 0.1% formic acid/60% acetonitrile-containing water (12 mL) in this order, thereby obtaining (9R, 12R)-3,8,11-trioxo-1-phenyl-9-(sulfomethyl)-12-(2-(4,7,10-tris(2-(tert-butoxy)-2-oxoethyl)-1, 4,7,10-tetraazacyclododecan-l-yl)acetamide)-2-oxa-4,7,10-triazatridecane-13-sulfonic acid (265 mg) as a white solid.

MS (ESI, m/z): 1151 [M + H]⁺

¹H-NMR (D₂O) δ: 7.49-7.35 (5H, m), 5.10 (2H, s), 4.35-2.82 (34H, m), 1.57-1.38 (27H, m)

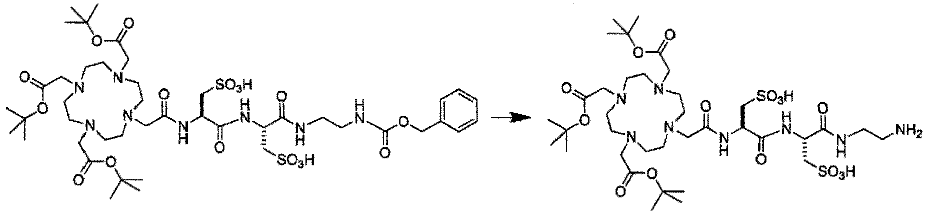

10% palladium on carbon (26 mg), (9R, 12R)-3,8,11-trioxo-1-phenyl-9-(sulfomethyl)-12-(2-(4,7,10-tris(2-(tert-butoxy)-2-oxoethyl)-1, 4,7,10-tetraazacyclododecan-1-yl)acetamide)-2-oxa-4,7,10-triazatridecane-13-sulfonic acid (244 mg), water (0.15 mL), and methanol (3 mL) were put into a stainless steel tube, followed by stirring for 2 hours at room temperature in a nitrogen atmosphere at 0.9 MPa. The insoluble matter was removed by filtration, and the solvent was distilled away under reduced pressure, thereby obtaining (R)-3-((2-aminoethyl)amino)-3-oxo-2-((R)-3-sulfo-2-(2-(4,7,10-tris(2-(tert-butoxy)-2-oxoethy l)-1,4,7,10-tetraazacyclododecan-1-yl)acetamide)propanamide)propane-1-sulfonic acid (209 mg) as a white solid.

MS (ESI, m/z): 917 [M + H]⁺

¹H-NMR (D₂O) δ: 3.83-3.25 (16H, m), 3.18-2.78 (18H, m), 1.51-1.45 (27H, m)

### Reference Example 7

(1-A-1)

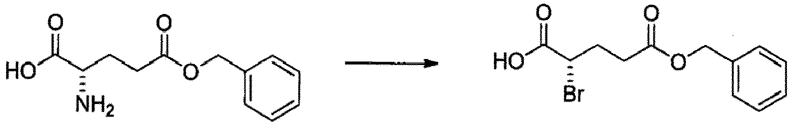

A mixture of potassium bromide (60.2 g), water (300 mL), hydrobromic acid (40 mL), and (S)-2-amino-5-(benzyloxy)-5-oxopentanoic acid (40.0 g) was cooled to 3°C, and an aqueous solution (60 mL) of sodium nintrite (23.3 g) was added dropwise thereto for 1 hour and 50 minutes, followed by stirring for 40 minutes. Sulfuric acid (10 mL) was added thereto, followed by stirring for 10 minutes. Then, the organic layer was fractionated by adding ethyl acetate (400 mL), washed twice with water (400 mL), and dried over anhydrous sodium sulfate, and then the solvent was concentrated under reduced pressure. The resultant was purified by silica gel column chromatography (hexane/ethyl acetate = 100/0 to 50/50), thereby obtaining (S)-5-(benzyloxy)-2-bromo-5-oxopentanoic acid (36.1 g) as a colorless oily substance.

MS (ESI, m/z): 299 [M - H]⁻

¹HNMR (CDCl₃, 300 MHz) δ: 7.38-7.33 (4H, m), 5.15 (2H, s), 4.42 (1H, dd, J = 8.6, 5.9 Hz), 2.67-2.59 (2H, m), 2.53-2.25 (2H, m)

Optical purity: 97% ee

(1-A-2)

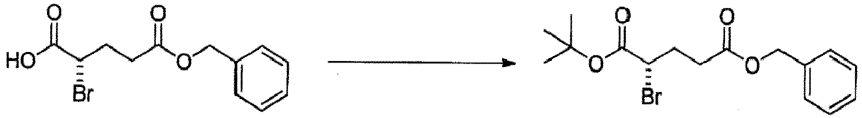

At a temperature of 4°C, a mixture of tert-butyl 2,2,2-trichloroacetimidate (43.0 mL) and hexane (72 mL) was added dropwise for 30 minutes to a mixture of (S)-5-(benzyloxy)-2-bromo-5-oxopentanoic acid (36.1 g), chloroform (72.0 mL), and hexane (72.0 mL), and then a boron trifluoride diethyl ether complex (1.51 mL) was added thereto for 5 minutes, followed by stirring for 1 hour. Sodium hydrogen carbonate (10 g) was added thereto, followed by stirring for 1 hour. Then, the insoluble matter was removed by filtration, and the solvent was distilled away under reduced pressure. The obtained residue was purified by silica gel column chromatography (hexane/ethyl acetate = 100/0 to 85/15), thereby obtaining (S)-O⁵-benzyl O¹-tert-butyl 2-bromopentanedioate (39.0 g) as a colorless oily substance.

¹HNMR (CDCl₃, 300 MHz) δ: 7.37 (5H, s), 5.14 (2H, s), 4.24 (1H, dd, J = 8.6, 5.9 Hz), 2.60-2.52 (2H, m), 2.44-2.18 (2H, m), 1.52 (9H, s)

Optical purity: 94% ee

(1-A-3)

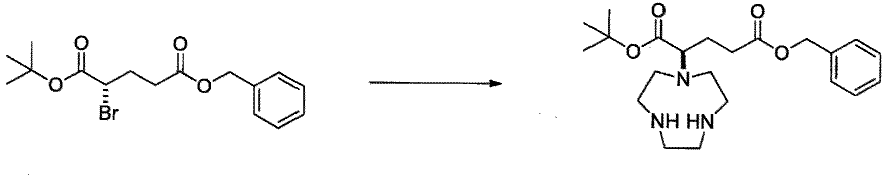

At room temperature, a chloroform (380 mL) solution of (S)-O⁵-benzyl O¹-tert-butyl 2-bromopentanedioate (38.0 g) was added dropwise for 30 minutes to a chloroform (200 mL) solution of 1,4,7-triazacyclononane (41.1 g), followed by stirring for 19 hours at room temperature. By adding water (400 mL) to the reaction mixture, the organic layer was fractionated, and the aqueous layer was extracted using chloroform (200 mL). The organic layer was dried over anhydrous sodium sulfate, and the solvent was distilled away under reduced pressure. The obtained residue was purified by silica gel column chromatography (dichloromethane/ethanol = 100/90 to 99/1), thereby obtaining (R)-O⁵-benzyl O¹-tert-butyl 2-(1,4,7-tiiazonan-1-yl)pentanedioate (23.0 g) as a colorless oily substance.

MS (ESI, m/z): 406 [M + H]⁺

¹HNMR (CDCl₃, 300 MHz) δ: 7.34 (5H, s), 5.13 (2H, s), 3.22 (1H, dd, J = 8.6, 6.6 Hz), 2.87-2.62 (12H, m), 2.61-2.51 (2H, m), 2.11-1.85 (4H, m), 1.46 (9H, s)

(1-A-4)

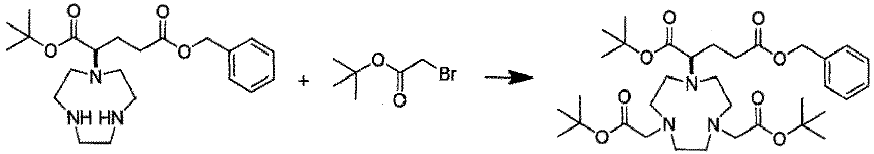

At a temperature of 3°C, potassium carbonate (19.6 g) and tert-butyl bromoacetate (16.5 mL) were added to an acetonitrile (230 mL) solution of (R)-O⁵-benzyl O¹-tert-butyl 2-(1,4,7-triazonan-1-yl)pentanedioate (23.0 g), followed by stirring for 5 hours at room temperature. The insoluble matter was removed by filtration, the solvent was distilled away under reduced pressure, and the obtained residue was purified by silica gel column chromatography (hexane/ethyl acetate = 100/0 to 85/15), thereby obtaining (R)-O⁵-benzyl O¹-tert-butyl 2-(4,7-bis(2-(tert-butoxy)-2-oxoethyl)-1,4,7-triazonan-1-yl)pentanedioate (30.7 g) as a colorless oily substance.

MS (ESI, m/z): 634 [M + H]⁺

¹HNMR (CDCl₃, 300 MHz) δ: 7.35 (5H, s), 5.13 (2H, s), 3.28 (4H, s), 3.18 (1H, dd, J = 8.9, 6.3 Hz), 2.98-2.65 (12H, m), 2.65-2.44 2H, m), 2.08-1.81 (2H, m), 1.44 (27H, s)

Optical purity: 96% ee

(1-A-5)

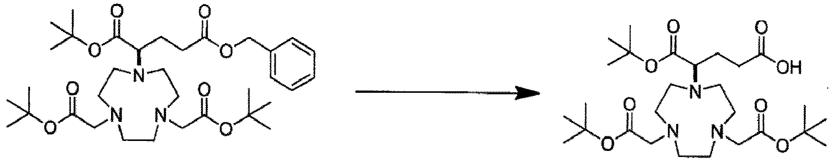

(R)-O⁵-benzyl O¹-tert-butyl 2-(4,7-bis(2-(tert-butoxy)-2-oxoethyl)-1,4,7-triazonan-1-yl)pentanedioate (30.6 g), tetrahydrofuran (150 mL), and palladium hydroxide/carbon (6.1 g) were put into an autoclave, followed by stirring for 3 hours at room temperature in a nitrogen atmosphere at 5.0 MPa. The insoluble matter was removed by celite filtration, the solvent was removed under reduced pressure, thereby obtaining a black oily substance. Ethyl acetate (150 mL) and active carbon (10 g) were added to the obtained oily substance. Then, the insoluble matter was removed by filtration, and the solvent was distilled away under reduced pressure, thereby obtaining (R)-4-(4,7-bis(2-(tert-butoxy)-2-oxoethyl)-1,4,7-triazonan-1-yl)-5-(tert-butoxy)-5-oxopentanoi c acid (24.2 g) as a light yellow solid.

LC/MS rt (min): 1.34

MS (ESI, m/z): 544.5 [M + H]⁺

¹HNMR (CDCl₃, 300 MHz) δ: 3.38 (4H, s), 3.32 (1H, dd, J = 9.8, 4.0 Hz), 3.19-2.93 (8H, m), 2.82 (4H, s), 2.74-2.64 (1H, m), 2.57-2.47 (1H, m), 2.07-1.89 (2H, m), 1.48 (9H, s), 1.45 (18H, s)

Optical purity: 98% ee

(1-B-1)

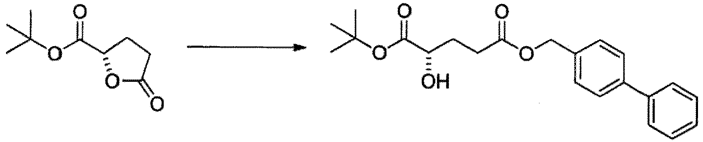

A 8.0 M aqueous potassium hydroxide solution (3.36 mL) was added to a tetrahydrofuran (20 mL) solution of (S)-tert-butyl 5-oxotetrahydrofuran-2-carboxylate (5.0 g), followed by stirring for 1 hour at 40°C. Water (23.5 mL) was added thereto, and the solution was stirred for 2 hours and then for 1.5 hours at 45°C. A 8.0 M aqueous potassium hydroxide solution (0.67 mL) and water (4.71 mL) were added thereto, followed by stirring for 2.5 hours, and the solvent was distilled away under reduced pressure. N,N'-dimethylformamide (20 mL) was added to the obtained solid, followed by stirring. Then, 4-bromomethylbiphenyl (5.98 g) was added thereto, followed by stirring for 16 hours. Water (150 mL) was added to the reaction mixture, extraction was performed twice by using ethyl acetate (50 mL). Thereafter, the organic layer was dried over anhydrous sodium sulfate, and the solvent was distilled away under reduced pressure. The obtained crude product was purified by silica gel column chromatography (CHROMATOREX (FUJI SILYSIA CHEMICAL LTD), hexane/ethyl acetate = 100/0 to 85/15), thereby obtaining a white solid (6.0 g). An ethyl acetate (8 mL) solution of the obtained solid (5.2 g) was stirred at 70°C such that the solid dissolved, and then hexane (72 mL) was added thereto, followed by stirring for 4 hours at room temperature. The precipitated solid was collected by filtration, washed with hexane, and dried under reduced pressure, thereby obtaining (S)-O⁵-([1,1'-biphenyl]-4-ylmethyl) O¹-tert-butyl 2-hydroxypentanedioate (4.28 g) as a white solid.

LC/MS rt (min): 1.78

MS (ESI, m/z): 371.3 [M + H]⁺

¹H-NMR (CDCl₃) δ: 7.62-7.56 (4H, m), 7.48-7.41 (4H, m), 7.39-7.32 (1H, m), 5.17 (2H, s), 4.13-4.07 (1H, m), 2.87 (1H, d, J = 5.3 Hz), 2.63-2.43 (2H, m), 2.23-2.12 (1H, m), 1.98-1.86 (1H, m), 1.49(9H, s)

Optical purity: 100% ee

(1-B-2)

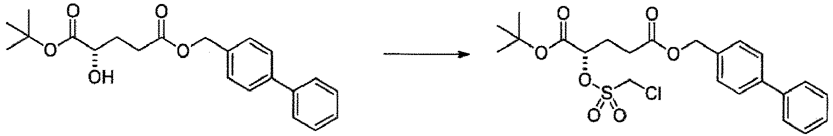

At a temperature of 0°C, 4-methylmorpholine (594 µL) and chloromethane sulfonyl chloride (436 µL) were added to a methylene chloride (5 mL) solution of (S)-O⁵-([1,1'-biphenyl]-4-ylmethyl) O¹-tert-butyl 2-hydroxypentanedioate (1.0 g), followed by stirring for 30 minutes. Water (20 mL) was added thereto, and then the organic layer was fractionated and dried over anhydrous sodium sulfate. Then, the solvent was distilled away under reduced pressure, thereby obtaining (S)-O⁵-([1,1'-biphenyl]-4-ylmethyl) O¹-tert-butyl 2-(((chloromethyl)sulfonyl)oxy)pentanedioate (1.46 g) as a light yellow oily substance.

LC/MS rt (min): 1.96

MS (ESI, m/z): 484.2 [M + H]⁺

¹H-NMR (CDCl₃) δ: 7.60-7.59 (4H, m), 7.48-7.41 (4H, m), 7.38-7.34 (1H, m), 5.19 (2H, s), 5.09 (1H, dd, J = 8.3, 4.3 Hz), 4.73 (2H, dd, J = 17.5, 12.2 Hz), 2.68-2.50 (2H, m), 2.43-2.15

### (2H, m)

(I-B-3)

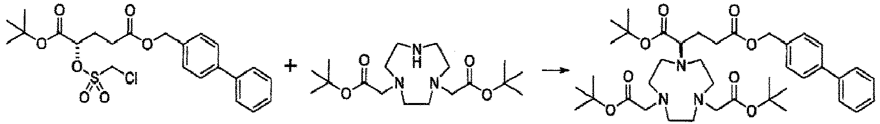

Potassium carbonate (57 mg) was added to an acetonitrile (1 mL) solution of di-tert-butyl 2,2'-(l,4,7-triazonane-l,4-diyl) diacetate (89 mg), followed by stirring for 5 minutes. An acetonitrile (1 mL) solution of (S)-O⁵-([1,1-biphenyl]-4-ylmethyl) O¹-tert-butyl 2-tert-butyl 2-(((chloromethyl)sulfonyl)oxy)pentanedioate (100 mg) was added to the obtained mixture, followed by stirring for 20 minutes. Water (2 mL) was added thereto, extraction was then performed twice by using ethyl acetate (3 mL), and the organic layer was fractionated. The solvent was distilled away under reduced pressure, and the residue was purified by silica gel column chromatography (KP-NH (Biotage), hexane/ethyl acetate = 100/0 to 80/20), thereby obtaining (R)-O⁵-([1,1'-biphenyl]-4-ylmethyl) O¹-tert-butyl 2-(4,7-bis(2-(tert-butoxy)-2-oxoetyl)-1,4,7-triazonan-1-yl)pentanedioate (126 mg) as a light yellow oily substance.

LC/MS rt (min): 1.96

MS (ESI, m/z): 710.5 [M + H]⁺

¹H-NMR (CDC13) δ: 7.62-7.56 (4H, m), 7.48-7.41 (4H, m), 7.39-7.32 (1H, m), 5.17 (2H, s), 3.28 (4H, s), 3.19 (1H, dd, J = 8.9, 6.3 Hz), 2.98-2.67 (12H, m), 2.65-2.46 (2H, m), 2.08-1.84 (2H, m), 1.44 (27H, s)

Optical purity: 98.9% ee

(I-B-4)

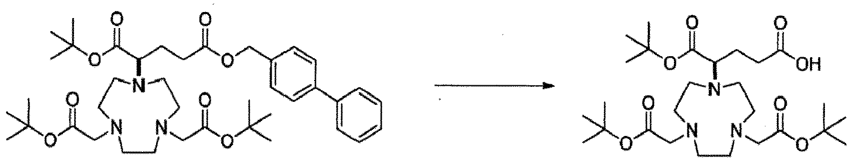

10% palladium hydroxide on carbon (340 mg) and a tetrahydrofuran (17 mL) solution of (R)-O⁵-([1,1'-biphenyl]-4-ylmethyl) O¹-tert-butyl 2-(4,7-bis(2-(tert-butoxy)-2-oxoetyl)-1,4,7-triazonan-1-yl)pentanedioate (1.71 g) were put into an autoclave, followed by stirring for 4 hours at room temperature in a nitrogen atmosphere at 0.9 MPa. The insoluble matter was removed by filtration, and the solvent was distilled away under reduced pressure. Ethyl acetate (8.5 mL) and activated carbon (510 mg) were added to the obtained black oily substance, followed by stirring for 15 minutes at room temperature. The insoluble matter was removed by filtration by using celite, and the solvent was distilled away under reduced pressure, thereby obtaining a brown oily substance (1.49 g). An aqueous solution (10 mL) was added to the obtained oily substance (500 mg), followed by stirring for 30 minutes with ice cooling, and the insoluble matter was removed by filtration. Extraction was performed twice for the obtained filtrate by using chloroform (10 mL), and the solvent was distilled away under reduced pressure, thereby obtaining (R)-4-(4,7-bis(2-(tert-butoxy)-2-oxoethyl)-1,4,7-triazonan-1-yl)-5-(tert-butoxy)-5-oxopentanoi c acid (456 mg) as a yellow oily substance.

LC/MS rt (min): 1.34

MS (ESI, m/z): 544.5 [M + H]⁺

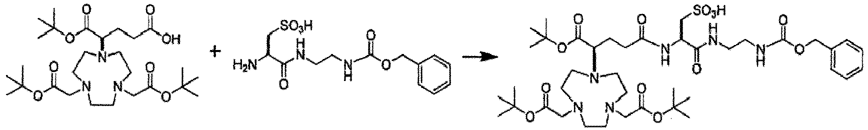

A mixture of (R)-4-(4,7-bis(2-(tert-butoxy)-2-oxoethyl)-1,4,7-triazonan-1-yl)-5-(tert-butoxy)-5-oxopentanoi c acid (22.0 g), DMAc (150 mL), DIEA (16.9 mL), and (R)-2-amino-3-((2-(((benzyloxy)carbonyl)amino)ethyl)amino)-3-oxopropane-1-sulfonic acid (14.7 g) was stirred for 20 minutes at room temperature, and then HBTU (16.9 g) was added thereto, followed by stirring for 1.5 hour at room temperature. The mixture was cooled to 7°C, and then an aqueous saturated sodium chloride solution (600 mL) and ethyl acetate (600 mL) were added thereto, followed by stirring. The organic layer was fractionated, washed twice with an aqueous saturated sodium hydrogen carbonate solution (600 mL), and dried over sodium sulfate, and the solvent was distilled away under reduced pressure, thereby obtaining (R)-3-((2-(((benzyloxy)carbonyl)amino)ethyl)amino)-2-((R)-4-(4,7-bis(2-(tert-butoxy)-2-oxoe thyl)-1,4,7-triazonan-1-yl)-5-(tert-butoxy)-5-oxopentanamide)-3-oxopropane-1-sulfonic acid (37.2 g) as a yellow solid.

LC/MS rt (min): 1.45

MS (ESI, m/z): 871 [M + H]⁺

¹H-NMR (CDCl₃, 300 MHz) δ: 7.52 (1H, brs), 7.30 (5H, m), 6.10 (1H, brs), 5.06 (2H, brs), 4.84 (1H, brs), 3.80-1.67 (29H, m), 1.47-1.41 (27H, m)

HPLC (Waters BEH C18, formic acid-based, gradient cycle: 0 min (A solution/B solution = 50/50), 15 min (A solution/B solution = 0/100), 18 min (A solution/B solution = 0/100), flow rate: 0.4 mL/min) rt (min)): 8.95

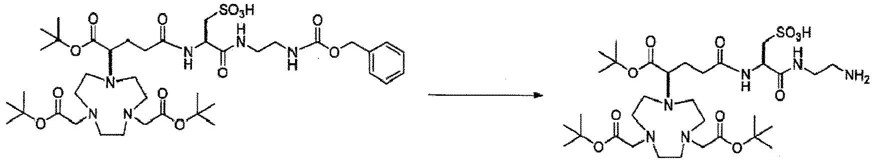

Palladium hydroxide on carbon (3.7 g), ethanol (250 mL), and (R)-3-((2-(((benzyloxy)carbonyl)amino)ethyl)amino)-2-((R)-4-(4,7-bis(2-(tert-butoxy)-2-oxoe thyl)-1,4,7-triazonan-l-yl)-5-(tert-butoxy)-5-oxopentanamide)-3-oxopropane-l-sulfonic acid (37.0 g) were put into an autoclave, followed by stirring for 3 hours in a nitrogen atmosphere at 4.0 MPa. The insoluble matter was removed by filtration, and the solvent was distilled away under reduced pressure. Ethanol (150 mL) and active carbon (8 g) were added to the obtained oily substance, followed by stirring for 30 minutes at room temperature, the insoluble matter was removed by filtration, and the solvent was distilled away under reduced pressure, thereby obtaining (R)-3-((2-aminoethyl)amino)-2-((R)-4-(4,7-bis(2-(tert-butoxy)-2-oxoethyl)-1,4,7-triazonan-1-yl)-5-(tert-butoxy)-5-oxopentanamide)-3-oxopropane-1-sulfonic acid (30.0 g) as a black solid.

LC/MS rt (min): 1.13

MS (ESI, m/z): 737 [M + H]⁺

¹H-NMR (CDCl₃, 300 MHz) δ: 7.72 (1H, brs), 4.83 (1H, brs), 3.75-1.82 (31H, m), 1.46 (27H, s)

HPLC (Waters BEH, formic acid-based, gradient cycle: 0 min (A solution/B solution = 50/50), 7 min (A solution/B solution = 40/60), 15 min (A solution/B solution = 0/100), flow rate: 0.4 mL/min) rt (min)): 6.70

### Reference Example 8

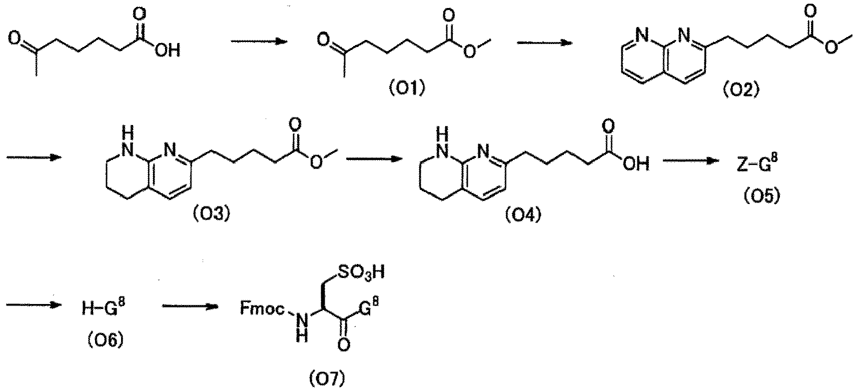

(1) Concentrated sulfuric acid (20 mL) was added to a methanol (1 L) solution of 6-oxopentaonic acid (99.2 g), and the mixture was heated for 4 hours under reflux. After the reaction mixture was cooled to room temperature, the solvent was distilled away under reduced pressure, and water (1 L) and ethyl acetate (600 mL) were added thereto. The organic layer was fractionated and washed with a 5% aqueous sodium hydrogen carbonate solution (600 mL) and an aqueous saturated sodium chlorides solution (600 mL), and the solvent was distilled away under reduced pressure, thereby obtaining (01) (95.2 g).
   TLC Rf: 0.45 (hexane/ethyl acetate = 2/1)
(2) (01) (189 g) and methanol (600 mL) were added to a mixture of 2-aminonicotinaldehyde (133 g) and methanol (500 mL), and then pyrrolidine (100 mL) was added thereto, and the mixture was heated for 8 hours under reflux. The reaction mixture was cooled to room temperature, the solvent was distilled away under reduced pressure, toluene (100 mL) was added thereto, and the solvent was distilled away under reduced pressure. Toluene (150 mL) was added to the obtained residue, followed by stirring for 2 hours at 50°C and then for 3 hours at room temperature, and the solid was collected by filtration, thereby obtaining (02) (149 g).
   TLC Rf: 0.56 (hexane/ethyl acetate = 5/1)
   LC/MS rt (min): 0.73
   MS (ESI, m/z): 245.2 [M + H]⁺
(3) 10% palladium on carbon (10.0 g), (02) (97.5 g), and methanol (250 mL) were put into an autoclave, followed by stirring for 8 hours in a hydrogen atmosphere at 5 MPa. The insoluble matter was removed by filtration, and the solvent was distilled away under reduced pressure. Acetonitrile (100 mL) was added to the obtained residue, and the solid was collected by filtration, thereby obtaining (03) (71.5 g).
   ¹H-NMR (CDCl₃, 300 MHz) δ: 7.05 (1H, d, J = 7.5 Hz), 6.34 (1H, d, 7.5 Hz), 4.74 (1H, brs ), 3.66 (3H, s), 3.37-3.42 (2H, m), 2.68 (2H, t, J = 6.0 Hz), 2.52-2.57 (2H, m), 2.30-2.37 (2H, m), 1.90 (2H, tt, J = 5.7, 6.0 Hz), 1.63-1.70 (4H, m)
   HPLC (Waters 600E system (Waters) (column: CAPCELL PAK C18MG, 4.6 × 150 mm (Shiseido Japan Co., Ltd.), solvent: A solution = formic acid:water (1:1,000), B solution = formic acid:methanol:acetonitrile (1:800:200), gradient cycle: 0 min (A solution/B solution = 80/20), 10 min (A solution/B solution = 0/100), 15 min (A solution/B solution = 0/100), flow rate: 1.0 mL/min) rt (min): 8.06
(4) Methanol (210 mL) was added to (03) (70.0 g), and the mixture was dissolved by being heated to 40°C. Then, a mixture of sodium hydroxide (16.9 g) and water (105 mL) was added dropwise thereto for 15 minutes, followed by stirring for 1 hour at 40°C. The solvent was distilled away under reduced pressure, and water (210 mL) was added thereto. The solution was heated to 40°C, and concentrated hydrochloric acid was added dropwise thereto until the pH became 5, at a temperature kept to be equal to or lower than 50°C. Water (50 mL) was added thereto, the solution was cooled to room temperature and left to stand overnight. The solid was collected by filtration, thereby obtaining (04) (62.2 g).
   LC/MS rt (min): 0.62
   MS (ESI, m/z): 235.2 [M + H]⁺
   HPLC (Waters 600E system (Waters) (column: CAPCELL PAK C18MG, 4.6 × 150 mm (Shiseido Japan Co., Ltd.), solvent: A solution = formic acid:water (1:1,000), B solution = formic acid:methanol:acetonitrile (1:800:200), gradient cycle: 0 min (A solution/B solution = 80/20), 10 min (A solution/B solution = 0/100), 15 min (A solution/B solution = 0/100), flow rate: 1.0 mL/min) rt (min): 7.03
(5) HBTU (4.98 g) was added little by little to a mixture of methyl (2S)-3-amino-2-((4-(4-((2-(benzyloxycarbonylamino)ethyl)amino)-4-oxobutoxy)-2,6-dimethyl phenyl)sulfonylamino)propanoate (7.40 g), (04) (3.37 g), DMF (50 mL), and DIEA (3.86 mL), followed by stirring for 2 hours at room temperature. A 5% aqueous sodium hydrogen carbonate solution (200 mL) and ethyl acetate (200 mL) were added to the reaction mixture, followed by stirring for 10 minutes at room temperature. The organic layer was fractionated, washed three times with an aqueous saturated sodium chloride solution, and dried over anhydrous sodium sulfate, and then the solvent was distilled away under reduced pressure. Ethyl acetate (50 mL) was added to the obtained residue, and the solid was collected by filtration, thereby obtaining (05) (9.20 g).
   LC/MS rt (min): 1.12
   MS (ESI, m/z): 781.5 [M + H]⁺, 779.6 [M - H]⁻
(6) Methanol (40 mL) was added to (05) (7.20 g) and 10% Pd/C (300 mg), followed by stirring for 3 hours at room temperature in a hydrogen atmosphere. The insoluble matter was removed by filtration, and the solvent was distilled away under reduced pressure. Toluene (50 mL) was added to the obtained residue, and the solvent was distilled away under reduced pressure, thereby obtaining (06) (5.45 g).
   LC/MS rt (min): 0.73
   MS (ESI, m/z): 647.4 [M + H]⁺
(7) A DMF (1.5 mL) solution of HBTU (141 mg) was added to a solution of (06) (120 mg), Fmoc-cysteic acid (145 mg), DMF (2 mL), and DIEA (140 ¡.tL), followed by stirring for 20 minutes at room temperature. Water (2 mL) was added thereto, and the resultant was purified by preparative HPLC, thereby obtaining (07) (87.7 mg).
   LC/MS (LCMS-2010EV (Shimadzu Corporation) (column: SunFire C 18 4.6 × 150 mm (Waters), solvent: A solution = 0.1% formic acid/water, B solution = 0.1% formic acid/methanol:acetonitrile (4:1), gradient cycle: 0 min (A solution/B solution = 80/20), 10 min (A solution/B solution = 0/100), 15 min (A solution/B solution = 0/100), flow rate: 1 mL/min) rt (min): 11.83
   MS (ESI, m/z): 1020.25 [M + H]⁺, 1018.50 [M - H]⁻ 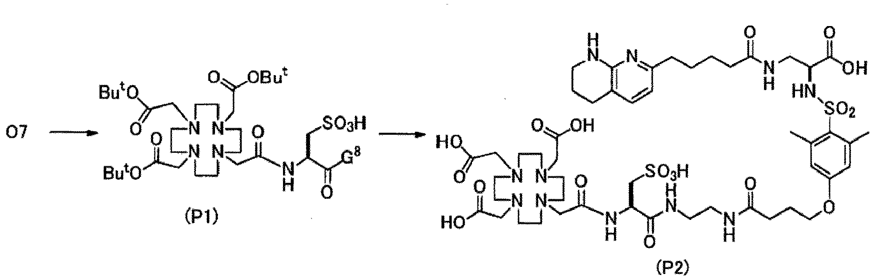
(8) Diethylamine (0.5 mL) was added to a DMF (0.5 mL) solution of (07) (28.1 mg), followed by stirring for 1.5 hours at room temperature. The solvent was distilled away under reduced pressure, DMF (400 µL) and DIEA (20 µL) were added, and then a DMF (150 µL) solution of tri-tert butyl 1,4,7,10-tetraazacyclododecane-1,4,7,10-tetraacetic acid (31.6 mg), DMF (150 ¡.tL), DIEA (20 ¡.tL), and HBTU (20.9 mg) was added thereto, followed by stirring for 45 minutes at room temperature. Water (500 µL) was added thereto, extraction was performed three times by using hexane/ethyl acetate (1/1) (0.5 mL), and the extract was purified by preparative HPLC, thereby obtaining (pi) (19.6 mg).
   LC/MS rt (min): 1.12
   MS (ESI, m/z): 1352.5 [M + H]⁺, 1350.6 [M - H]⁻
   HPLC (Waters 600E system (Waters) (column: SunFire C180BD, 4.6 × 150 mm (Waters), solvent: A solution = formic acid:water (1:1,000), B solution = formic acid:methanol:acetonitrile (1:800:200), gradient cycle: 0 min (A solution/B solution = 80/20), 10 min (A solution/B solution = 0/100), 15 min (A solution/B solution = 0/100), flow rate: 1.0 mL/min) rt (min): 9.71
(9) THF (1.4 mL), water (200 ¡.tL), and a 3 mol/L aqueous lithium hydroxide solution (200 µL) were added to (PI) (11.8 mg), followed by stirring for 1.5 hours at room temperature. TFA was added thereto, and the solvent was distilled away under reduced pressure. TFA/triethylsilane (95/5) (1 mL) were added to the obtained residue, followed by stirring for 100 minutes at room temperature. The solvent was distilled away under reduced pressure, water/acetonitrile (2/1) (1.8 mL) and formic acid (1.8 µL) were added thereto, and the obtained resultant was purified by preparative HPLC, thereby obtaining (P2) (compound A) (8.9 mg).
   LC/MS rt (min): 0.75
   MS (ESI, m/z): 1170.4 [M + H]⁺, 585.9 [M + 2H]²⁺, 1168.4 [M - H]⁻
   HPLC (Waters 600E system (Waters) (column: SunFire C180BD, 4.6 × 150 mm (Waters), solvent: A solution = formic acid:water (1:1,000), B solution = formic acid:methanol:acetonitrile (1:800:200), gradient cycle: 0 min (A solution/B solution = 80/20), 10 min (A solution/B solution = 0/100), 15 min (A solution/B solution = 0/100), flow rate: 1.0 mL/min) rt (min): 8.75

### Reference Example 9

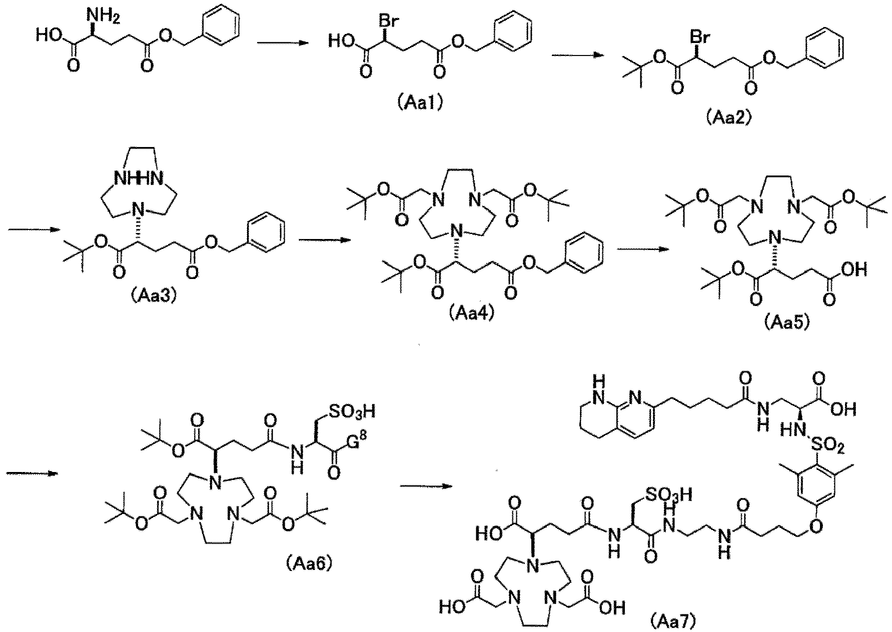

(1) Sodium nitrite (2.6 g) was added for 10 minutes to a mixture of L-glutamic acid γ benzyl ester (5.0 g), water (10 mL), sodium bromide (7.6 g), and hydrobromic acid (6 mL) at a temperature of equal to or lower than 5°C, followed by stirring for 2 hours at 5°C. Diisopropylether and concentrated sulfuric acid (2 mL) were added to the reaction mixture, and the organic layer was fractionated, sequentially washed with water and an aqueous saturated sodium chloride solution, and then dried over anhydrous sodium sulfate. The solvent was distilled away under reduced pressure, and the obtained residue was purified by silica gel column chromatography (hexane/ethyl acetate = 1/1), thereby obtaining (Aa1) (3.1 g).
   LC/MS rt (min): 1.32
   MS (ESI, m/z): 301.1 [M + H]⁺
   ¹H-NMR (300 MHz, CDCl₃) δ: 7.31-7.38 (5H, m), 5.1 (2H, s), 4.41 (1H, dd, J = 6.0, 7.8 Hz), 2.58-2.63 (2H, m), 2.25-2.50 (2H, m)
(2) A mixture of tert-butyl 2,2,2-trichloroacetimidate (4.3 mL) and hexane (12 mL) was added for 20 minutes to a chloroform (15 mL) solution of (Aa1) (3.1 g) at room temperature. DMAc (1.5 mL) and BF₃·OEt₂ (220 µL) were added thereto, followed by stirring for 40 minutes at room temperature, the solvent was distilled away under reduced pressure, and the obtained resultant was purified by silica gel column chromatography (hexane/ethyl acetate = 95/5 to 85/15), thereby obtaining (Aa2) (2.84 g).
   ¹H-NMR (300 MHz, CDCl₃) δ: 7.31-7.38 (5H, m), 5.14 (2H, s), 4.24 (1H, dd, J = 6.0, 8.7 Hz), 2.53-2.59 (2H, m), 2.19-2.43 (2H, m), 1.47 (9H, s)
(3) A chloroform (50 mL) solution of (Aa2) (1.70 g) was added for 90 minutes to a chloroform (60 mL) solution of 1,4,7-triazacyclononane (1.84 g), followed by stirring for 3 days at room temperature. The solvent was distilled away under reduced pressure, and the obtained resultant was purified by silica gel column chromatography (hexane/ethyl acetate = 50/50 to 0/100 and then ethyl acetate/methanol = 80/20), thereby obtaining (Aa3) (0.76 g).
   LC/MS rt (min): 0.91
   MS (ESI, m/z): 406.5 [M + H]⁺
(4) Tert-butyl bromoacetate (580 µL) was added to a mixture of (Aa3) (0.76 g), DMAc (7 mL), and potassium carbonate (607 mg), followed by stirring for 2 hours at room temperature. Ethyl acetate (30 mL) and water (30 mL) were added thereto, and the organic layer was fractionated, sequentially washed twice with water (30 mL) and once with an aqueous saturated sodium chloride solution (30 mL), and dried over anhydrous sodium sulfate. The solvent was distilled away under reduced pressure, and the obtained residue was purified by silica gel column chromatography (hexane/ethyl acetate = 95/5 to 60/40), thereby obtaining (Aa4) (1.04 g).
   LC/MS rt (min): 1.63
   MS (ESI, m/z): 634.7 [M + H]⁺
(5) (Aa 4) (0.28 g), isopropyl alcohol (20 mL), water (0.5 mL), and 10% palladium on carbon (0.10 g) were put into a sealed tube, followed by stirring for 7 hours in a hydrogen atmosphere at 0.5 MPa. The insoluble matter was removed by filtration, and the solvent was distilled away under reduced pressure, thereby obtaining (Aa5) (0.24 g).
   LC/MS rt (min): 1.34
   MS (ESI, m/z): 544.7 [M + H]⁺
(6) HBTU (64.5 mg) was added to a mixture of (Aa5) (94.9 mg), (R)-2-amino-3-((2-(4-(4-(N-((S)-1-methoxy-1-oxo-3-(5-(5,6,7,8-tetrahydro-1,8-naphthyridin-2 -yl)pentanamide)propan-2-yl)sulfamoyl)-3,5-dimethylphenoxy)butanamide)ethyl)amino)-3-ox opropane-3,5-dimethylphenoxy)butanamide)ethyl)amino)-3-oxopropan-1-sulfonic acid (104 mg), DMF (0.8 mL), and N,N-diisopropylethylamine (61 ¡.tL), followed by stirring for 35 minutes at room temperature. Water (1.1 mL) and acetonitrile (0.8 mL) were added thereto, followed by stirring, and then the resultant was purified by preparative HPLC, thereby obtaining (Aa6) (151 mg).
   LC/MS rt (min): 1.28
   MS (ESI, m/z): 1324.2 [M + H]⁺, 1322.2 [M - H]⁻
   HPLC (Waters 600E system (Waters) (column: CAPCELL PAK C18MG, 4.6 × 150 mm (Shiseido Japan Co., Ltd.), solvent: A solution = formic acid:water (1:1,000), B solution = formic acid:methanol:acetonitrile (1:800:200), gradient cycle: 0 min (A solution/B solution = 80/20), 10 min (A solution/B solution = 0/100), 15 min (A solution/B solution = 0/100), flow rate: 1.0 mL/min) rt (min): 11.82
(7) Concentrated hydrochloric acid (2.5 mL) was added to (Aa6) (73 mg), followed by stirring for 2 days at room temperature, and the resultant was concentrated under reduced pressure. The resultant was diluted with 50% hydrous acetonitrile (2 mL) and then purified by preparative HPLC, thereby obtaining (Aa7) (compound B) (33.3 mg).
   LC/MS rt (min): 0.77
   MS (ESI, m/z): 1141.8 [M + H]⁺, 1139.8 [M - H]⁻
   HPLC (Waters 600E system (Waters) (column: CAPCELL PAK C18MG, 4.6 × 150 mm (Shiseido Japan Co., Ltd.), solvent: A solution = formic acid:water (1:1,000), B solution = formic acid:methanol:acetonitrile (1:800:200), gradient cycle: 0 min (A solution/B solution = 80/20), 10 min (A solution/B solution = 0/100), 15 min (A solution/B solution = 0/100), flow rate: 1.0 mL/min) rt (min): 9.37

### Reference Example 10

In the present reference example, the compound A obtained in Reference Example 8 and the compound B obtained in Reference Example 9 were used.
(A) A indium [¹¹¹In] chloride solution (80 MBq, 100 µL) was added to a mixture of the compound A (8.5 µg) and a 0.2 mol/L sodium acetate buffer solution (pH 4.0) (1.5 mL). The solution was heated to 100°C for 15 minutes and then left to stand for 5 minutes at room temperature, thereby obtaining [¹¹¹In]-(compound A). As a result of analyzing the compound by using reversed-phase TLC (Whatman, KC18F, developing solvent: methanol/0.5 mol/ L aqueous sodium acetate solution (50/50)), the Rf value of the radiolabeled compound was found to be 0.4. The radiochemical purity measured immediately after the compound was prepared and measured after 24 hours at room temperature was equal to or higher than 95%.
(B) A yttrium [⁹⁰Y] chloride solution (700 MBq, 240 µL) was added to a mixture of the compound (79 µg), gentisic acid (1.8 mg), a 0.6 mol/L sodium acetate buffer solution (pH 4.0, 120 ¡.tL), and 0.4 mol/L aqueous sodium hydroxide solution (24 µL). The solution was heated to 100°C for 20 minutes and then left to stand for 5 minutes at room temperature, thereby obtaining [90^{Y}]-(compound A). As a result of analyzing the compound by using reversed-phase TLC (Whatman, KC18F, developing solvent: methanol/0.5 mol/ L aqueous ammonium acetate solution (50/50)), the Rf value of the radiolabeled compound was found to be 0.4. The radiochemical purity measured immediately after the compound was prepared and measured after 24 hours at room temperature was equal to or higher than 95%.
(C) A copper [⁶⁴Cu] chloride solution (pH 5, 35 MBq, 55 µL) was added to a mixture of the compound A (5.8 µg) and 0.2 mol/L sodium acetate buffer solution (pH 4.0, 219 µL). The solution was heated to 100°C for 15 minutes and left to stand for 5 minute at room temperature, thereby obtaining [⁶⁴Cu]-(compound A). As a result of analyzing the compound by using reversed-phase TLC (Whatman, KC18F, developing solvent: methanol/0.5 mol/ L aqueous ammonium acetate solution (50/50)), the Rf value of the radiolabeled compound was found to be 0.4. The radiochemical purity measured immediately after the compound was prepared and measured after 22 hours at room temperature was equal to or higher than 90%.
(D) A 0.2 mol/L sodium acetate buffer solution of Copper [⁶⁴Cu] chloride (pH 4.0) (40 MBq, 155 µL) was added to a mixture of the compound B (4.2 µg), gentisic acid (1 mg), and a 0.2 mol/L sodium acetate buffer solution (pH 4.0) (5.0 µL). The solution was heated to 100°C for 15 minutes and left to stand for 5 minutes at room temperature, thereby obtaining [⁶⁴Cu]-(compound B). As a result of analyzing the compound by using reversed-phase TLC (Merck, RP-8 F_{254S}, developing solvent: methanol/0.5 mol/ L aqueous ammonium acetate solution (50/50)), the Rf value of the radiolabeled compound was found to be 0.4. The radiochemical purity measured immediately after the compound was prepared and measured after 24 hours at room temperature was equal to or higher than 90%.

### Example 1

(1)

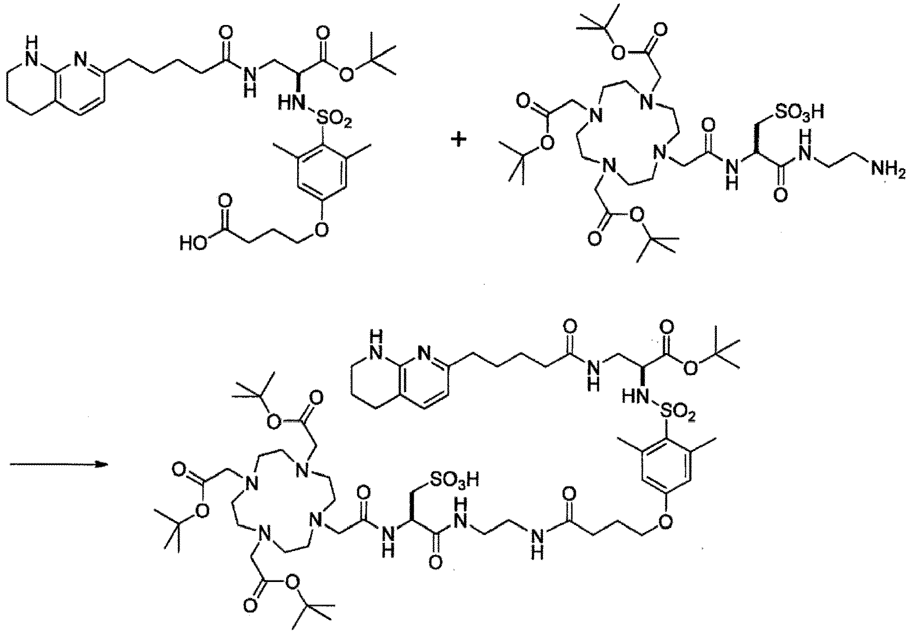

HBTU (0.252 g) was added to a mixture of (S)-4-(4-(N-(1-(tert-butoxy)-1-oxo-3-(5-(5,6,7,8-tetrahydro-1,8-naphthyridin-2-yl)pentanamid e)propan-2-yl)sulfamoyl)-3,5-dimethylphenoxy)butanoic acid (0.500 g), (R)-3-((2-aminoethyl)amino)-3-oxo-2-(2-(4,7,10-tris(2-(tert-butoxy)-2-oxoethyl)-1,4,7,10-tetr aazacyclododecan-1-yl)acetamide)propane-1-sulfonic acid (0.711 g), DIEA (0.328 mL), and DMAc (5.0 mL), followed by stirring for 10 minutes. Then, HBTU (0.100 g) was further added thereto, followed by stirring for 2 hours. Water (15 mL) was added to the reaction mixture, followed by stirring for 10 minutes. Thereafter, water (10 mL) was added thereto, followed by stirring for 30 minutes. The supernatant liquid was removed, water (10 mL) was added to the residue, followed by stirring for 10 minutes. Subsequently, the supernatant liquid was removed, methanol (10 mL) was added thereto, and the solvent was distilled away under reduced pressure. The obtained residue was dissolved in ethyl acetate (2.26 mL) containing 12% methanol and purified by silica gel column chromatography (NH silica gel, methanol/ethyl acetate = 3/97 to 30/70), thereby obtaining (R)-3-((2-(4-(4-(N-((S)-1-(tert-butoxy)-1-oxo-3-(5-(5,6,7,8-tetrahydro-1,8-naphthyridin-2-yl)p entanamide)propan-2-yl)sulfamoyl)-3,5-dimethylphenoxy)butanamide)ethyl)amino)-3-oxo-2-( 2-(4,7,10-tris(2-(tert-butoxy)-2-oxoethyl)-1,4,7,10-tetraazacyclododecan-1-yl)acetamide)propa ne-1-sulfonic acid (0.776 g) as a white amorphous solid.

MS (ESI, m/z): 1394 [M + H]⁺

¹H-NMR (CDCl₃) δ: 8.57-8.29 (1H, m), 8.14-8.00 (1H, m), 7.10-6.97 (2H, m), 6.66 (2H, s), 6.33 (1H, d, J = 7.3 Hz), 6.30-6.22 (1H, m), 4.98-4.84 (1H, m), 4.74-4.61 (1H, m), 4.08-3.94 (2H, m), 3.78-1.55 (59H, m), 1.51-1.38 (27H, m), 1.30 (9H, s)

HPLC (TSKgel ODS-100Z) rt (min): 15.84

(2-1)

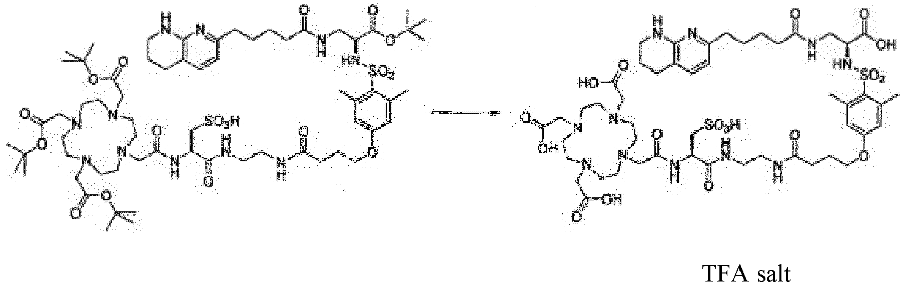

At room temperature, A mixture of TFA/triethylsilane (100 mL/13.7 mL) was added for 10 minutes to (R)-3-((2-(4-(4-(N-((S)-1-(tert-butoxy)-1-oxo-3-(5-(5,6,7,8-tetrahydro-1,8-naphthyridin-2-yl)p entanamide)propan-2-yl)sulfamoyl)-3,5-dimethylphenoxy)butanamide)ethyl)amino)-3-oxo-2-( 2-(4,7,10-tris(2-(tert-butoxy)-2-oxoethyl)-1,4,7,10-tetraazacyclododecan-1-yl)acetamide)propa ne-1-sulfonic acid (20.0 g), followed by stirring for 7 hours. TFA was distilled away under reduced pressure, and an operation of adding acetonitrile (50 mL) to the obtained residue and distilling away the solvent was repeated twice. The obtained residue was dissolved in acetonitrile (80 mL), and TBME (160 mL) was added thereto at room temperature, followed by stirring for 30 minutes. The precipitated solid was collected by filtration, thereby obtaining a TFA salt (21.3 g) of 2,2',2"-(10-(2-(((R)-1-((2-(4-(4-(N-((S)-1-carboxy-2-(5-(5,6,7,8-tetrahydro-1,8-naphthyridin-2-yl)pentanamide)ethyl)sulfamoyl)-3,5-dimethylphenoxy)butanamide)ethyl)amino)-1-oxo-3-s ulfopropan-2-yl)amino)-2-oxoethyl)-1,4,7,10-tetraazacyclododecane-1,4,7-triyl)triacetic acid (compound A) as a white solid.

MS (ESI, m/z): 1170 [free from M + H]⁺

(2-2)

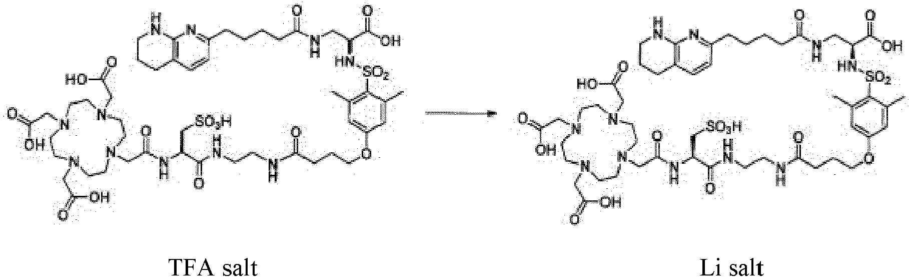

Water (200 mL) and lithium carbonate (5.0 g) were added to the solid obtained in (2-1) such that the pH was adjusted to be 8.3. A reversed-phase silica gel column (inner diameter of glass column: 10.5 cm, Daisogel-SR120-40/60-ODS-RPS: 400g) was charged with the reaction mixture, and elution was performed under a normal pressure by using 5% methanol-containing water (400 mL), 10% methanol-containing water (1,200 mL), 20% methanol-containing water (800 mL), and 30% methanol-containing water (1,600 mL) in this order, thereby obtaining a lithium salt (12.3 g) of the compound A as a white amorphous solid. MS (ESI, m/z): 1170 [free from M + H]⁺

(2-3)

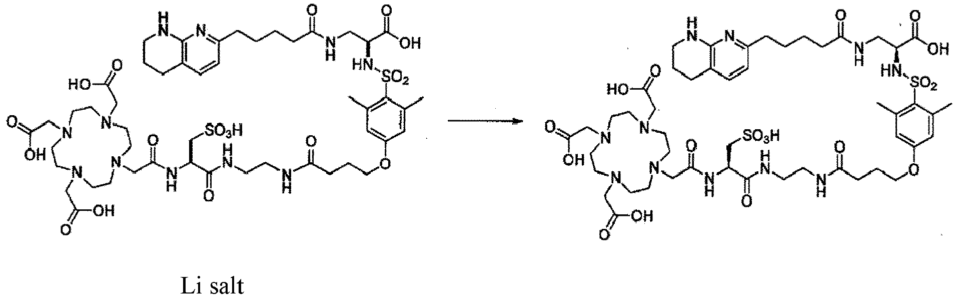

Water (120 mL) and formic acid (5.0 mL) were added to the solid (12.3 g) obtained in (2-2). A reversed-phase silica gel column (inner diameter of glass column: 10.5 cm, Daisogel-SR120-40/60-ODS-RPS: 400g) was charged with the reaction mixture, and elution was performed under a normal pressure by using 0.1% formic acid/5% acetonitrile-containing water (800 mL), 10% acetonitrile-containing water (800 mL), and 30% acetonitrile-containing water (2,400 mL) in this order, thereby obtaining a compound A (11.1 g) as a white solid.

LC/MS rt (min): 0.75

MS (ESI, m/z): 1170.4 [M + H]⁺, 1168.4 [M - H]⁻,

¹H-NMR (D₂O) δ: 7.49 (1H, d, J = 7.3 Hz), 6.71 (2H, s), 6.50 (1H, d, J = 7.3 Hz), 4.67 (1H, dd, J = 7.9, 5.0 Hz), 4.00 (2H, t, J = 5.9 Hz), 3.93-3.02 (34H, m), 2.72 (2H, t, J = 6.1 Hz), 2.59 (2H, t, J = 7.3 Hz), 2.34 (2H, t, J = 7.3 Hz), 2.12-1.80 (6H, m), 1.62-1.38 (4H, m)

HPLC (TSKgel ODS-100Z, formic acid-based) rt (min): 11.17

### Example 2

(1)

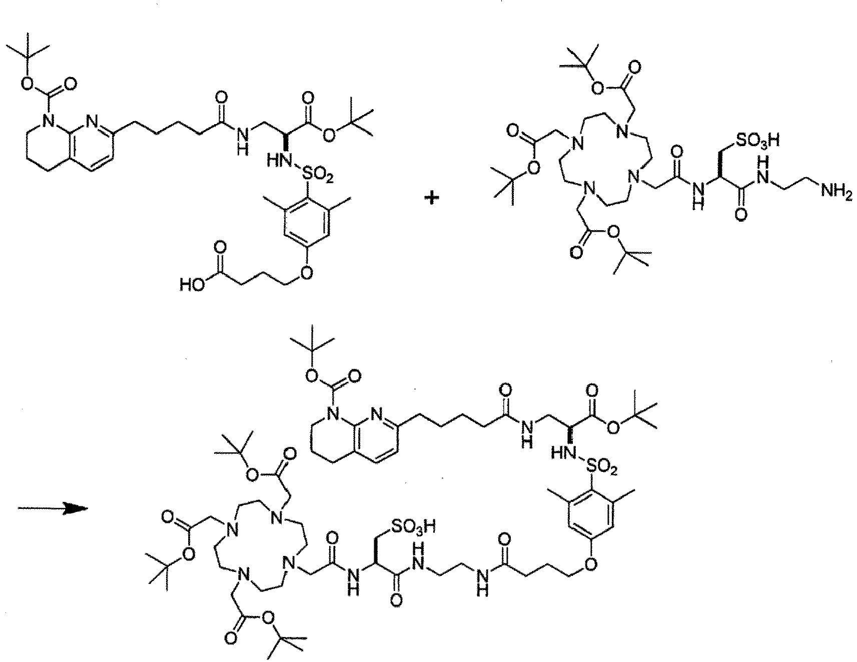

HBTU (509 mg) was added to a NMP (4 mL) solution of (S)-4-(4-(N-(1-(tert-butoxy)-3-(5-(8-(tert-butoxycarbonyl)-5,6,7,8-tetrahydro-1,8-naphthyridin -2-yl)pentanamide)-1-oxopropan-2-yl)sulfamoyl)-3,5-dimethylphenoxy)butanoic acid (668 mg), (R)-3-((2-aminoethyl)amino)-3-oxo-2-(2-(4,7,10-tris(2-(tert-butoxy)-2-oxoethyl)-1,4,7,10-tetr aazacyclododecan-1-yl)acetamide)propane-1-sulfonic acid (0.719 mg), and DIEA (187 ¡.tL), followed by stirring for 2 hours at room temperature. Then, water (50 mL) was added thereto, followed by stirring. The generated solid was collected by filtration and purified by silica gel column chromatography (hexane/ethyl acetate = 25/75 to 0/100 and then ethyl acetate/methanol = 70/30)), thereby obtaining (R)-3-((2-(4-(4-(N-((S)-1-(tert-butoxy)-3-(5-(8-(tert-butoxycarbonyl)-5,6,7,8-tetrahydro-1,8-n aphthyridin-2-yl)pentanamide)-1-oxopropan-2-yl)sulfamoyl)-3,5-dimethylphenoxy)butanamid e)ethyl)amino)-3-oxo-2-(2-(4,7,10-tris(2-(tert-butoxy)-2-oxoethyl)-1,4,7,10-tetraazacyclodode can-1-yl)acetamide)propane-1-sulfonic acid (381 mg) as a white amorphous solid.
LC/MS rt (min): 1.27
MS (ESI, m/z): 1495 [M + H]⁺

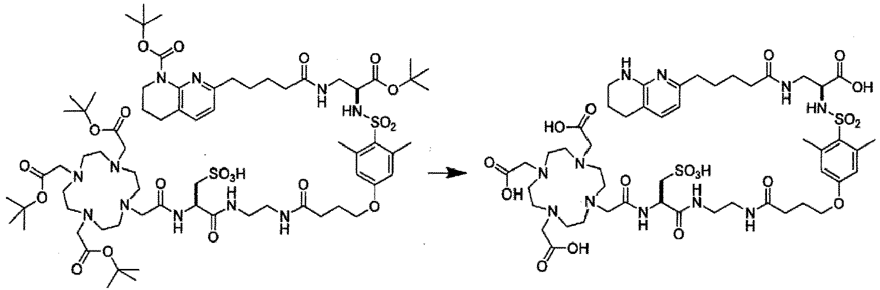

TFA/triethylsilane (1/1) (2 mL) were added to a chloroform (1 mL) suspension of (R)-3-((2-(4-(4-(N-((S)-1-(tert-butoxy)-3-(5-(8-(tert-butoxycarbonyl)-5,6,7,8-tetrahydro-1,8-n aphthyridin-2-yl)pentanamide)-1-oxopropan-2-yl)sulfamoyl)-3,5-dimethylphenoxy)butanamid e)ethyl)amino)-3-oxo-2-(2-(4,7,10-tris(2-(tert-butoxy)-2-oxoethyl)-1,4,7,10-tetraazacyclodode can-1-yl)acetamide)propane-1-sulfonic acid (250 mg), followed by stirring for 4 hours at room temperature. The solvent was distilled away under reduced pressure, and an operation of adding acetonitrile (1 mL) to the obtained residue and distilling away the solvent under reduced pressure was repeated twice. The obtained residue was purified using a reversed-phase silica gel column (Sep-Pak C18, Waters), water/methanol = 100/0 to 60/40), thereby obtaining the compound A (109.9 mg) as a white solid.

LC/MS rt (min): 0.75

MS (ESI, m/z): 1170.4 [M + H]⁺

¹H-NMR (D₂O) δ: 7.49 (1H, d, J = 7.3 Hz), 6.71 (2H, s), 6.50 (1H, d, J = 7.3 Hz), 4.67 (1H, dd, J = 7.9, 5.0 Hz), 4.00 (2H, t, J = 5.9 Hz), 3.93-3.02 (34H, m), 2.72 (2H, t, J = 6.1 Hz), 2.59 (2H, t, J = 7.3 Hz), 2.34 (2H, t, J = 7.3 Hz), 2.12-1.80 (6H, m), 1.62-1.38 (4H, m)

### Example 3

(1)

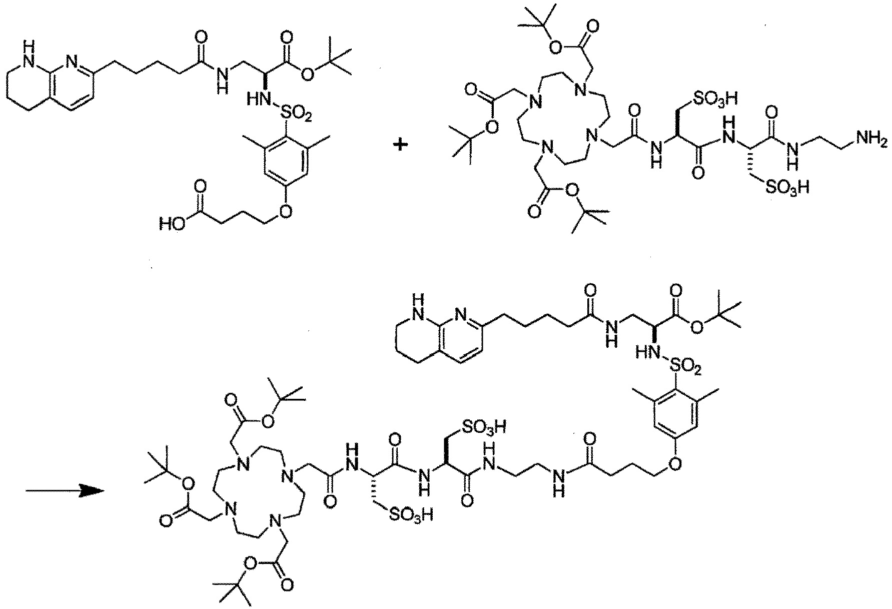

In an ice bath, HBTU (82 mg) was added to a mixture of (S)-4-(4-(N-(1-(tert-butoxy)-1-oxo-3-(5-(5,6,7,8-tetrahydro-1,8-naphthyridin-2-yl)-pentanami de)propan-2-yl)sulfamoyl)-3,5-dimethylphenoxy)butanoic acid (140 mg), (R)-3-((2-aminoethyl)amino)-3-oxo-2-((R)-3-sulfo-2-(2-(4,7,10-tris(2-(tert-butoxy)-2-oxoethy l)-1,4,7,10-tetraazacyclododecan-1-yl)acetamide)propanamide)propane-1-sulfonic acid (189 mg), DIEA (0.076 mL), and DMF (3.0 mL), followed by stirring for 1 hour at room temperature. Water (0.1 mL) was added to the reaction mixture, and the solvent was distilled away under reduced pressure. By adding acetonitrile (4 mL) and an aqueous saturated sodium chloride solution (1.5 mL) to the residue, the organic layer was fractionated, and the aqueous layer was extracted twice by using acetonitrile (3 mL). The solvent of the organic layer was distilled away under reduced pressure, and the residue was purified by silica gel column chromatography (diol silica gel (Purif-Pack DIOL 60·m, Shoko Scientific Co., Ltd.), hexane/ethyl acetate = 50/50 to 0/100, chloroform/ethanol = 100/0 to 80/20), thereby obtaining (R)-3-((2-(4-(4-(N-((S)-1-(tert-butoxy)-1-oxo-3-(5-(5,6,7,8-tetrahydro-1,8-naphthyridin-2-yl)p entanamide)propan-2-yl)sulfamoyl)-3,5-dimethylphenoxy)butananmide)ethyl)amino)-3-oxo-2 -((R)-3-sulfo-2-(2-(4,7,10-tris(2-(tert-butoxy)-2-oxoethyl)-1,4,7,10-tetraazacyclododecan-1-yl) acetamide)propanamide)propane-1-sulfonic acid (296 mg) as a white amorphous solid.

MS (ESI, m/z): 1545 [M + H]⁺

¹H-NMR (D₂O) δ: 7.35 (1H, d, J = 7.3 Hz), 6.80 (2H, s), 6.52 (1H, d, J = 7.3 Hz), 4.63-4.58 (1H, m), 4.10-4.02 (2H, m), 3.92-3.83 (1H, m), 3.75-2.95 (33H, m), 2.70 (2H, t, J = 6.1 Hz), 2.55 (6H, s), 2.36 (2H, t, J = 7.3 Hz), 2.17-2.08 (2H, m), 2.07-1.98 (2H, m), 1.90 (9H, s), 1.88-1.81 (2H, m), 1.58-1.12 (37H, m)

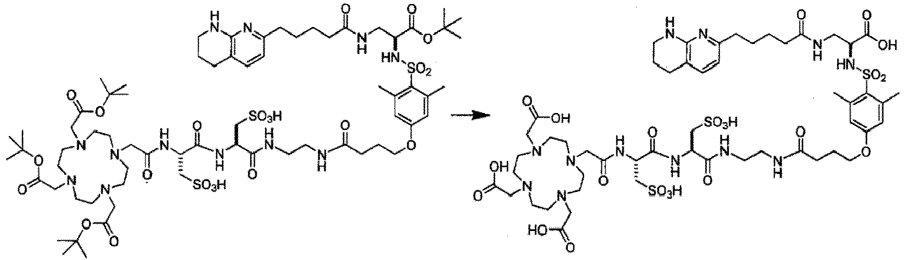

6 mol/L hydrochloric acid (1 mL) cooled to 0°C was added to (R)-3-((2-(4-(4-(N-((S)-1-(tert-butoxy)-1-oxo-3-(5-(5,6,7,8-tetrahydro-1,8-naphthyridin-2-yl)p entanamide)propan-2-yl)sulfamoyl)-3,5-dimethylphenoxy)butananmide)ethyl)amino)-3-oxo-2 -((R)-3-sulfo-2-(2-(4,7,10-tris(2-(tert-butoxy)-2-oxoethyl)-1,4,7,10-tetraazacyclododecan-1-yl) acetamide)propanamide)propane-1-sulfonic acid (262 mg), followed by stirring for 12.5 hours at room temperature. The mixture was cooled in an ice bath, and then a 5 mol/L aqueous sodium hydroxide solution (1 mL) was added thereto for 10 minutes in a state where the internal temperature was being kept to be equal to or lower than 13°C. Then, sodium acetate trihydrate (172 mg) was added thereto. A reversed-phase silica gel column (Sep-Pak C18, Waters) was charged with the obtained reaction mixture, and elution was performed under a normal pressure by using 0.1% formic acid-containing water (12 mL), 0.1% formic acid·5% acetonitrile-containing water (6 mL), 0.1% formic acid·10% acetonitrile-containing water (6 mL), 0.1% formic acid·15% acetonitrile-containing water (6 mL), 0.1% formic acid·20% acetonitrile-containing water (6 mL), 0.1% formic acid·25% acetonitrile-containing water (6 mL), 0.1% formic acid·30% acetonitrile-containing water (6 mL), 0.1% formic acid·35% acetonitrile-containing water (6 mL), and 0.1% formic acid·40% acetonitrile-containing water (6 mL) in this order, and the solvent was distilled away under reduced pressure, thereby obtaining 2,2',2"-(10-((4R,7R)-16-(4-(N-((S)-1-carboxy-2-(5-(5,6,7,8-tetrahydro-1,8-naphthyridin-2-yl) pentanamide)ethyl)sulfamoyl)-3,5-dimethylphenoxy)-2,5,8,13-tetraoxo-4,7-bis(sulfomethyl)-3 ,6,9,12-tetraazahexadecyl)-1,4,7,10-tetraacacyclododecane-1,4,7-triyl)triacetic acid (159 mg) as a colorless solid.

MS (ESI, m/z): 1321 [M + H]⁺

¹H-NMR (D₂O) δ: 7.52 (1H, d, J = 7.3 Hz), 6.76 (2H, s), 6.53 (1H, d, J = 7.3 Hz), 4.68-4.61 (1H, m), 4.04 (2H, t, J = 6.1 Hz), 3.99-3.10 (38H, m), 2.74 (2H, t, J = 6.1 Hz), 2.61 (2H, t, J = 7.4 Hz), 2.54 (6H, s), 2.33 (2H, t, J = 7.3 Hz), 1.95 (6H, ddt, J= 42.9, 19.8, 6.6 Hz), 1.60-1.35 (4H, m)

### Example 4

(1)

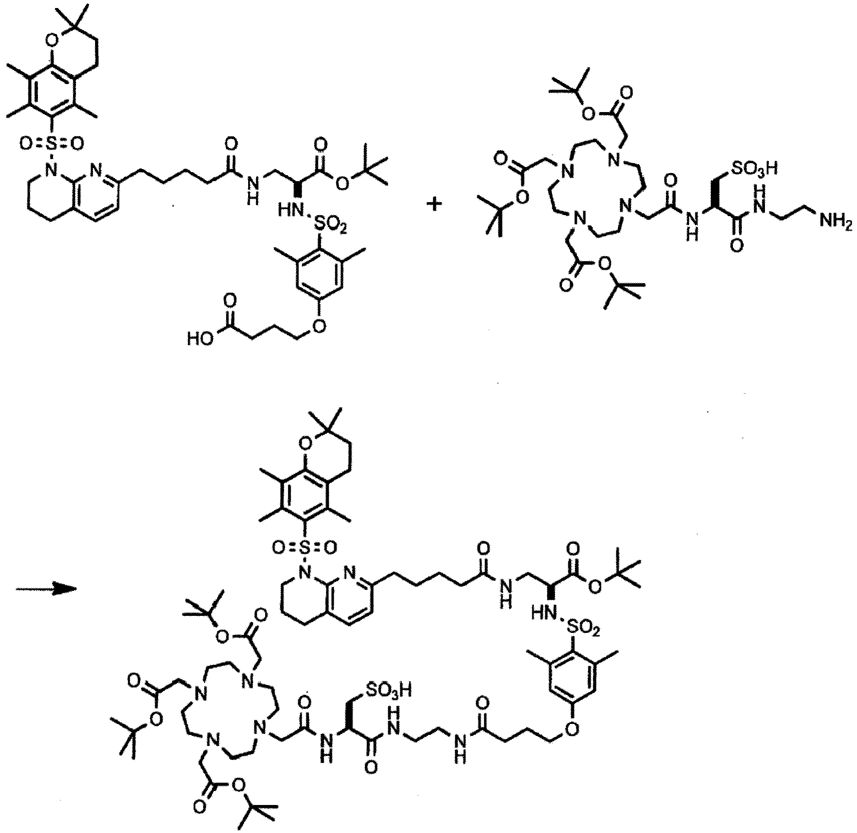

(R)-3-((2-aminoethyl)amino)-3-oxo-2-(2-(4,7,10-tris(2-(tert-butoxy)-2-oxoethyl)-l,4,7, 10-tetraazacyclododecan-1-yl)acetamide)propane-1-sulfonic acid (143 mg) and diisopropylethylamine (66.3 µL) were added to a reaction mixture containing (S)-4-(4-(N-(1-(tert-butoxy)-1-oxo-3-(5-(8-((2,2,5,7,8-pentamethylchroman-6-yl)sulfonyl)-5,6 ,7,8-tetrahydro-l,8-naphthyridin-2-yl)pentanamide)propan-2-yl)sulfamoyl)-3,5-dimethylpheno xy)butanoic acid obtained in (3) of Reference Example 3, followed by stirring for 20 minutes at room temperature. Then, HBTU (70.9 mg) was added thereto, followed by stirring for 21 hours at room temperature. Thereafter, water was added to the reaction solution, followed by stirring for 3 hours. The aqueous layer was removed by a decantation operation, and then water was added thereto so as to make a suspension with stirring, and the solid was collected by filtration. The solid was purified by silica gel column chromatography (chloroform/methanol), thereby obtaining (R)-3-((2-(4-(4-(N-((S)-1-(tert-butoxy)-1-oxo-3-(5-(8-((2,2,5,7,8-pentamethylchroman-6-yl)su lfonyl)-5,6,7,8-tetrahydro-1,8-naphthyridin-2-yl)pentanamide)propan-2-yl)sulfamoyl)-3,5-dim ethylphenoxyl)butanamide)ethyl)amino)-3-oxo-2-(2-(4,7,10-tris(2-(tert-butoxy)-2-oxoethyl)-1, 4,7,10-tetraazacyclododecan-1-yl)acetamide)propane-1-sulfonic acid (83.0 mg) as a white solid.

LC/MS rt (min): 1.75

MS (ESI, m/z): 1662.1 [M + H]⁺

¹H-NMR (CDCl₃) δ: 8.60-8.48 (m, 1H), 7.72-7.54 (m, 1H), 7.18 (d, 1H, J = 7.6 Hz), 6.66 (s, 2H), 6.56 (d, 1H, J = 7.6 Hz), 6.18-6.07 (m, 1H), 5.79-5.69 (m, 1H), 4.81-4.69 (m, 1H), 4.12-2.47 (m, 45H), 2.42-2.28 (m, 4H), 2.14-1.12 (m, 67H)

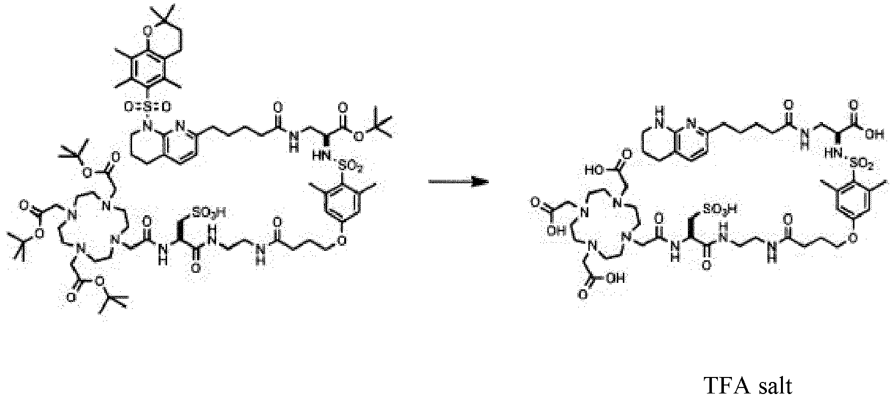

A mixed solution of trifluoroacetic acid (0.4 mL) and triethylsilane (19.2 µL) was added to (R)-3-((2-(4-(4-(N-((S)-1-(tert-butoxy)-1-oxo-3-(5-(8-((2,2,5,7,8-pentamethylchroman-6-yl)su lfonyl)-5,6,7,8-tetrahydro-1,8-naphthyridin-2-yl)pentanamide)propan-2-yl)sulfamoyl)-3,5-dim ethylphenoxyl)butanamide)ethyl)amino)-3-oxo-2-(2-(4,7,10-tris(2-(tert-butoxy)-2-oxoethyl)-1, 4,7,10-tetraazacyclododecan-1-yl)acetamide)propane-1-sulfonic acid (20 mg), followed by stirring for 24 hours at room temperature. Then the solvent was distilled away under reduced pressure. TBME (5 mL) was added to the obtained residue, the solid was collected by filtration, thereby obtaining TFA (21.4 mg) of the compound A was obtained as a white solid.

LC/MS rt (min): 0.70

MS (ESI, m/z): 1170.9 [M + H]⁺, 1168.9 [M - H]⁻

### Example 5

(1)

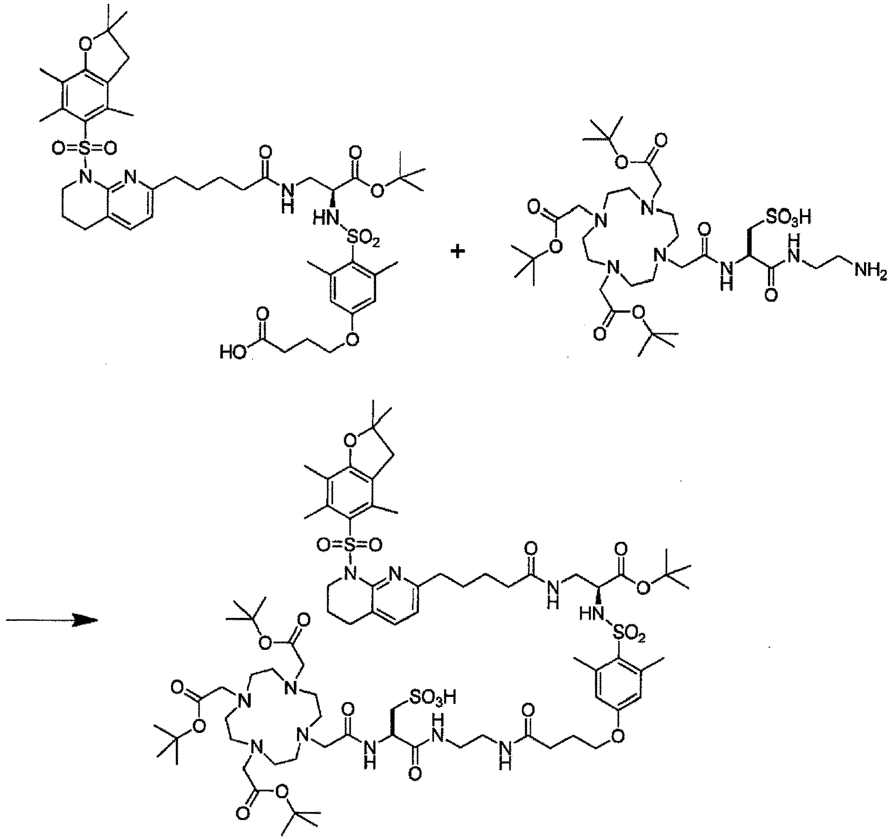

By the same method as in (1) of Example 4, a reaction mixture containing (S)-4-(4-(N-(1-(tert-butoxy)-1-oxo-3-(5-(8-((2,2,4,6,7-pentamethyl-2,3-dihydrobenzofuran-5-y l)sulfonyl)-5,6,7,8-tetrahydro-1,8-naphthyridin-2-yl)pentanamide)propan-2-yl)sulfamoyl)-3,5-dimethylphenoxy)butanoic acid obtained in (3) of Reference Example 4 was reacted with (R)-3-((2-aminoethyl)amino)-3-oxo-2-(2-(4,7,10-tris(2-(tert-butoxy)-2-oxoethyl)-1,4,7,10-tetr aazacyclododecan-1-yl)actamide)propane-1-sulfonic acid (21.3 mg), thereby obtaining (R)-3-((2-(4-(4-(N-((S)-1-(tert-butoxy)-1-oxo-3-(5-(8-((2,2,4,6,7-pentamethyl-2,3-dihydroben zofuran-5-yl)sulfonyl)-5,6,7,8-tetrahydro-1,8-naphthyridin-2-yl)pentanamide)propan-2-yl)sulf amoyl)-3,5-dimethylphenoxy)butanamide)ethyl)amino)-3-oxo-2-(2-(4,7,10-tris(2-(tert-butoxy) -2-oxoethyl)-1,4,7,10-tetraazacyclododecan-1-yl)acetamide)propane-1-sulfonic acid as a white solid (56.2 mg).

LC/MS rt (min): 1.68

MS (ESI, m/z): 1648.1 [M + H]⁺

¹H-NMR (CDCl₃) δ: 8.59-8.46 (m, 1H), 7.74-7.64 (m, 1H), 7.59-7.46 (m, 1H), 7.19 (d, 1H, J = 7.6 Hz), 6.66 (s, 2H), 6.58 (d, 1H, J = 7.6 Hz), 6.17-6.06 (m, 1H), 5.77-5.67 (m, 1H), 4.81-4.70 (m, 1H), 4.12-2.79 (m, 30H), 2.74 (t, 2H, J = 6.3 Hz), 2.61 (s, 6H), 2.56 (s, 3H), 2.50 (s, 3H), 2.44-2.30 (m, 4H), 2.14-1.19 (m, 65H)

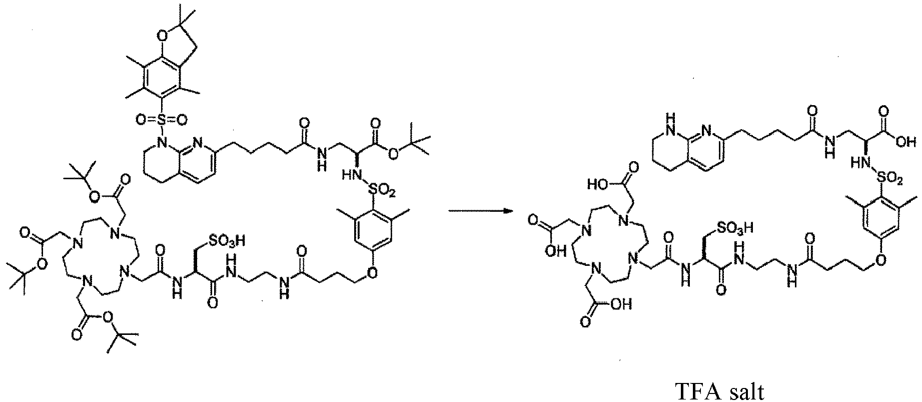

A mixed solution of trifluoroacetic acid (0.4 mL) and triethylsilane (19.2 µL) was added to (R)-3-((2-(4-(4-(N-((S)-1-(tert-butoxy)-1-oxo-3-(5-(8-((2,2,4,6,7-pentamethyl-2,3-dihydroben zofuran-5-yl)sulfonyl)-5,6,7,8-tetrahydro-1,8-naphthyridin-2-yl)pentanamide)propan-2-yl)sulf amoyl)-3,5-dimethylphenoxy)butanamide)ethyl)amino)-3-oxo-2-(2-(4,7,10-tris(2-(tert-butoxy) -2-oxoethyl)-1,4,7,10-tetraazacyclododecan-1-yl)acetamide)propane-1-sulfonic acid (10 mg), followed by stirring for 24 hours at room temperature. Then, the solvent was distilled away under reduced pressure. TBME (5 mL) was added to the obtained residue, the solid was collected by filtration, thereby obtaining TFA (8.2 mg) of the compound A as a white solid.

LC/MS rt (min): 0.70

MS (ESI, m/z): 1170.9 [M + H]⁺, 1168.9 [M - H]⁻

### Example 6

(1)

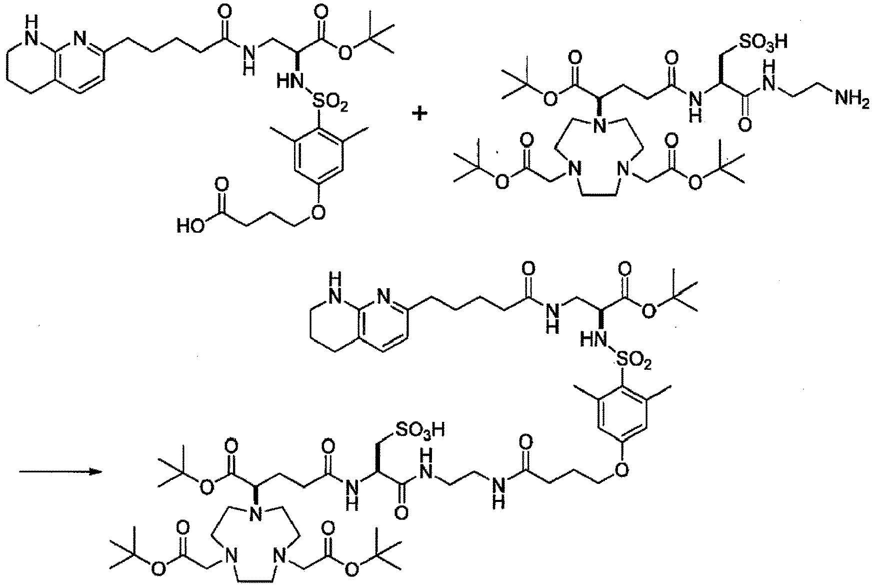

HBTU (18.3 g) was added to a mixture of (R)-3-((2-aminoethyl)amino)-2-((R)-4-(4,7-bis(2-(tert-butoxy)-2-oxoethyl)-1,4,7-triazonan-1-yl)-5-(tert-butoxy)-5-oxopentanamide)-3-oxopropane-1-sulfonic acid (29.5 g), DMAc (150 mL), DIEA (17.4 mL), and (S)-4-(4-(N-(1-(tert-butoxy)-1-oxo-3-(5-(5,6,7,8-tetrahydro-1,8-naphthyridin-2-yl)pentanamid e)propan-2-yl)sulfamoyl)-3,5-dimethylphenoxy)butanoic acid (23.3 g), followed by stirring for 1.5 hours at room temperature. The reaction solution was added dropwise to an aqueous saturated ammonium chloride solution (600 mL) cooled to 6°C, followed by stirring for 10 minutes. The supernatant was removed, and then water (600 mL) was added to the residue, followed by stirring for 10 minutes. Thereafter, the supernatant was removed again, the obtained viscous solid was dissolved in ethanol/chloroform (20/1) (100 mL) and then concentrated under reduced pressure. Water was removed by repeating twice an operation of adding ethanol (100 mL) to the residue and concentrating the solvent under reduced pressure, and then the residue was purified by silica gel column chromatography (NH silica (NH-Sil, Biotage), chloroform/methanol = 100/0 to 70/30 to 20/80), thereby obtaining (R)-2-((R)-4-(4,7-bis(2-(tert-butoxy)-2-oxoethyl)-1,4,7-triazonan-1-yl)-5-(tert-butoxy)-5-oxop entanamide)-3-((2-(4-(4-(N-((S)-1-(tert-butoxy)-1-oxo-3-(5-(5,6,7,8-tetrahydro-1,8-naphthyrid in-2-yl)pentanamide)propan-2-yl)sulfamoyl)-3,5-dimethylphenoxy)butanamide)ethyl)amino)-3-oxopropane-1-sulfonic acid (27.7 g) as a white solid.

LC/MS rt (min): 1.40

MS (ESI, m/z): 1365 [M + H]⁺

¹H-NMR (CDCl₃, 300 MHz) δ: 7.61 (1H, brs), 7.33 (1H, brs), 7.23 (1H, brs), 6.65 (2H, s), 6.52 (1H, brs), 6.36 (1H, d, J = 7.3 Hz), 4.71 (1H, m), 3.98 (2H, t, J = 6.3 Hz), 3.87 (1H, brs), 3.60-1.53 (57H, m), 1.49-1.42 (27H, m), 1.34 (9H, s)

HPLC (Waters BEH C18, formic acid-based, gradient cycle: 0 min (A solution/B solution = 30/70), 10 min (A solution/B solution = 0/100), 12 min (A solution/B solution = 0/100), flow rate: 0.4 mL/min)) rt (min): 4.81

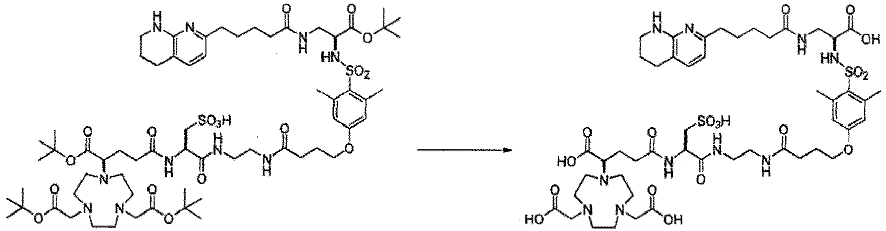

6 mol/L hydrochloric acid (300 mL) was added to (R)-2-((R)-4-(4,7-bis(2-(tert-butoxy)-2-oxoethyl)-1,4,7-triazonan-1-yl)-5-(tert-butoxy)-5-oxop entanamide)-3-((2-(4-(4-(N-((S)-1-(tert-butoxy)-1-oxo-3-(5-(5,6,7,8-tetrahydro-1,8-naphthyrid in-2-yl)pentanamide)propan-2-yl)sulfamoyl)-3,5-dimethylphenoxy)butanamide)ethyl)amino)-3-oxopropane-1-sulfonic acid (15.1 g), followed by stirring for 14 hours at room temperature. The mixture was cooled in an ice bath, and then a 5 mol/L aqueous sodium hydroxide solution (300 mL) was added thereto for 1 hour and 20 minutes in a state where the internal temperature was being conrolled to become equal to or lower than 13°C. Thereafter, anhydrous sodium acetate (49.5 g) was added thereto, and the pH of the reaction solution was adjusted to be 4.07. A reversed-phase silica gel column (inner diameter of glass column: 10.5 cm, Daisogel-SR120-40/60-ODS-RPS: 315 g) was charged with the obtained reaction mixture, and elution was performed under a normal pressure by using water (600 mL), 10% acetonitrile-containing water (600 mL), and 30% acetonitrile-containing water (1,800 mL) in this order. A fraction containing 2,2'-(7-((R)-1-carboxy-4-(((R)-1-((2-(4-(4-(N-((S)-1-carboxy-2-(5-(5,6,7,8-tetrahydro-1,8-nap hthyridin-2-yl)pentanamide)ethyl)sulfamoyl)-3,5-dimethylphenoxy)butanamide)ethyl)amino)-1-oxo-3-sulfopropan-2-yl)amino)-4-oxobutyl)-1,4,7,-triazonane-1,4-diyl)diacetic acid was combined, and the solvent was distilled away under reduced pressure. Water (100 mL) was added to the obtained residue, and while the solution was being stirred with ice cooling, lithium carbonate (1.38 g) was added thereto in four divided portions such that the pH of the reaction solution was adjusted to be 8.10. Thereafter, a reversed-phase silica gel column (inner diameter of glass column: 10.5 cm, Daisogel-SR120-40/60-ODS-RPS: 315 g) was charged with the reaction solution, elution was performed under a normal pressure by using water (600 mL), 5% acetonitrile-containing water (600 mL), 10% acetonitrile-containing water (600 mL), 15% acetonitrile-containing water (600 mL), 20% acetonitrile-containing water (600 mL), and 25% acetonitrile-containing water (600 mL) in this order, and a fraction containing a lithium salt of 2,2'-(7-((R)-1-carboxy-4-(((R)-1-((2-(4-(4-(N-((S)-1-carboxy-2-(5-(5,6,7,8-tetrahydro-1,8-nap hthyridin-2-yl)pentanamide)ethyl)sulfamoyl)-3,5-dimethylphenoxy)butanamide)ethyl)amino)-1-oxo-3 -sulfopropan-2-yl)amino)-4-oxobutyl)-1,4,7-triazonane-1,4-diyl)diacetic acid was concentrated under reduced pressure. Water (100 mL) was added to the solution, and formic acid (2.79 mL) was added thereto with stirring in an ice bath. A reversed-phase silica gel column (inner diameter of glass column: 6.5 cm, Daisogel-SR120-40/60-ODS-RPS: 150 g) was charged with the obtained mixture, and elution was performed under a normal pressure by using a 0.1% aqueous formic acid solution (300 mL), water (300 mL), 30% acetonitrile-containing water (300 mL), 40% acetonitrile-containing water (300 mL), and 50% acetonitrile-containing water (300 mL) in this order. A fraction containing 2,2'-(7-((R)-1-carboxy-4-(((R)-1-((2-(4-(4-(N-((S)-1-carboxy-2-(5-(5,6,7,8-tetrahydro-1,8-nap hthyridin-2-yl)pentanamide)ethyl)sulfamoyl)-3,5-dimethylphenoxy)butanamide)ethyl)amino)-1-oxo-3 -sulfopropan-2-yl)amino)-4-oxobutyl)-1,4,7-triazonane-1,4-diyl)diacetic acid was collected, and the solvent was distilled away under reduced pressure. Water (150 mL) was added to the obtained residue, and then the solution was freeze-dried, thereby obtaining 2,2'-(7-((R)-1-carboxy-4-(((R)-1-((2-(4-(4-(N-((S)-carboxy-2-(5-(5,6,7,8-tetrahydro-1,8-napht hyridin-2-yl)pentanamide)ethyl)sulfamoyl)-3,5-dimethylphenoxy)butanamide)ethyl)amino)-1-oxo-3-sulfopropan-2-yl)amino)-4-oxobutyl)-1,4,7-triazonane-1,4-diyl)diacetic acid (8.93 g) as a white solid.

LC/MS rt (min): 0.76

MS (ESI, m/z): 1141 [M + H]^{+ 1}H-NMR (D₂O, 300 MHz) δ: 7.51 (1H, d, J = 7.5 Hz), 6.75 (2H, s), 6.53 (1H, d, J = 7.5 Hz), 4.65 (1H, dd, J = 7.9, 5.0 Hz), 4.03 (2H, t, J = 5.9 Hz), 3.91 (1H, dd, J = 9.2, 4.3 Hz), 3.74 (4H, s), 3.60-2.88 (24H, m), 2.73 (2H, t, J = 5.9 Hz), 2.61 (2H, t, J = 7.3 Hz), 2.53 (6H, s), 2.50-2.41 (1H, m), 2.32 (2H, t, J = 7.4 Hz), 2.13-1.80 (8H, m), 1.62-1.37 (4H, m)

HPLC (GL Inertsustain C18, TFA-based, gradient cycle: 0 min (A solution/B solution = 90/10), 20 min (A solution/B solution =75/25), 30 min (A solution/B solution = 75/25), flow rate: 1.0 mL/min) rt (min): 9.80

### Example 7

(1)

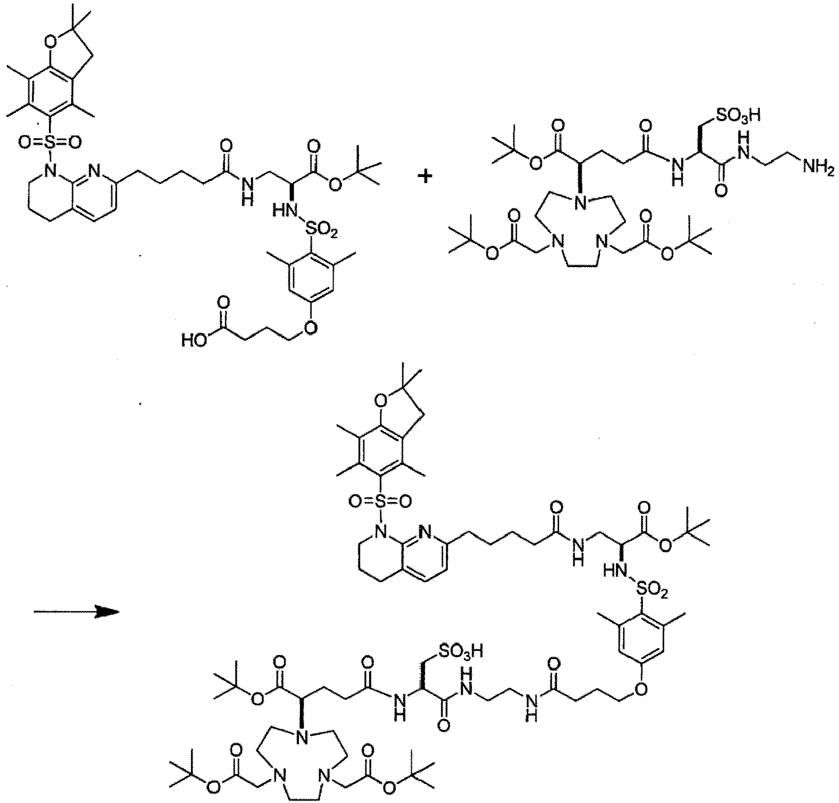

DIEA (67 µL), (R)-3-((2-aminoethyl)emino)-2-((R)-4-(4,7-bis(2-(tert-butoxy)-2-oxoethyl)-1,4,7-triazonan-1-yl)-5-(tert-butoxy)-5-oxopentanamide)-3-oxopropane-1-sulfonic acid (140 mg), and HBTU (72 mg) were added to a reaction mixture containing (S)-4-(4-(N-(1-(tert-butoxy)-1-oxo-3-(5-(8-((2,2,4,6,7-pentamethyl-2,3-dihydrobenzofuran-5-y l)sulfonyl)-5,6,7,8-tetrahydro-l,8-naphthyridin-2-yl)pentanamide)propan-2-yl)sulfamoyl)-3,5-dimethylphenoxy)butanoic acid obtained in (3) of Reference Example 4, followed by stirring for 2.5 hours at room temperature. An aqueous saturated ammonium chloride solution (4 mL) was added to the reaction mixture, the supernatant was then removed, and the obtained viscous solid was purified by silica gel column chromatography (NH silica, chloroform/methanol = 100/0 to 80/20 to 70/30 to 50/50), thereby obtaining (R)-2-((R)-4-(4,7-bis(2-(tert-butoxy)-2-oxoethyl)-1,4,7-triazonan-1-yl)-5-(tert-butoxy)-5-oxop entanamide)-3-((2-(4-(4-(N-((S)-1-(tert-butoxy)-1-oxo-3-(5-(8-((2,2,4,6,7-pentamethyl-2,3-dih ydroxybenzofuran-5-yl)sulfonyl)-5,6,7,8-tetrahydro-1,8-naphthyridin-2-yl)pentanamide)propa n-2-yl)sulfamoyl)-3,5-dimethylphenoxy)butanamide)ethyl)amino)-3-oxopropane-l-sulfonic acid (25 mg) as a white solid.

LC/MS rt (min): 1.90

MS (ESI, m/z): 1618 [M + H]⁺

¹H-NMR (CDCl₃, 300 MHz) δ: 7.95-7.31 (2H, m), 7.18 (1H, d, J = 7.3 Hz), 6.65 (2H, s), 6.58 (1H, d, J = 7.3 Hz), 6.11 (1H, s), 5.72 (1H, brs), 4.80 (1H, brs), 4.13-3.95 (4H, m), 3.75-2.24 (49H, m), 2.19-1.75 (12H, m), 1.54-1.16 (46H, m)

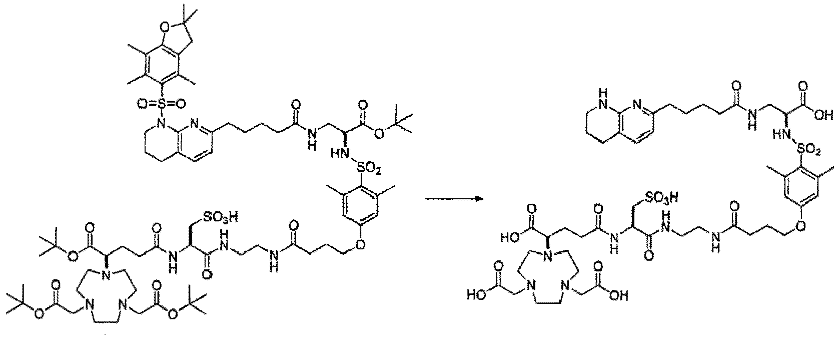

TFA (0.5 mL) was added to (R)-2-((R)-4-(4,7-bis(2-(tert-butoxy)-2-oxoethyl)-1,4,7-triazonan-1-yl)-5-(tert-butoxy)-5-oxop entanamide)-3-((2-(4-(4-(N-((S)-1-(tert-butoxy)-1-oxo-3-(5-(8-((2,2,4,6,7-pentamethyl-2,3-dih ydroxybenzofuran-5-yl)sulfonyl)-5,6,7,8-tetrahydro-1,8-naphthyridin-2-yl)pentanamide)propa n-2-yl)sulfamoyl)-3,5-dimethylphenoxy)butanamide)ethyl)amino)-3-oxopropane-1-sulfonic acid (5 mg), followed by stirring for 6 hours at room temperature, and the solvent was distilled away. The residue was purified by preparative HPLC, thereby obtaining 2,2'-(7-((R) -1-carboxy-4-(((R)-1-((2-(4-(4-(N-((S)-1-carboxy-2-(5-(5,6,7,8-tetrahydro-1,8-naphthyridin-2-yl)pentanamide)ethyl)sulfamoyl)-3,5-dimethylphenoxy)butanamide)ethyl)amino)-1-oxy-3-sulf opropan-2-yl)amino)-4-oxobutyl)-1,4,7-triazonane-1,4-diyl)diacetic acid (2 mg) as a white solid.

LC/MS rt (min): 0.75

MS (ESI, m/z): 1141 [M + H]⁺

### Example 8

In the present example, the compound A obtained in Example 1 and the compound B obtained in Example 6 were used.
(A) A gallium [⁶⁷Ga] chloride solution (200 MBq, 63 µL) was added to a mixed solution of the compound A (21 µg), gentisic acid (1.0 mg), a 0.2 mol/L sodium acetate buffer solution (pH 4.0, 730.7 ¡.tL), and 4.5 mol/L aqueous sodium hydroxide solution (6.3 µL). The solution was heated to 100°C for 15 minutes and then left to stand for 5 minutes at room temperature, thereby obtaining [⁶⁷Ga]-(compound A). As a result of analyzing the compound by using reversed-phase TLC (Merck, RP-8 F_{254S}, developing solvent: methanol/0.5 mol/ L aqueous ammonium acetate solution/28% aqueous ammonia (50/50/1), the Rf value of the radiolabeled compound was found to be 0.4. The radiochemical purity measured immediately after the compound was prepared and measured after 3.5 hours at room temperature was equal to or higher than 95%.
(B) A lutetium [¹⁷⁷Lu] chloride solution (666 MBq, 333 µL) dissolved in a 0.2 mol/L sodium acetate buffer solution (pH 4.0) was added to a mixed solution of the compound A (70.0 µg), gentisic acid (1.8 mg), and a 0.2 mol/L sodium acetate buffer solution (pH 4.0, 83.3 µL). The solution was heated to 100°C for 15 minutes and then left to stand for 5 minutes at room temperature, thereby obtaining [¹⁷⁷Lu]-(compound A). As a result of analyzing the compound by using reversed-phase TLC (Merck, RP-8 F_{254S}, developing solvent: methanol/0.5 mol/ L aqueous ammonium acetate solution/28% aqueous ammonia (50/50/1), the Rf value of the radiolabeled compound was found to be 0.4. The radiochemical purity measured immediately after the compound was prepared and measured after 3 hours at room temperature was equal to or higher than 95%.
(C) A gallium [⁶⁷Ga] chloride solution (40 MBq, 11.7 µL) was added to a mixed solution of the compound B (4.1 µg), gentisic acid (1.0 mg), a 0.2 mol/L sodium acetate buffer solution (pH 4.5, 147.13 ¡.tL), and 4.5 mol/L aqueous sodium hydroxide solution (1.17 µL). The solution was heated to 100°C for 15 minutes and then left to stand for 5 minutes at room temperature, thereby obtaining [⁶⁷Ga]-(compound B). As a result of analyzing the compound by using reversed-phase TLC (Merck, RP-8 F_{254S}, developing solvent: methanol/0.5 mol/ L aqueous ammonium acetate solution/28% aqueous ammonia (50/50/1), the Rf value of the radiolabeled compound was found to be 0.5. The radiochemical purity measured immediately after the compound was prepared and measured after 5.5 hours at room temperature was equal to or higher than 95%.

In the following Test Examples 1 to 9, the compound A obtained in Reference Example 8 and the compound B obtained in Reference Example 9 were used.

### Test Example 1 integrin δᵥβ₃ binding affinity test

0.2 µg/mL of δᵥβ₃ was immobilized in a 96-well plates (Corning Incorporated) and then blocked using a 1% Block Ace (DS Pharma Biomedical Co., Ltd.) solution, and then the plate was washed with T-PBS (PBS containing 0.05% Tween 20). A 2X concentrated evaluation compound solution (10X concentration of 3.16X dilution from 0.3 µmol/L, buffer (20 mM Tris-HCl pH 7.5, 150 mM NaCl, 1 mM CaCl₂, 1 mM MgCl₂, 1 mM MnCl₂)) and a 4 µg/mL biotinylated vitronectin solution (labeling vitronectin (Upstate Biotechnology Inc.) by using EZ-Link Sulfo-NHS-Biotinylation Kit (Pierce Protein Biology) and then adjusting concentration) were each added to the plate in an amount of 50 µL, and the plate was shaken for 2 hours at room temperature. The plate was washed with T-PBS, a 0.2 µg/mL avidin·peroxidase (Pierce Protein Biology) was added thereto, and the plate was shaken for 1 hour at room temperature. The plate was washed with T-PBS, an o-phenylenediamine (Sigma-Aldrich Co., LLC.) solution was added thereto such that color was produced (stopped using 4 mol/L sulfuric acid), and the absorbance (490 nm, Reference: 595 nm) was measured. The IC₅₀ value was calcluated using XLfit 3.0 (ID Business Solutions Ltd.). For each plate, as a QC sample, RGDfV (Bachem AG) was measured in duplicate.

### Test Example 2 integrin δᵥβ₅ binding affinity test

0.2 µg/mL of δᵥβ₅ was immobilized in a 96-well plates (Corning Incorporated) and then blocked using a 1% Block Ace (DS Pharma Biomedical Co., Ltd.) solution, and then the plate was washed with PBST (10 mM Na₂HPO₄ pH 7.5, 150 mM NaCl, 0.01% Tween 20).

A 2X concentrated evaluation compound solution (10X concentration of 3.16X dilution from 0.3 µmol/L, buffer (20 mM Tris-HCl pH 7.5, 150 mM NaCl, 1 mM CaCl₂, 1 mM MgCl₂, 1 mM MnCl₂)) and a 4 µg/mL biotinylated vitronectin solution (labeling vitronectin (Upstate Biotechnology Inc.) by using EZ-Link Sulfo-NHS-Biotinylation Kit (Pierce Protein Biology) and then adjusting concentration) were each added to the plate in an amount of 50 µL, and the plate was shaken for 2 hours at room temperature. The plate was washed with PBST, a 0.2 µg/mL avidin·peroxidase (Pierce Protein Biology) was added thereto, and the plate was shaken for 1 hour at room temperature. The plate was washed with PBST, an o-phenylenediamine (Sigma-Aldrich Co., LLC.) solution was added thereto such that color was produced (stopped using 4 mol/L sulfuric acid), and the absorbance (490 nm, Reference: 595 nm) was measured. The IC₅₀ value was calcluated using XLfit 3.0 (ID Business Solutions Ltd.). For each plate, as a QC sample, RGDfV (Bachem AG) was measured in duplicate.

As the evaluation compounds of Test Examples 1 and 2, the compound A and the compound B were used. The results are shown below.

**[Table 1]**

| IC₅₀ value | Evaluation |
|---|---|
| Less than 1 nmol/L | +++ |
| 1~10 nmol/L | ++ |
| 10~100 nmol/L | + |

**[Table 2]**

| Compound | α_{V}β₃ | α_{V}β₅ |
|---|---|---|
| Compound A | +++ | +++ |
| Compound B | +++ | +++ |

The compounds in Table 2 exhibited excellent integrin binding affinity.

### Test Example 3 Evaluation based on radioactivity concentration of ¹¹¹In-labeled compound, ⁶⁴Cu-labeled compound, and ⁹⁰Y-labeled compound in tumor

1 × 10⁷ U87MG cells were transplanted into the subcutaneous space of the right flank of Balb/c AJcl-nu/nu (6 to 9-week-old, KURARAY CO., LTD. or Japan SLC. Inc). After 2 to 3 weeks, at a point in time when the tumor volume became 200 to 500 mm³, 3 mice were sorted into one group at each point in time. The ¹¹¹In-labeled compound (740 k Bq) was administered into the caudal vein, the animals were sacrificed after a certain period of time, and the tumor was extracted. The weight of the tumor was measured, the radioactivity was measured using a gamma counter, and the radioactivity concentration in tumor (% ID/g) was calculated. For the ⁶⁴Cu-labeled compound (500 k Bq) and the ⁹⁰Y-labeled compound (500 k Bq), the radioactivity concentration in tumor (% ID/g) was calculated by the same method.

The results are shown below.

**[Table 3]**

| Radiolabeled compound | Radioactivity concentration in tumor (% ID/g) | |
|---|---|---|
| | After 4 hours | After 24 hours |
| [^{m}In]-(compound A) | 11.10 | 9.62 |
| [90^{Y}]-(compound A) | 12.52 | 15.29 |
| [⁶⁴Cu]-(compound A) | 9.25 | 8.48 |
| [⁶⁴Cu]-(compound B) | 11.19 | 8.53 |

The radioactivity concentration of the compounds in Table 3 reached 9.25 to 12.52% ID/g in the tumor within 4 hours after administration and reached 8.48 to 15.29% ID/g within 24 hours after administration.

### Test Example 4 Imaging of integrin expression tumor by positron emission tomography (PET) using [⁶⁴Cu]-(compound A) and [⁶⁴Cu]-(compound B)

1 × 10⁷ U87MG cells were transplanted into the subcutaneous space of the right flank of Balb/c AJcl-nu/nu (males, 6 to 9-week-old, KURARAY CO., LTD. or Japan SLC. Inc). After 2 weeks, [⁶⁴Cu]-(compound A) was administered at 4.8 MBq/mouse into the caudal vein of the mice with a tumor having a volume of 250 mm to 650 mm³. After 1, 4, 24, and 48 hours, the mice were imaged by microPET/CT (Inveon, Siemens Healthcare GmbH) under isoflurane anesthesia. 48 hours after imaging, whole blood was collected from the postcava under deep isoflurane anesthesia, the animals were euthanased, and then the tumor was extracted. The weight of the tumor was measured, the radioactivity was measured using a gamma counter, and the radioactivity concentration in tumor (% ID/g) was calculated. For [⁶⁴Cu]-(compound B), imaging was performed by the same method.

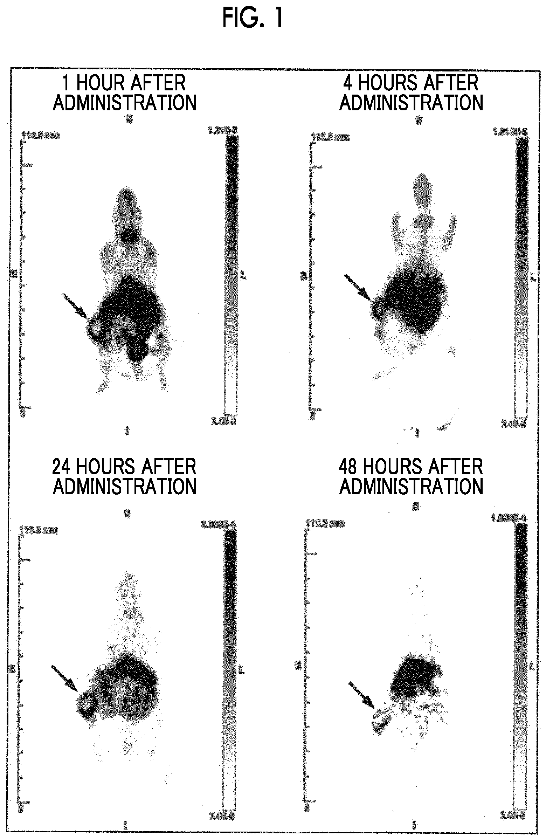

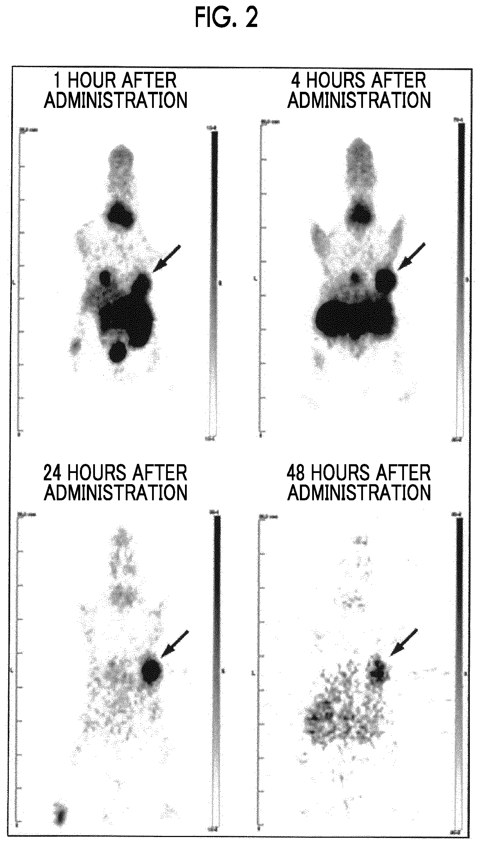

Figs. 1 and 2 show PET images relating to each compound captured at each point in time.

1 hour after administration, all of the compounds were found to integrate with the tumor, and the tumor was portrayed within 48 hours. For [⁶⁴Cu]-(compound A), because the image showed a portion in which the compound was integrated to a low degree in the central portion of the tumor, the tumor extracted after the end of the 48 hours of imaging was observed. As a result, a hematoma matching with the image was found in the central portion. At the time of dissection (48 hours after administration), the radioactivity concentration in tumor was 5.6% ID/g.

### Test Example 5 Imaging of integrin expression tumor by gamma camera using [¹¹¹In]-(compound A)

1 × 10⁷ U87MG cells were transplanted into the subcutaneous space of the right flank of Balb/c AJcl-nu/nu (males, 6-week-old, KURARAY CO., LTD). After 2 weeks, a [^{m}In]-(compound A) solution was administered at 1 MBq/mouse into the caudal vein of the mice with a tumor having a volume of 300 mm to 600 mm³. 24, 48, and 72 hours after administration, planar imaging was performed under isoflurane anesthesia by using a gamma camera (Symbia, Siemens Healthcare GmbH). Through image analysis, the radioactivity (% ID) of the tumor was calculated.

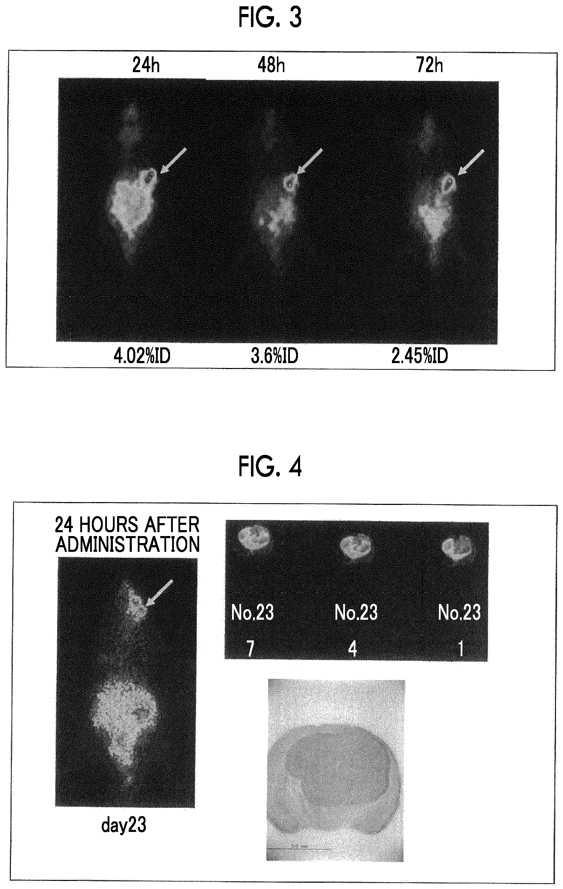

Fig. 3 shows the image and the tumor radioactivity at each point in time. Within 24 to 72 hours after administration, the radioactivity of the tumor was higher than that of other organs, and the tumor could be clearly confirmed.

### Test Example 6 Imaging of integrin expression tumor using [^{m}In]-(compound A) (intracranial tumor model)

1 × 10⁷ U87MG cells were transplanted into the cranium of Balb/c AJcl-nu/nu (males, 6-week-old, KURARAY CO., LTD) by using a two-stage needle. After 2 to 4 weeks, a [^{m}In]-(compound A) solution was administered at 1 MBq/mouse into the caudal vein of the mice. 24, 48, and 72 hours after administration, planar imaging was performed under isoflurane anesthesia by using a gamma camera (Symbia, Siemens Healthcare GmbH) (Fig. 4). After the final planar imaging was finished, the brain was extracted, and frozen sections were prepared. By bringing some of the tumor sections into contact with an IP plate, integration images were obtained by autoradiography (ARG). For the consecutive sections, hhematoxylin·eosin staining was performed, and the tumor was checked. Through planar imaging and ARG, the integration of [¹¹¹In]-(compound A) matching with the tumor was confirmed in the intracranial tumor model.

### Test Example 7 Therapeutic test on U87MG subcutaneous transplantation model using [90^{Y}]-(compound A)

1 × 10⁷ U87MG cells were transplanted into the subcutaneous space of the right flank of Balb/c Slc-nu/nu (males, 6-week-old, Japan SLC. Inc). After 2 weeks, the mice with a tumor having a volume of 100 to 500 mm³ were grouped. A phosphate buffered saline (PBS) or [90^{Y}]-(compound A) was administered into the caudal vein, and the tumor volume was measured. At a point in time when the tumor volume of the mice of the PBS group exceeded 2,000 mm³ which is a humane endpoint, the antitumor activity was evaluated. As evaluation values, a tumor growth inhibition rate ((1 - (average tumor volume of group administered with compound - average tumor volume of group administered with compound before administration)/(average tumor volume of PBS group - average tumor volume of PBS group before administration)) × 100 (here, in a case where the inhibition rate exceeded 100%, the inhibition rate was regarded as being 100%)) and the number of individuals with a tumor having a volume of equal to or less than the initial tumor volume (number of animals showing regression).

The results are shown below.

**[Table 4]**

| Compound | Dose (MBq) | Dosing frequency (number of times) | Number of animals | Tumor volume (mm³) | | Inhibition rate (%) | Number of animals showing regression |
|---|---|---|---|---|---|---|---|
| | | | | At the beginning of administration | 16 days after administration | | |
| PBS | - | 1 | 8 | 333 + 117 | 1994 ± 225 | - | 0 |
| [90^{Y}]-(compound A) | 14.8 | 1 | 8 | 351 + 72 | 429 ± 188 | 95 | 2 |
| | 22.2 | 1 | 8 | 343 ± 88 | 386 ± 142 | 97 | 2 |

| | | | | | | | |
|---|---|---|---|---|---|---|---|
| (Mean±SD) | | | | | | | |

The compound in Table 4 demonstrated excellent antitumor activity.

### Test Example 8 Therapeutic test on T98G subcutaneous transplantation model using [90^{Y}]-(compound A)

A mixture obtained by mixing T98G cell suspension (human glioblastoma, 1 × 10⁷ cells) with MATRIGEL (BD Biosciences, Japan) in an equal amount was transplanted into the subcutaneous space of the right flank of Balb/c Slc-nu/nu (males, 6-week-old, Japan SLC. Inc). After 77 days, at a point in time when the tumor volume reached 300 to 1,200 mm³, the mice were grouped. A phosphate buffered saline (PBS) or [⁹⁰Y]-(P2) was administered into the caudal vein, and the tumor volume was measured. The evaluation values were calculated by the same method as in Test Example 7, and the antitumor activity was evaluated.

The results are shown below.

**[Table 5]**

| Compound | Dose (MBq) | Dosing frequency (number of times) | Number of animals | Tumor volume (mm³) | | Inhibition rate (%) | Number of animals showing regression |
|---|---|---|---|---|---|---|---|
| | | | | At the beginning of administration | 13 days after administration | | |
| PBS | - | 1 | 6 | 754 ± 317 | 1439 ± 638 | - | 0 |
| [90^{Y}]-(compound A) | 22.2 | 1 | 6 | 755 ± 293 | 882 ± 399 | 82 | 1 |
| | 29.6 | 1 | 6 | 736 ± 264 | 755 ± 313 | 97 | 3 |

| | | | | | | | |
|---|---|---|---|---|---|---|---|
| (Mean±SD) | | | | | | | |

The compound in Table 5 demonstrated excellent antitumor activity.

### Test Example 9 Imaging of monkey by using [¹¹¹In]-(compound A)

Blood was collected from a crab-eating macaque over time, and by using [^{m}In]-(compound A), from the radioactivity concentration in the blood, kinetic parameters of the compound in blood were calculated by OLINDA/EXM 1.0. Furthermore, by using [^{m}In]-(compound A), from the organ distribution obtained by imaging, the absorbed dose of each organ obtained in a case where the compound is administered to a human being was calculated using OLINDA/EXM 1.0.

[^{m}In]-(compound A) (98 MBq/9.3 µg) was administered to a crab-eating macaque (Hamri Co., Ltd., males, 3-year-old, 3.4 kg) under anesthesia. After the administration, blood was collected over time, and imaging was performed using a gamma camera. The blood was collected 10, 30, and 60 minutes after the administration and 2, 4, 5, 6, 24, 48, 72, and 144 hours after the administration. Regarding the imaging, after 1, 2, 4, 6, 24, 48, 72, and 144 hours, planar imaging was performed using a gamma camera (Symbia, Siemens Healthcare GmbH). Regarding the anesthesia, the animal was anesthetized with ketamine at 20 mg/kg before the administration of [^{m}In]-(compound A) and kept anesthetized until the end of imaging, which was continued for 6 hours after the administration, by inhalation anesthesia (isofluran 2 to 3%, 5 to 8 L/min). After 24 hours, ketamine (20 mg/kg) and xylazine (2 mg/kg) were administered to perform blood collection and imaging.

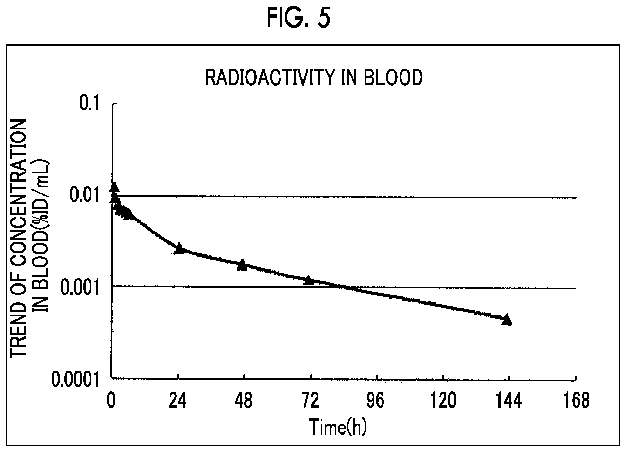

Fig. 5 shows a trend of radioactivity concentration in the blood of the monkey for which [¹¹¹In]-(compound A) was used. The kinetic parameters in blood are also shown below.

**[Table 6]**

| Kinetic parameters in blood | evaluation |
|---|---|
| AUC (%ID·h/mL) | 0.22 |
| T1/2α (h) | 0.46 |
| T1/2β (h) | 19.3 |
| Cmax (%ID/mL) | 0.018 |
| CL (mL/h/kg) | 130.2 |
| Vss (L/kg) | 3.52 |

AUC was 0.22 (% ID·h/mL), T_{1/2}α was 0.46 (h), T_{1/2}β was 19.3 (h), Cmax was 0.018 (% ID/mL), CL was 130.2 (mL/h/kg), and Vss was 3.52 (L/kg).

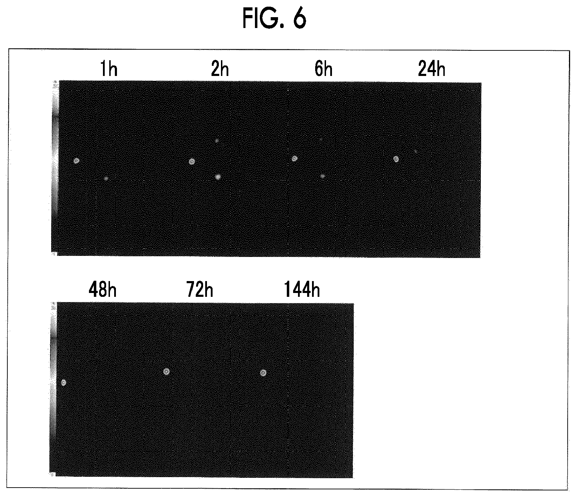

Fig. 6 shows results obtained by temporally performing planar imaging on the monkey for which [^{m}In]-(compound A) was used. During the imaging, up to 6 hours after the administration, the integration of the compound into the bladder and the gall bladder increased over time. Furthermore, by using exposure dose analysis software OLINDA/EXM 1.0, the absorbed dose in a human being was simulated using each of the labeled compounds, and the results are shown below.

**[Table 7]**

| Organ | Absorbed dose (mGy/MBq) | | |
|---|---|---|---|
| | [90^{Y}]-(compound A) | [^{m}In]-(compound A) | [⁶⁴Cu]-(compound A) |
| Whole body | 0.27 | 0.05 | 0.02 |
| Red marrow | 0.06 | 0.05 | 0.01 |
| Brain | 1.96 | 0.25 | 0.11 |
| Lung | 1.35 | 0.12 | 0.08 |
| Liver | 1.12 | 0.19 | 0.09 |
| Kidney | 14.7 | 1.19 | 0.69 |
| Small intestine | 0.87 | 0.05 | 0.06 |

The manufacturing method of the present invention is a useful as a method for manufacturing a novel nitrogen-containing compound or a salt thereof. Furthermore, the manufacturing intermediate of the present invention is useful as an intermediate for efficiently manufacturing a novel nitrogen-containing compound and a salt thereof.

### Embodiments:

1. A method for manufacturing a compound represented by Formula [11] or a salt thereof, comprising:
   (1) a step of reacting a compound represented by Formula [1] or a salt thereof 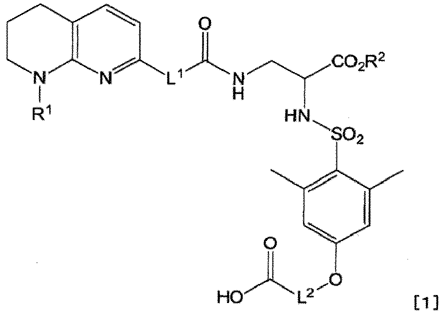 (in the formula, R¹ represents a hydrogen atom or an amino-protecting group; R² represents a carboxyl-protecting group; L¹ represents a group represented by Formula [2a] 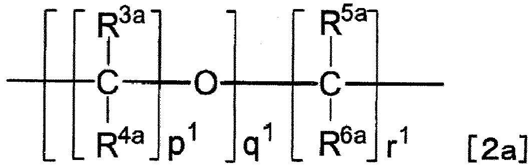 (in the formula, R^{3a}, R^{4a}, R^{5a} and R^{6a} are the same as or different from each other and represent a hydrogen atom or a C₁₋₆ alkyl group; p¹ represents an integer of 1 to 3; q¹ represents an integer of 0 to 3; and r¹ represents an integer of 1 to 6); and L² represents a group represented by Formula [2b] 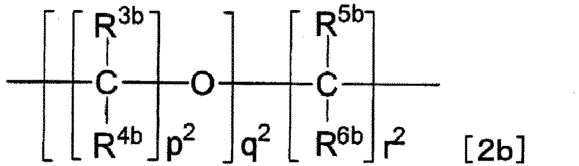 (in the formula, R^{3b}, R^{4b}, R^{5b}, and R^{6b} are the same as or different from each other and represent a hydrogen atom or a C₁₋₆ alkyl group; p² represents an integer of 1 to 3, q² represents an integer of 0 to 3; and r² represents an integer of 1 to 6)) with a compound represented by Formula [3] or a salt thereof 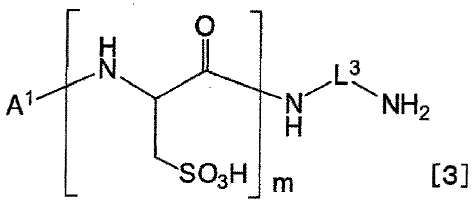 (in the formula, L³ represents a group represented by Formula [2c] 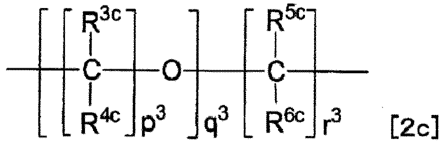 (in the formula, R^{3c}, R^{4c}, R^{5c}, and R^{6c} are the same as or different from each other and represent a hydrogen atom or a C₁₋₆ alkyl group; p³ represents an integer of 1 to 3; q³ represents an integer of 0 to 3; and r³ represents an integer of 1 to 6); A¹ represents any one of the groups represented by Formulae [4] to [9] 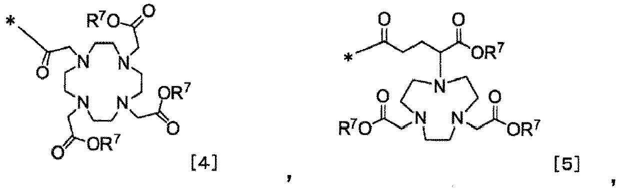 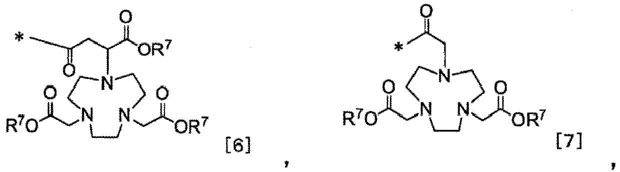 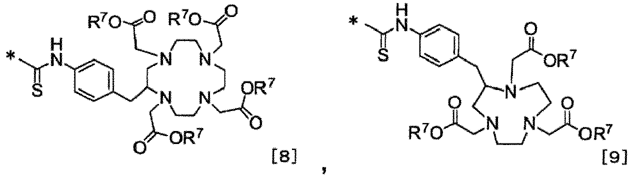 (in the formulae, * represents a binding position; and R^{7'}s are the same as or different from each other and represent a carboxyl-protecting group); and m represents an integer of 1 to 3)) so as to obtain a compound represented by Formula [10] or a salt thereof; 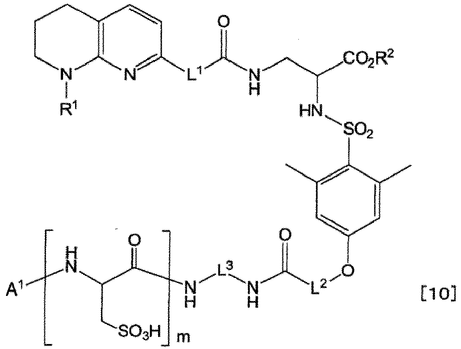 (in the formula, R¹, R², L¹, L², L³, A¹, and m have the same definition as R¹, R², L¹, L², L³, A¹, and m described above); and
   (2) a step of deprotecting the compound represented by Formula [10] or a salt thereof, 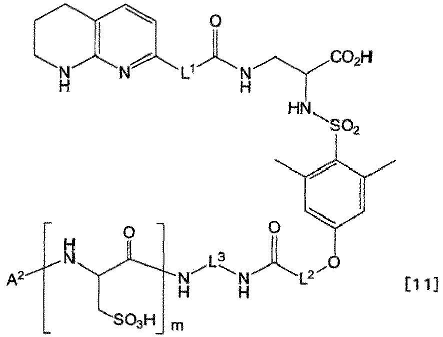 (in the formula, A² represents any one of the groups represented by Formulae [12] to [17] 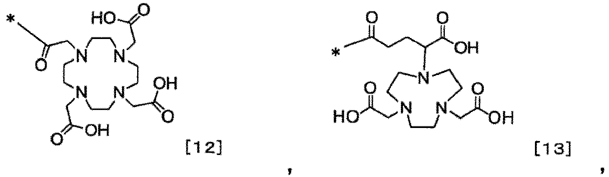 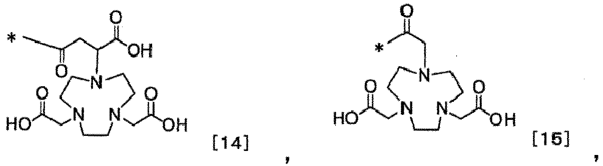 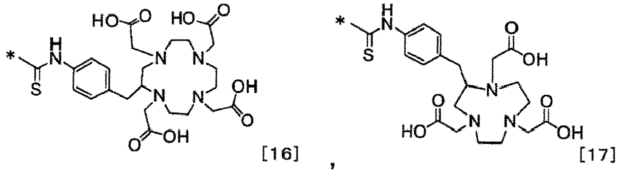 (in the formulae, * represents a binding position); and L¹, L², L³, and m have the same definition as L¹, L², L³, and m described above).
2. The manufacturing method according to clause 1,
   wherein R² is a C₁₋₆ alkyl group which may be substituted or a benzyl group which may be substituted.
3. The manufacturing method according to clause 1 or 2,
   wherein L³ is a group represented by Formula [18c] 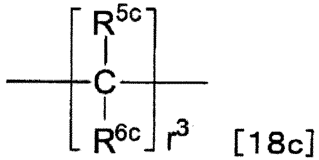 (in the formula, R^{5c} and R^{6c} may be the same as or different from each other and represent a hydrogen atom or a C₁₋₆ alkyl group; and r³ represents an integer of 1 to 6).
4. The manufacturing method according to any one clauses 1 to 3,
   wherein L¹ is a group represented by Formula [18a] 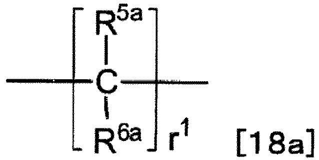 (in the formula, R^{5a} and R^{6a} are the same as or different from each other and represent a hydrogen atom or a C₁₋₆ alkyl group; and r¹ represents an integer of 1 to 6).
5. The manufacturing method according to any one of clauses 1 to 4,
   wherein L² is a group represented by Formula [18b] 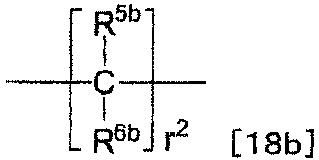 (in the formula, R^{5b} and R^{6b} are the same as or different from each other and represent a hydrogen atom or a C₁₋₆ alkyl group; and r² represents an integer of 1 to 6).
6. The manufacturing method according to any one of clauses 1 to 5,
   wherein R¹ is a hydrogen atom, a C₁₋₆ alkoxycarbonyl group which may be substituted, an arylsulfonyl group which may be substituted, or a heterocyclic sulfonyl group which may be substituted.
7. The manufacturing method according to any one of clauses 1 to 6,
   wherein R⁷ is a C₁₋₆ alkyl group which may be substituted or a benzyl group which may be substituted.
8. The manufacturing method according to any one of clauses 1 to 7,
   wherein the step of deprotecting is a step of deprotecting by using an acid.
9. A method for manufacturing a metal complex, comprising:
   a step of reacting the compound represented by Formula [11] or a salt thereof obtained by the manufacturing method according to any one of clauses 1 to 7 with a metal ion.
10. A compound represented by Formula [19] or a salt thereof 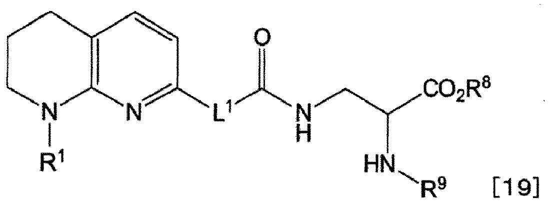 (in the formula, R¹ represents a hydrogen atom or an amino-protecting group; R⁸ represents a C₂₋₆ alkyl group which may be substituted or a benzyl group which may be substituted; R⁹ represents a hydrogen atom, an amino-protecting group, or a group represented by Formula [20] 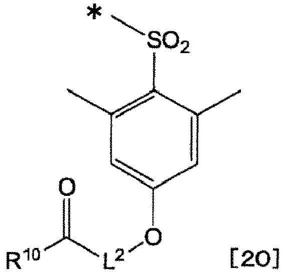 (in the formula, * represents a binding position; R¹⁰ represents a hydroxyl group or a group represented by Formula [21] 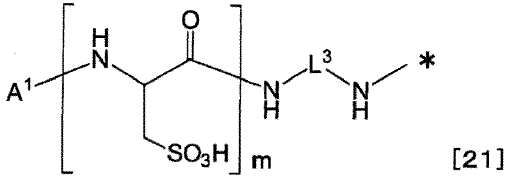 (in the formula, * represents a binding position, L³ represents a group represented by Formula [2c] 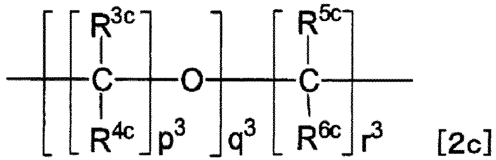 (in the formula, R^{3c}, R^{4c}, R^{5c}, and R^{6c} are the same as or different from each other and represent a hydrogen atom or a C₁₋₆ alkyl group; p³ represents an integer of 1 to 3; q³ represents an integer of 0 to 3; and r³ represents an integer of 1 to 6); A¹ represents any one of the groups represented by Formulae [4] to [9] 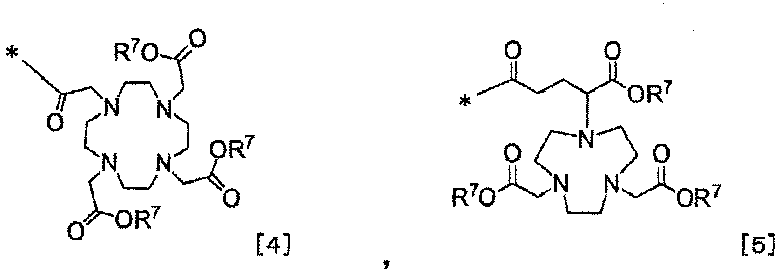 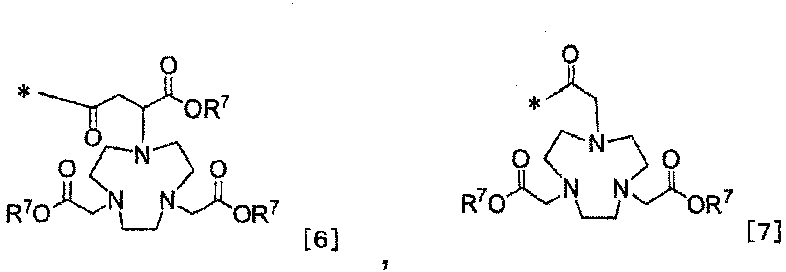 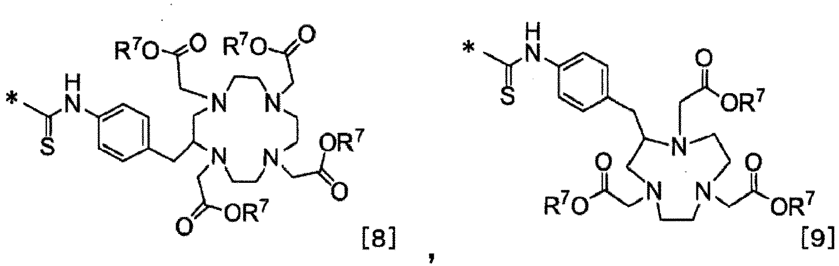 (in the formulae, * represents a binding position; and R⁷ represents a carboxyl-protecting group); and m represents an integer of 1 to 3); L² represents a group represented by Formula [2b] 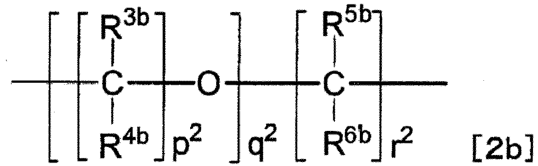 (in the formula, R^{3b}, R^{4b}, R^{5b}, and R^{6b} are the same as or different from each other and represent a hydrogen atom or a C₁₋₆ alkyl group; p² represents an integer of 1 to 3; q² represents an integer of 0 to 3; and r² represents an integer of 1 to 6); and L¹ represents a group represented by Formula [2a] 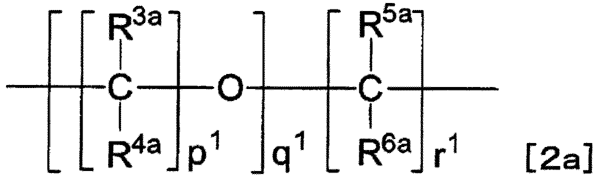 (in the formula, R^{3a}, R^{4a}, R^{5a} and R^{6a} are the same as or different from each other and represent a hydrogen atom or a C₁₋₆ alkyl group; p¹ represents an integer of 1 to 3; q¹ represents an integer of 0 to 3; and r¹ represents an integer of 1 to 6)).
11. The compound according to clause 10 or a salt thereof,
   wherein R⁸ is a C₂₋₆ alkyl group which may be substituted.
12. The compound according to clause 10 or 11 or a salt thereof, wherein L³ is a group represented by Formula [18c] 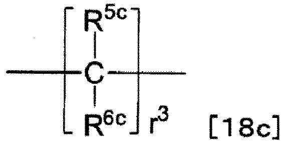 (in the formula, R^{5c} and R^{6c} are the same as or different from each other and represent a hydrogen atom or a C₁₋₆ alkyl group; and r³ represents an integer of 1 to 6).
13. The compound according to any one of clauses 10 to 12 or a salt thereof,
   wherein L¹ is a group represented by Formula [18a] 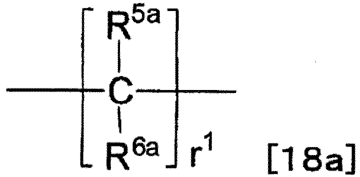 (in the formula, R^{5a} and R^{6a} are the same as or different from each other and represent a hydrogen atom or a C₁₋₆ alkyl group; and r¹ represents an integer of 1 to 6).
14. The compound according to any one of clauses 10 to 13 or a salt thereof, wherein L² represents a group represented by Formula [18b] 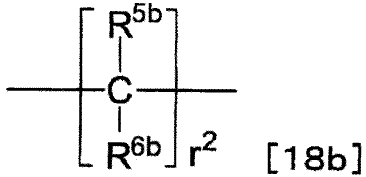 (in the formula, R^{5b} and R^{6b} are the same as or different from each other and represent a hydrogen atom or a C₁₋₆ alkyl group; and r² represents an integer of 1 to 6).
15. The compound according to any one of clauses 10 to 14 or a salt thereof,
   wherein R¹ is a hydrogen atom, a C₁₋₆ alkoxycarbonyl group which may be substituted, an arylsulfonyl group which may be substituted, or a heterocyclic sulfonyl group which may be substituted.
16. The compound according to any one of clauses 10 to 15 or a salt thereof,
   wherein R⁷ is a C₁₋₆ alkyl group which may be substituted or a benzyl group which may be substituted.
17. A compound represented by Formula [3] or a salt thereof 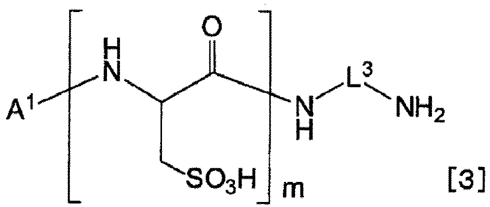 (in the formula, L³ represents a group represented by Formula [2c] 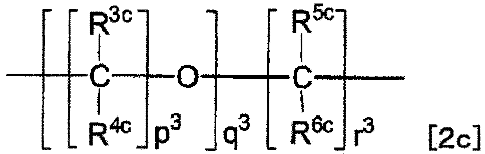 (in the formula, R^{3c}, R^{4c}, R^{5c}, and R^{6c} are the same as or different from each other and represent a hydrogen atom or a C₁₋₆ alkyl group; p³ represents an integer of 1 to 3; q³ represents an integer of 0 to 3; and r³ represents an integer of 1 to 6); A¹ represents any one of the groups represented by Formulae [4] to [9] 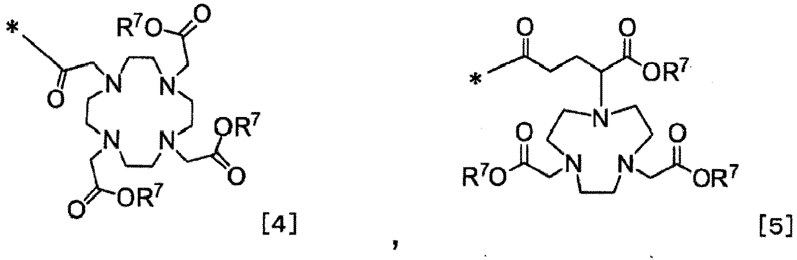 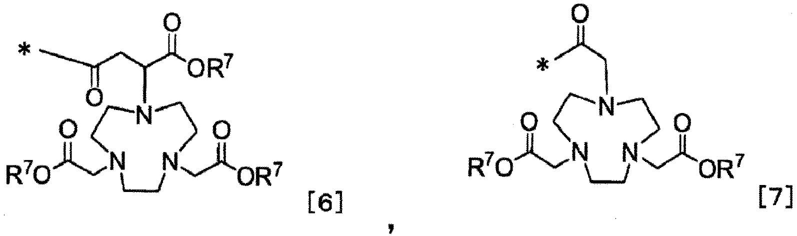 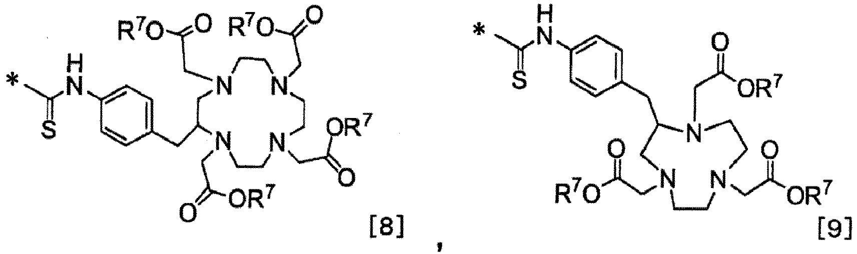 (in the formulae, * represents a binding position; and R⁷ represents a carboxyl-protecting group); and m represents an integer of 1 to 3).
18. The compound according to clause 17 or a salt thereof,
   wherein R⁷ is a C₁₋₆ alkyl group which may be substituted or a benzyl group which may be substituted.

Further embodiments of the present description are:
19. A complex of 2,2',2"-(10-(2-(((R)-1-((2-(4-(4-(N-((S)-1-carboxy-2-(5-(5,6,7,8-tetrahydro-1,8-naphthyridin-2 -yl)pentanamido)ethyl)sulfamoyl)-3,5-dimethylphenoxy)butanamido)ethyl)amino)-1-oxo-3-su lfopropan-2-yl)amino)-2-oxoethyl)-1,4,7,10-tetraazacyclododecane-1,4,7-triyl)triacetic acid or a salt thereof and 67Ga.
20. The complex according to clause 19 for use in diagnosis of a disease involving an integrin.
21. The complex for use according to clause 20, wherein the disease is cancer.
22. The complex for use according to clause 21, wherein the cancer is solid cancer.
23. The complex for use according to clause 21 or 22, wherein the cancer is head and neck cancer, colorectal cancer, breast cancer, small-cell lung cancer, non-small cell lung cancer, glioblastoma, malignant melanoma, pancreatic cancer, or prostate cancer.

## Claims

1. A complex of 2,2',2"-(10-(2-(((R)-1-((2-(4-(4-(N-((S)-1-carboxy-2-(5-(5,6,7,8-tetrahydro-1,8-naphthyridin-2 -yl)pentanamido)ethyl)sulfamoyl)-3,5-dimethylphenoxy)butanamido)ethyl)amino)-1-oxo-3-su lfopropan-2-yl)amino)-2-oxoethyl)-1,4,7,10-tetraazacyclododecane-1,4,7-triyl)triacetic acid or a salt thereof and 177Lu.

2. The complex according to claim 1 for use in the treatment of a disease involving an integrin.

3. The complex for use according to claim 2, wherein the disease is cancer.
